# EUROPEAN PATENT APPLICATION

(11) **EP 4 830 744 A2**
(43) Date of publication of application: **28.10.2026**
(21) Application number: 26204808.5
(22) Date of filing: 04.06.2024
(51) Int. Cl.: A61M 5/14

(54) **DRUG DELIVERY DEVICE**

(30) Priority: 16.06.2023 US 202363508591 P; 11.08.2023 US 202363532275 P; 05.09.2023 US 202363536677 P; 04.01.2024 US 202463617684 P
(62) Document number(s) of the earlier application(s) in accordance with Art. 76 EPC: 24734981.4 / 4 727 619
(71) Applicant: Janssen Biotech, Inc., Horsham, PA 19044 (US)
(72) Inventor: BINNER, Curt, Fort Washington, 19034 (US); WALSH, Ryan, Fort Washington, 19034 (US); MELLINGER, Justin, Fort Washington, 19034 (US)
(74) Representative: Carpmaels & Ransford LLP

(57) **Abstract**

In one example, a drug delivery system, such as an on-body or off-body delivery system is configured to deliver a therapeutic into a patient. The system has a curved track, a plunger, and a driver. The driver causes the plunger to translate along the curved track such that a flexible plunger rod of the plunger bends along the curved track to drive a plunger seal of a drug container to expel a liquid drug from the drug container.

## Description

### TECHNICAL FIELD

The present disclosure relates to drug delivery systems, and more specifically, but not necessarily exclusively, to drug delivery systems that deliver a liquid drug.

### BACKGROUND

Pharmaceutical products (including large and small molecule pharmaceuticals, hereinafter "drugs") are administered to patients using a variety of different drug delivery devices for the treatment of a variety of different medical indications. Drug delivery devices for delivering liquid drugs include, for example, syringes, manual injectors, pen injectors, autoinjectors, on-body delivery devices, and off-body delivery devices. These delivery devices commonly include an actuator, a drug container, and a needle or cannula. The drug container contains the liquid drug and the actuator drives the liquid drug from the drug container, and through the needle or cannula to the patient.

### BRIEF DESCRIPTION OF THE DRAWINGS

The following description of the illustrative embodiments may be better understood when read in conjunction with the appended drawings. It is understood that potential embodiments of the disclosed systems and methods are not limited to those depicted.
Fig. 1 shows a simplified schematic diagram of a drug delivery system according to one example;
Fig. 2 shows a perspective view of a drug container according to one example that can be implemented with the drug delivery system of Fig. 1;
Fig. 3 shows a perspective view of the drug delivery system of Fig. 1 according to one example with a closure in a closed position;
Fig. 4 shows another perspective view of the drug delivery system of Fig. 1 according to one example with the closure in an open position and a container in an uninstalled position;
Fig. 5 shows a side view of the drug delivery system of Fig. 1 according to one example;
Fig. 6 shows a perspective view of an actuator and track according to one example that may be used to implement an actuator and track of the drug delivery system of Fig. 1;
Fig. 7 shows a plan view of the actuator of Fig. 6 with at least a portion of the track removed;
Fig. 8 shows a perspective view of a portion of the actuator of Fig. 6 that includes a flexible plunger rod;
Fig. 9 shows an enlarged perspective view of a portion of the flexible plunger rod of Fig. 8;
Fig. 10 shows a cross-sectional view of a portion of the flexible plunger rod of Fig. 8;
Fig. 11 shows a perspective view of flexible plunger rod and track according to another example that may be used to implement the flexible plunger rod and track of the drug delivery system of Fig. 1;
Fig. 12 shows a perspective view of flexible plunger rod according to yet another example that may be used to the plunger rod of the drug delivery system of Fig. 1;
Fig. 13 graphically illustrates exemplary delivery forces needed to deliver a 100 centipoise (cP) fluid and a 326 centipoise fluid at different flow rates and different needle gauges from a drug container using a straight plunger rod;
Fig. 14 graphically illustrates a performance envelope observed for a prototype drug delivery device of this disclosure;
Fig. 15 shows a sectional view of a portion of the drug delivery system according to another example;
Fig. 16A shows a sectional view of the drug delivery system of Fig. 15 according to one example with a drug container in a pre-pierced position;
Fig. 16B shows a sectional view of a portion of the drug delivery system of Fig. 15 according to one example with a drug container in a pierced position;
Fig. 17A shows a perspective view of a portion of the drug delivery system according to another example with a needle shield in a shielded position;
Fig. 17B shows a perspective view of a portion of the drug delivery system of Fig. 17A with the needle shield in an exposed position;
Fig. 18 shows schematic views of the drug delivery system of Fig. 15 according to one example with a drug container moving between the pre-pierced position, the pierced position, and a removed position;
Fig. 19 shows a schematic view of the drug delivery system of Fig. 15 according to one example illustrating a position of the drug container relative to a septum piercing needle and a plunger;
Fig. 20 shows another schematic view of the drug delivery system of Fig. 15 according to one example illustrating the position of the drug container relative to the septum piercing needle and the plunger;
Fig. 21 shows an exemplary method of using the drug delivery system according to one example;
Figs. 22A through 22G show schematic views of the drug delivery system according to another example, during various stages of inserting a drug container, piercing the drug container, delivering a drug from the drug container, and removing the drug container after delivery of the drug;
Figs. 23A through 23N show schematic views of the drug delivery system according to another example, during various stages of delivering a drug from a first drug container, changing out the first drug container with a second drug container, and delivering the drug from the second drug container;
Fig. 24 shows a perspective view of the drug delivery system according to another example;
Fig. 25 shows a perspective view of the drug delivery system according to an exemplary use case;
Fig. 26 shows a perspective view of the drug delivery system according to another exemplary use case;
Fig. 27 shows a perspective view of the drug delivery system according to another exemplary use case;
Fig. 28 shows an exemplary method of using the drug delivery system according to one example;
Fig. 29 shows a sectional view of the drug delivery system according to another example;
Fig. 30 shows a sectional view of a portion of the drug delivery system of Fig. 29 according to one example with a septum piercing assembly in an unpierced configuration of the drug delivery system;
Fig. 31 shows a sectional view of another portion of the drug delivery system of Fig. 29 according to one example with a drug container in the unpierced configuration of the drug delivery system;
Fig. 32A shows a sectional view of a portion of the drug delivery system in an unengaged and unpierced configuration according to another example;
Fig. 32B shows a section view of a portion of the drug system in an engaged configuration, but prior to piercing according to the example of Fig. 32A
Fig. 33 shows an exemplary method of using the drug delivery system of Fig. 29 according to one example;
Figs. 34A through 34H show sectional views of the drug delivery system of Fig. 29 during various stages, including the unpierced configuration, a pierced configuration, and a post-pierced configuration; and
Figs. 35A and 35B show sectional views of the drug delivery system according to another example.

### DETAILED DESCRIPTION OF ILLUSTRATIVE EMBODIMENTS

The present disclosure relates to on-body delivery systems (OBDSs) and off-body delivery systems that are configured to inject a liquid therapeutic (e.g., drug or pharmaceutical) into a patient. Although some existing on-body delivery systems are in various stages of commercial development, the inventors have found that these existing systems might not meet the needs of some future therapeutics, particularly the needs of some future large molecule (e.g., biologic) therapeutics. Some of these therapeutics might require systems that are capable of delivering the therapeutic to the patient with significantly higher driving forces that exceed the capabilities of existing on-body delivery systems. For example, some future therapeutics may have relatively high viscosities (discussed further below) that require higher driving forces to deliver the therapeutics. The need for higher driving forces may also be dictated by a need for subcutaneous injections, a need for relatively fast flow rates, and a need for relatively short injection times. These future therapeutics may also require the ability to deliver multiple doses of the same therapeutic, separate doses of different therapeutics, and/or variable volume doses based on, for example, patients' weight and/or age. The present application relates to drug delivery systems, and features thereof, that address various needs of future therapeutics.

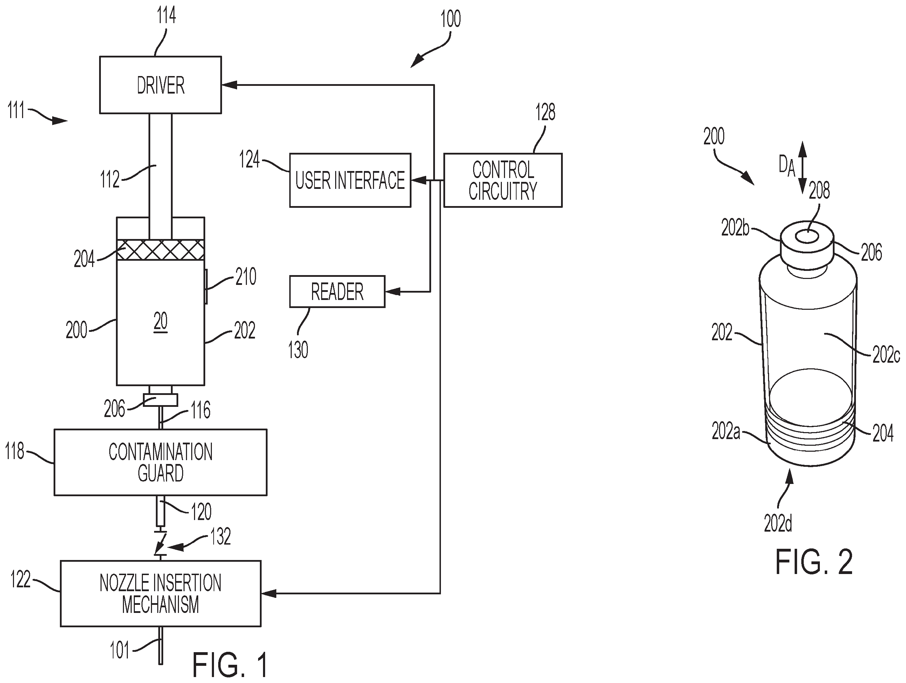

Referring to Fig. 1, a simplified schematic of a drug product according to one example is illustrated. The drug product comprises a drug delivery system 100 and a liquid therapeutic 20 contained within the drug delivery system 100. The drug delivery system 100 can be a prefilled drug delivery system 100 distributed with the therapeutic 20 contained therein such that the user (e.g., healthcare provider or patient) need not fill the drug delivery system 100 with the therapeutic 20 prior to use. In alternative examples, the drug delivery system 100 can be distributed separately from the therapeutic 20 such that the drug delivery system 100 needs to be filled with the therapeutic 20 prior to use.

The drug delivery system 100 is configured to expel the liquid therapeutic 20 from a drug container 200 and into a patient via a nozzle 101. The nozzle 101 is configured to be inserted into a patient such as into a patient's skin. The nozzle 101 can be, for example, a needle or cannula. Preferably, the drug delivery system 100 is a subcutaneous delivery system configured to deliver the therapeutic 20 to a subcutaneous layer of the patient's skin. Thus, the nozzle 101 can configured to extend from the system 100 by a distance that extends into, but not beyond, the subcutaneous layer. This distance can be in a range of, for example, about 6mm to about 8mm.

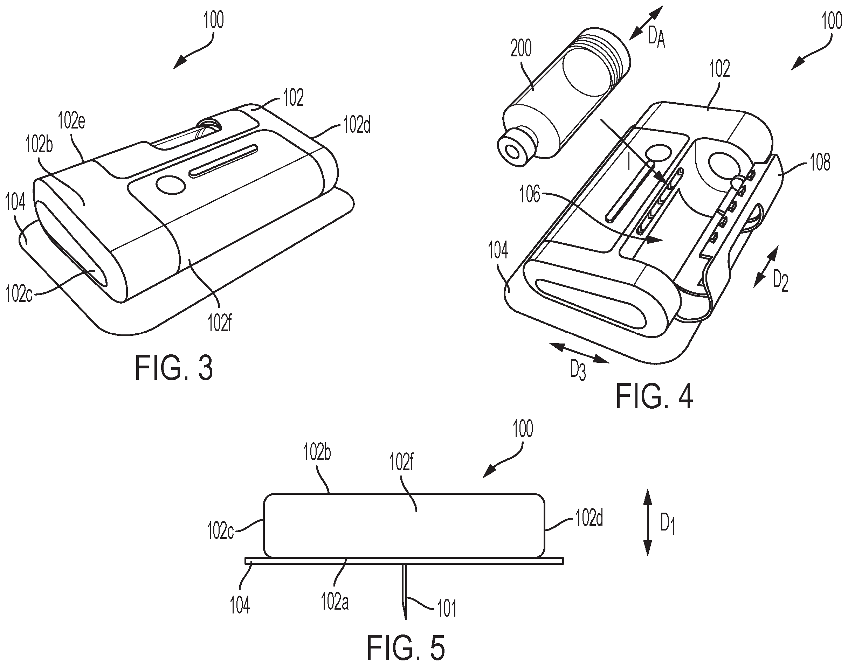

The drug delivery system 100 can be used as an on-body delivery system (OBDS), where the drug delivery system 100 abuts the patient's body. In such examples, the nozzle 101 can extend from a housing (e.g., 102 of Figs. 3 to 5 below) of the drug delivery system 100 into the patient, where the housing abuts the patient. Alternatively, the drug delivery system 100 can be used as an off-body delivery system, where the housing of the drug delivery system 100 is spaced from the nozzle when the nozzle is inserted into the patient. In such examples, the drug delivery system 100 can include conduits (e.g., tubing) that span a gap between the housing of the drug delivery system 100 and the nozzle 101 to route the drug from the drug delivery system 100 to the nozzle 101. Alternatively still, the drug delivery system 100 can be selectively configurable to be used as either an on-body delivery system or an off-body delivery system.

The drug delivery system 100 can comprise the drug container 200 or the drug container 200 can be a separate component from the drug delivery system 100. The drug container 200 can be supported by, or configured to be supported by, a housing of the drug delivery system 100. In some examples, the drug container 200 can be removably attachable to or removably insertable into the housing of the drug delivery system 100. In other examples, the drug container 200 can be fixedly attached to or fixedly inserted into the housing. In yet other examples, the drug container 200 can be integral with the housing.

The drug container 200 can be any suitable container for containing a liquid drug, such as a cartridge or a syringe. Fig. 2 shows an example of a drug container 200 according to one example, where the drug container 200 is a cartridge. The drug container 200 comprises a container body 202 defining a cavity 202c configured to hold a liquid drug therein. The container body 202 has a first end 202a and a second end 202b. The container body 202 can have a central axis that extends along an axial direction D_{A}. The first end 202a can define an opening 202d therein that is open to the cavity 202c. The drug container 200 can comprise a seal 204 disposed in the cavity that forms a seal with an interior surface of the container body 202. The seal 204 can be received through the opening 202d into the cavity 202c. The seal 204 is configured to translate towards the second end 202b to drive the liquid drug from the cavity 202c.

In some examples, such as where the drug container 200 is a cartridge, the drug container 200 can comprise a cap 206 on the second end 202b. The cap 206 can be formed from any suitable material, such as a metal. The cap 206 can be crimped onto a head of the container body 202 at the second end 202b. The drug container can comprise a septum 208 supported by the cap 206. The septum 208 is configured to seal the second end 202b. The septum 208 is configured to be pierced by a piercing needle to open a fluid path into the drug container 200. The septum 208 can optionally be configured to reseal the second end 202b when the piercing needle is removed from the septum 208. The space between the cap 206 and the seal 204 is filled with a substantially incompressible fluid (e.g., the therapeutic and possibly air) that prevents movement of the seal 204 until the septum 208 is pierced.

Returning to Fig. 1, the drug delivery system 100 comprises an actuator 111 configured to drive a liquid therapeutic 20 from the drug container 200 out of the needle or cannula 101. The actuator 111 can be any suitable actuator for expelling a liquid therapeutic 20 from the drug container 200. The actuator 111 can comprise a plunger 112 that is configured to move the seal 204 of the drug container 200 to drive a liquid therapeutic 20 from the drug container 200. The actuator 111 can comprise a driver 114 that is configured to cause the plunger 112 to move the seal 204. The driver 114 can be any suitable driver, such as (without limitation) a motor, a spring, a hydraulic driver, or a pneumatic driver. The plunger 112 can be any suitable plunger, such as a flexible plunger or a telescoping plunger.

The drug delivery system 100 can comprise a septum piercing needle 116 that is configured to pierce the septum 208 of the drug container 200. At least one of the septum piercing needle 116 and the drug container 200 can be configured to move towards the other to cause the piercing needle 116 to pierce the septum 208. Piercing the septum 208 can place the septum piercing needle 116 in fluid communication with the liquid therapeutic 20 contained within the drug container 200. The drug delivery system 100 can comprise a conduit 120, such as tubing, that fluidly connects the septum piercing needle 116 to the nozzle 101. Thus, piercing the septum 208 can place the nozzle 101 in fluid communication with the liquid therapeutic 20 contained within the drug container 200 via the septum piercing needle 116 and the conduit 120.

The drug delivery system 100 can optionally comprise a contamination guard 118 that protects the piercing needle 116 from contamination when the piercing needle 116 is not piercing the septum 208 of the drug container 200. For example, the contamination guard 118 can be configured to protect the piercing needle 116 from contamination before the drug container 200 is supported by a housing of the drug delivery system 100, while the drug container 200 is supported by the housing but before the septum 208 is pierced, and/or after the drug container 200 is removed from the housing post injection.

The drug delivery system 100 can comprise a nozzle insertion mechanism 122 that is configured to cause the nozzle 101 to be inserted into a patient, such as into a patient's skin. The nozzle insertion mechanism 122 can be configured to cause the nozzle 101 to extend out of a housing of the drug delivery system 100 and into the patient. In some examples, the nozzle insertion mechanism 122 can be configured to cause the nozzle 101 to retract back into the housing after injection. Additionally, or alternatively, the drug delivery system 100 can comprise a needle guard (not shown) that extends over the nozzle 101 after injection. Retracting and/or covering the nozzle 101 after injection can prevent inadvertent needle sticks and/or limit human contact with biological materials remaining on the nozzle 101. The nozzle insertion mechanism 122 can be any suitable mechanism, including (without limitation) those known in the art, for inserting the nozzle 101 into a patient. The nozzle insertion mechanism 122 can include a driver, such as a motor or spring, that causes the nozzle 101 to be inserted into the patient.

The drug delivery system 100 can comprise control circuitry 128 that is configured to control various features of the drug delivery system 100. For example, the control circuitry 128 can be configured to control operation of the driver 114 of the actuator 111. The control circuitry 128 can be configured to cause the actuator 111 to begin driving the liquid therapeutic 20 from the drug container 200. The control circuitry 128 can be configured to control the flow rate in which the liquid therapeutic 20 is driven from the drug container 200. The control circuitry 128 can be configured to cause the actuator 111 to stop driving the liquid therapeutic 20 when an injection is complete and/or when an error is detected during the injection. The control circuitry 128 can also be configured to control operation of the nozzle insertion mechanism 122 to cause the nozzle 101 to be inserted into the patient before injection and/or removed from the patient after injection.

The drug delivery system 100 can comprise a user interface 124 that is configured to be engaged by a user such as a health care provider or patient to operate the drug delivery system 100. The user interface 124 can be configured to provide information to a user of the drug delivery system 100. The user can be, for example, a patient, or a patient's care giver or health care professional assisting the patient in using the drug delivery system 100. The user interface 124 can have a variety of configurations, and the drug delivery system 100 can include a single type of user interface or can include more than one type of user interface. For example, the user interface 124 can include one or more lights, e.g., a light emitting diode (LED) or other type of light, configured to illuminate to provide various information. Examples of the information indicated by the user interface 124 include power (on/off) status, error state (e.g., low power supply, improper nozzle advancement into the patient, incompatible type of container 200 loaded into the drug delivery system 100, etc.), drug delivery status (e.g., indication that drug delivery is currently occurring), drug delivery progress information, an orientation of the drug delivery system 100 relative to gravity, an indication of a dose of the drug 20 to be provided in each delivery of the drug 20 to the patient, and other types of information.

For another example, the user interface 124 can include a display configured to show information thereon, such as by using text and/or graphics. The display can include a display screen having any of a variety of configurations, such as a cathode ray tube (CRT), a liquid crystal display (LCD), a touchscreen, etc. For yet another example, the user interface 124 can include a vibration mechanism configured to vibrate with the vibration being configured to be felt by the patient wearing the drug delivery system 100. For still another example, the user interface 124 can include a speaker configured to provide an audio signal. For another example, the user interface 124 can include a mechanical level configured to indicate the pump's orientation.

In some examples, the system can comprise a data storage component 210 supported by the drug container 200 and a reader 130 that is configured to read the data storage component 210. The reader 130 can be supported by, for example, the housing 102. The data storage component 210 can be attached to drug container 200, such as the body 202 or cap 206 of the drug container 200, such as by being adhered to the drug container 200, or that is otherwise part of the container 200, such as by being printed thereon. The data storage component 210 can have a variety of configurations. For example, the data storage component 210 can include an integrated circuit configured to communicate the reservoir data from the reservoir. One example of an integrated circuit is a near field communication (NFC) tag, also referred to as a proximity-integrated circuit card (PICC). An ISO14443 A passive NFC tag, an ISO15693 passive NFC tag, an ISO18000-3 passive NFC tag, an ISO14443 A/B passive NFC tag, a passive FeliCa NFC tag, or other type of NFC tag (passive or active) can be used. For another example, the data storage component 210 can include a radio frequency identification (RFID) tag. For yet another example, the data storage component can be in the form of a barcode. One example of a barcode is a QR code. Another example of a barcode is a Universal Product Code (UPC) code.

The drug container 200 includes a single data storage component 210 in this illustrated embodiment but can include a plurality of data storage components. If a plurality of data storage components are used, each can be different from one another, which may help provide redundancy and/or allow for data retrieval even if a certain type of data communication is currently unavailable, e.g., if an RFID tag is absent or damaged so as to be unreadable a QR code may still be read.

The data storage component 210 is configured to store data regarding the drug container 200, regarding the drug 20 contained in the drug container 200, and/or regarding delivery parameters of the drug 20. For example, the data storage component 210 can store information related to a dosing regimen of the drug 20 in the drug container 200. For instance, the data storage component can store one or more of: the volume of the drug 20 stored in the drug container 200, the amount of drug 20 to be delivered (dose amount) by the system 100 (which can be less than the amount stored in the container 200), the flow rate in which the system 100 is to deliver the drug 20, or any suitable data for configuring an operating parameter of the system 100.

In some examples, the reader 130 can transmit data as well as receive data, and optionally cause that data to be written to the data storage component 210. In some such examples, the data storage component 210 can be updated at regular intervals (e.g., at delivery completion of each mL) so that it contains a reasonably-accurate record of the delivery progress at any given time. If the system 100 should fail during delivery, resulting in a partially-delivered dose, the reservoir containing a record of the partial dose can be transferred to a secondary system, where the remaining dose could be delivered. In some such examples, the data storage component 210 can also be updated with any information relevant to the delivery and state of the system 100 during delivery.

For example, the information can include the date and time of delivery, the model and serial number of the system 100, the ambient system and container temperatures, system user input settings, system wireless communications events, system warning or alarm events, user-initiated pauses and durations, user interface events, and/or relevant system parameters settings and measurements during delivery (force, pressure, battery voltage/current, etc.). As such, the data storage component 210 could serve as a record of the delivery (e.g., a delivery "black-box" recording). The data storage component 210 can be designed to easily peel off the reservoir, so that it can be transferred to a monitoring party or HCP for subsequent reading, recording and analysis. Upon completion of delivery, the data storage component 210 can be updated with a "delivery-completed" status, thus preventing the reservoir from being refilled and reused.

The control circuitry 128 is configured to control administration of the drug 20 from the system 100 according to a dosing regimen. This can be accomplished using the data read by the reader 130 from the data storage component 210 and/or data stored in the control circuitry 128. The dosing regimen refers to the specific manner in which the drug is delivered, including (without limitation) formulation, route of administration, dose interval (frequency of dosing), dose amount or volume, delivery rate (flow rate), delivery duration, pauses in delivery, pauses between delivery phases in a multi-drug delivery sequence, and sequencing order of a multi-drug delivery sequence. The dosing regimen can be stored as an algorithm in a memory of the control circuitry 128 that a processor of the control circuitry 128 is configured to execute. The algorithm is stored in the form of one or more sets of pluralities of data points defining and/or representing instructions, notifications, signals, etc. to control administration of a drug from the system 100.

Turning to Figs. 3 to 5, a housing 102 is shown according to one example that can be used to implement a housing of the drug delivery system 100 of Fig. 1. In examples where the drug delivery system 100 is an on-body system, the drug delivery device housing 102 can support, such as house, the actuator 111, the piercing needle 116, the contamination guard 118, the conduit 120, the nozzle insertion mechanism 122, the control circuitry 128, and the user interface 124. In alternative examples, where the drug delivery device is an off-body system, the housing 102 can support, such as house, the actuator 111, the piercing needle 116, the contamination guard 118, the control circuitry 128, and the user interface 124, while the conduit 120 can extend from and outside the housing 102 to the nozzle insertion mechanism 122 that is physically separate from the housing 102.

The housing 102 can be configured to support, such as house, at least a portion up to an entirety of the drug container 200 therein. The housing 102 can be configured to receive, and in some cases removably receive, the drug container 200. Thus, the drug container 200 can be insertable into and/or removable from the housing 102. The housing 102 can define an opening 106 therein that is configured to receive the drug container 200 at least partially or fully into the housing 102. In some examples, the housing 102 can comprise a closure 108, such as a door, that is configured to close at least a portion of the opening 106 to maintain the drug container 200 in the opening 106. The opening 106 can be configured such that the drug container 200 is received into the opening 106 along an insertion direction I. The insertion direction I can be transverse to a longitudinal axis of the drug container 200 and/or a longitudinal axis of the opening 106.

The drug delivery device housing 102 can have a bottom 102a and an opposing top 102b that are opposite from one another along a first direction D₁. In this example, the drug delivery system 100 is an on-body delivery system, and the drug delivery system 100 is configured such that the bottom 102a faces the skin of the patient when the drug delivery system 100 is attached to the patient. The housing 102 can have a first side 102c and a second side 102d opposite from one another along a second direction D₂. The first and second sides 102c and 102d can extend between the bottom 102a and the top 102b. The housing 102 can have a first end 102e and a second end 102f that are opposite one another along a third direction D₃. When the drug container 200 is received in the opening 106, the central axis of the drug container 200 can extend along the second direction D₂. Thus, the axial direction D_{A} can be substantially aligned with the second direction D₂. The first and second ends 102e and 102f can extend between the first and second sides 102c and 102d and between the bottom 102a and bottom 102b. The opening 106 extends into the top 102b and the first end 102e. In alternative examples, the opening can extend into another suitable surface, such as one or more of the bottom 102a, the top 102b, the first end 102e, and the second end 102f.

In this example, the drug delivery system 100 can be used as an on-body delivery system. Thus, the drug delivery system 100 comprises a fastener 104 configured to attach the housing 102 to a patient's body. The fastener 104 can be any suitable fastener for attaching to a patient's body, such as (without limitation) an adhesive, including a tape with adhesive, a strap, or other suitable fastener. The fastener 104 can be supported by the bottom 102a of the housing 102. Additionally, or alternatively, drug delivery system 100 can be used as an off-body delivery system. In such alternatives, the drug delivery system 100 might not employ the fastener 104.

### Drug Delivery System Actuator

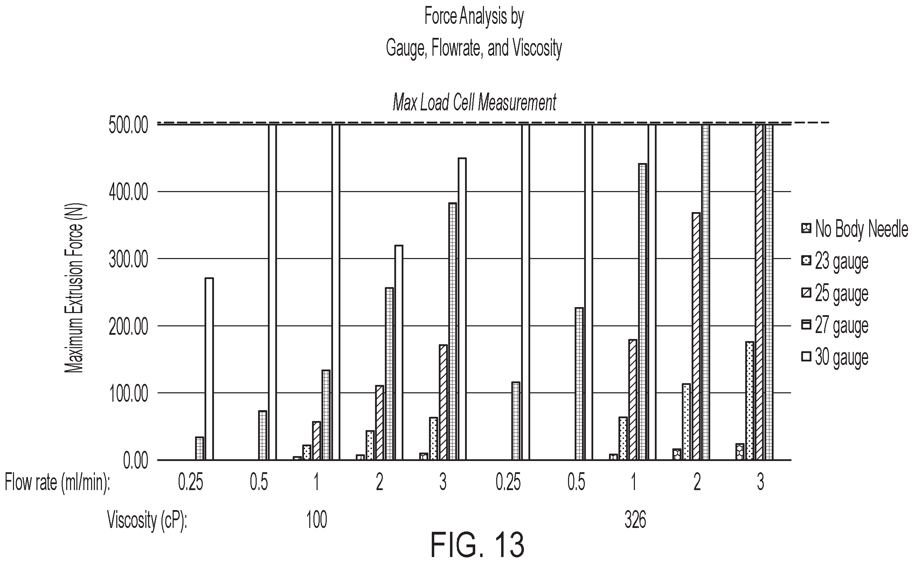

The ability of an on-body delivery system or off-body delivery system to deliver a liquid drug into a patient is dependent on several parameters, including viscosity of the drug, drug type (e.g., solution or suspension), particle size of the drug, needle gauge, and flow rate. Variation of one or more of these parameters can significantly increase or decrease the amount of drive force needed to deliver the liquid drug. Thus, for a given needle gauge and flow rate, higher driving forces are typically needed to deliver higher viscosity drugs (e.g., 100 cp, 200 cp, 300 cp, or even 400 cp), while lower driving forces are typically needed to delivery lower viscosity drugs (e.g., <100 cp). For a given viscosity and flow rate, higher driving forces are typically needed to deliver the drug through a needle having a larger needle gauge, while lower driving forces are typically needed to deliver the drug through a needle having a smaller needle gauge. For a given viscosity and needle gauge, higher driving forces are typically needed to deliver the drug at a higher flow rate, while lower driving forces are typically needed to deliver the drug at a lower flow rate. This problem can be exacerbated when two or more of these factors are varied. For instance, even higher driving forces are typically needed to deliver a higher viscosity drug through a larger gauge needle at a higher flow rate. The forces needed to deliver drugs by varying these parameters can exceed 50N, 75N, 100N, 150N, 200N, 250N, 300N, 350N, or even 400N. This is illustrated by Fig. 13, which shows exemplary delivery forces needed to deliver a 100 centipoise (cP) fluid and a 326 centipoise fluid at different flow rates and different needle gauges from a drug container using a straight plunger rod.

Conventional on-body delivery systems are not typically designed to deliver drugs with such high forces. Rather, conventional on-body delivery systems commonly have driving mechanisms that deliver liquid drugs using relatively low driving forces (e.g., < 30 N). This may be due in part to a lack of need for on-body delivery systems with higher driving forces and/or size and weight constraints of on-body delivery systems. For instance, on-body delivery systems tend to be used with lower viscosity drugs that do not require higher driving forces. Difficulties in delivering these lower viscosity drugs can be often resolved by simply decreasing the flow rate of an existing on-body delivery systems or decreasing the gauge (i.e., increasing the diameter) of the needle of an existing on-body delivery systems. However, decreasing needle gauge can increase patient discomfort.

Conventional on-body delivery systems might also lack higher driving forces due to size and weight preferences of on-body delivery systems. On-body delivery systems are adhered to, or otherwise supported by, the patient's body. Therefore, it is desirable for on-body delivery systems to be light weight and compact in size for patient comfort. However, increasing the driving forces of an on-body delivery system may require increasing the size, and consequently the weight, of the driver used to drive the on-body delivery system to such an extent that the on-body delivery system is no longer suitable for on-body use. In on-body delivery systems in which the drive mechanism is electromechanical, increasing the size of the drive mechanism (e.g., motor) can also require increasing the size of other components, such as a power source (e.g., battery) that powers the on-body delivery system, to accommodate the increased driving forces.

More recently, there has been some interest in delivering drugs having higher viscosities (e.g., 100 cp, 150 cp, 200 cp, 250 cp, 300 cp, 350 cp or even 400 cp) and delivering drugs with larger gauge needles (e.g., 23 gauge, 24 gauge, 25 gauge, 26 gauge, 27 gauge, 28 gauge, 29 gauge, or even 30 gauge) for patient comfort. To satisfy these interests, there is a need for on-body delivery systems that are capable of delivering drugs with higher driving forces (e.g., 50N, 75N, 100N, 150N, 200N, 250N, 300N, 350N, or even 400N), and yet are light weight and compact in size for patient comfort.

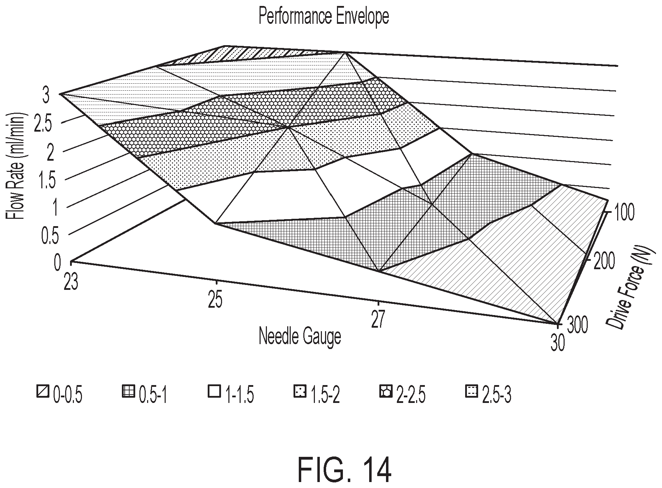

In preferred examples, the actuator 111 of Fig. 1 is capable of driving liquid drugs from the drug container 200 with higher driving forces above 30N, such as above one of 50N, 75N, 100N, 125N, 150N, 175N, 200N, 225N, 250N, 275N, 300N, 325N, 350N, 375N, or 400N. Note that the actuator 111 may still be capable of driving lower forces less than the aforementioned values. The actuator 111 is capable of delivering drugs having higher viscosities such as 100 cp, 125 cp, 150 cp, 175 cp, 200 cp, 225 cp, 250 cp, 275 cp, 300 cp, 325 cp, 350 cp, 375 cp, or 400 cp, in addition to, or alternatively to, lower viscosities less than any of the aforementioned values. Further, the actuator 111 is capable of delivering drugs with larger gauge needles such as 23 gauge, 24 gauge, 25 gauge, 26 gauge, 27 gauge, 28 gauge, 29 gauge, or 30 gauge needles, at lower or higher viscosities. Fig. 14 graphically illustrates a performance envelope observed for a prototype drug delivery device of this disclosure. In this example, a 400N force is applied to a plunger 302 (discussed below), and the surface represents the upper limit of the delivery capabilities of the device at various flow rates, needle gauges, and viscosities.

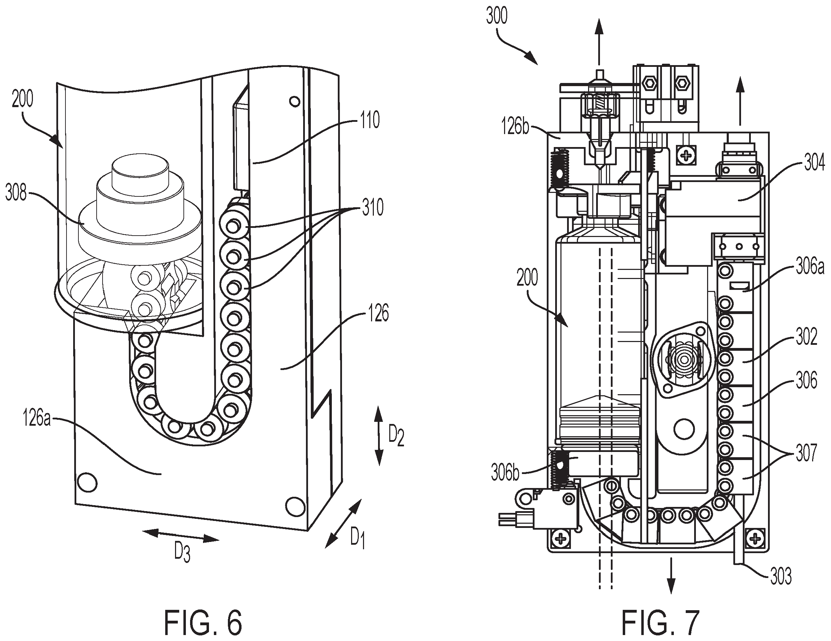

Referring to Figs. 6 and 7, internal features of the drug delivery system 100 are shown, including one example of an actuator 300 that can be used to implement the actuator 111 of the drug delivery system 100. The drug delivery system 100 comprises at least one track 110, and the actuator 300. The actuator 300 comprises a plunger 302 that is configured to be guided by the at least one track 110. The plunger 302 can implement the plunger 112 of Fig. 1. The plunger 302 can be rotationally and/or torsionally fixed relative to a central axis of the plunger 302. Note that the central axis can be curved along a length of the plunger 302.

The actuator 300 comprises a driver 304 that is configured to cause the plunger 302 to translate within the drug container 200 to drive the seal 204 of the drug container 200 to expel the liquid drug from the drug container 200. The driver 304 can implement the driver 114 of Fig. 1. The driver 304 can be any suitable driver that can drive the plunger 302, such as (without limitation) a motor, a spring, a pneumatic actuator, a hydraulic actuator, or an electric actuator. In a preferred example, the driver 304 comprises a motor and the actuator 300 comprises a threaded rod 303. The threaded rod 303 can extend inside at least a portion of the plunger 302 and engage internal threads of the plunger 302. The actuator 304 can be configured such that, when the motor rotates the threaded rod 303, the threads of the threaded rod 303 engage the threads of the plunger 302, thereby causing the plunger 302 to translate within the drug container 200.

The plunger 302 can have a flexible plunger rod 306, a first plunger end 306a and a second plunger end 306b. The second plunger end 306b is configured to engage the seal 204 of the drug container 200. The flexible plunger rod 306 is configured to bend as it drives the second plunger end 306b. The flexible plunger rod 306 can comprise a plurality of links 307 (as shown) that are pivotably connected to one another. In other examples, the flexible plunger rod 306 can additionally or alternatively comprise a flexible material that is capable of bending (e.g., an elongate bar made from a flexible material that bends). The first plunger end 306a can be configured to engage the threaded rod 303. For example, the first plunger end 306a can define the internal threads that engage the threaded rod 303. It will be understood that, in alternative examples, the plunger 302 can be driven by a mechanism other than the motor 304 and threaded rod 303, such as by a magnetic drive.

The at least one track 110 can define a curved path that guides the plunger 302 to bend within a range from 45 degrees to 225 degrees, such as about 90 degrees or preferably about 180 degrees. In some examples, the track 110 can define a U-shaped or J-shaped path for the plunger rod 306. The track can be defined by a recess or opening as shown. Alternatively, the track can be defined by a rail. The flexible plunger rod 306 is configured to bend within the range from 45 degrees to 225 degrees as it is guided around the track 110. By employing the flexible plunger rod 306 and curved track 110, the distance that the plunger rod 306 extends out behind the drug container 200 is significantly reduced compared to comparable devices in which the plunger rod extends straight out behind the drug container. As a result, the overall length of the drug delivery system 100 can be less than that of such comparable devices, resulting in system that is more compact for patient comfort.

Friction resulting from the flexible plunger rod 306 bending along the curved track 110 can result in significant losses of force between the driver 304 and the point where the plunger 302 engages the seal 204 of the drug container 200. The loss can be, for example, a loss of 50 percent of the force or greater. In order to drive a liquid drug from the drug container 200 with the higher driving forces mentioned above, the size of the driver 304 can be increased. However, increasing the size of the driver 304 (and size of associated parts such as power sources) increases patient discomfort. In fact, the size of the driver 304 may need to be increased to such an extent that it would be incompatible for use in an on-body delivery system.

Instead of increasing the size of the driver 304, the interface between the plunger rod 306 and track 110 can be implemented with friction reduction so that a force needed to translate the plunger seal 204 within the container 200 via the plunger rod 306 is no greater than 30%, such as no greater than 25%, 20%, 15%, 10%, or 5% more than a force that would be needed to translate the plunger seal within the container with a straight plunger rod. Use of the friction reduction at the interface between the plunger rod 306 and the track 110 can enable the drug delivery system 100 to be implemented with smaller drivers (and hence smaller power sources) that are more compatible with on-body use, while still having the ability to drive the larger forces discussed above. In some examples, the interface can be implemented with a friction reduction coating that reduces friction between the plunger rod 306 and the track 110. In other examples as shown in Figs. 6 to 12, the interface can comprise friction reduction members 310 such as rollers or bearings that reduce friction at the interface.

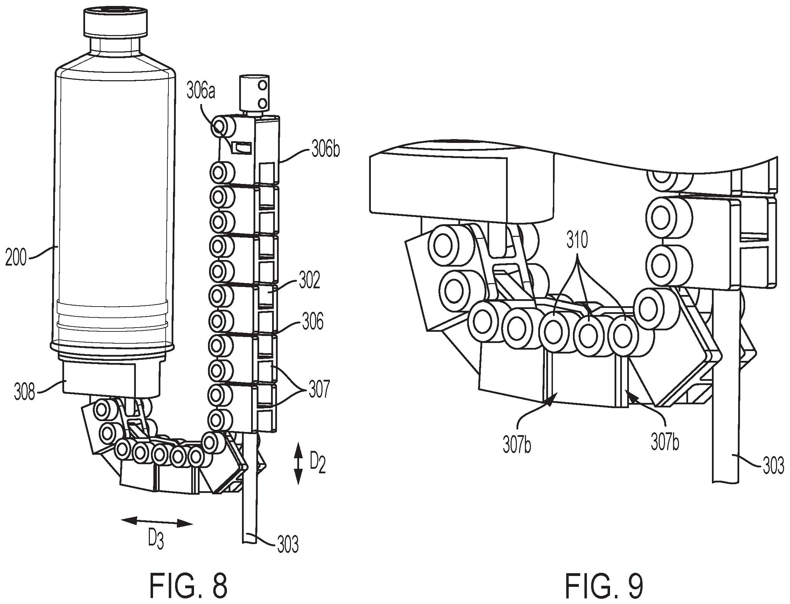

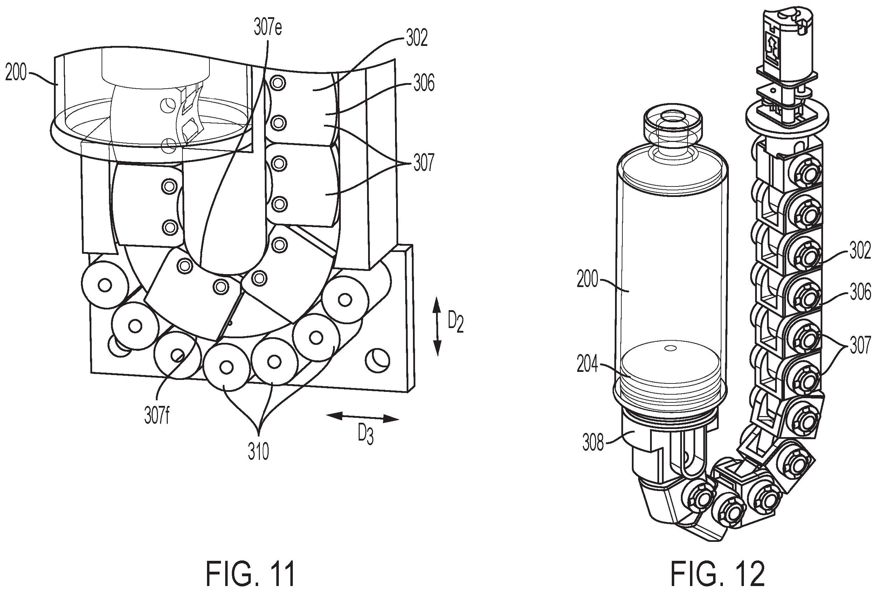

For instance, the drug delivery system 100 can comprise at least one roller or bearing 310 configured to guide the flexible plunger rod 306 as the flexible plunger rod 306 translates along the curved track 110 to limit any loss in force. The flexible plunger rod 306 can support the least one roller or bearing 310 (see e.g., Figs. 8 to 10) such that the least one roller or bearing 310 moves with the flexible plunger rod 306 relative to (e.g., along) the track 110. Alternatively, the track 110 can support the least one roller or bearing 310 (see e.g., Fig. 11) such that plunger rod 306 moves relative to (e.g., along) the at least one roller or bearing 310 and the track 110.

With continued reference to Figs. 6 to 9, the flexible plunger rod 306 can have a first outboard side 306a and a second outboard side 306b. The first and second outboard sides 306a and 306b can be opposite from one another along the first direction D₁. The at least one roller or bearing 310 can comprise one or more rollers or bearings 310 disposed on the first outboard side 306a of the flexible plunger rod 306. In some examples, the at least one roller or bearing 310 can comprise one or more rollers or bearings 310 disposed on the second outboard side 306b of the flexible plunger rod 306. In such examples, the at least one track 110 can comprise a pair of tracks 110. The pair of tracks 110 can be opposite from one another along the first direction D₁. The one or more rollers or bearings 310 of the first outboard side 306a can ride along a first one of the tracks 110, and the one or more rollers or bearings 310 of the second outboard side 306b can ride along a second one of the tracks 110. In alternative examples, the at least one roller or bearing 310 can be disposed between the first and second outboard sides 306a and 306b.

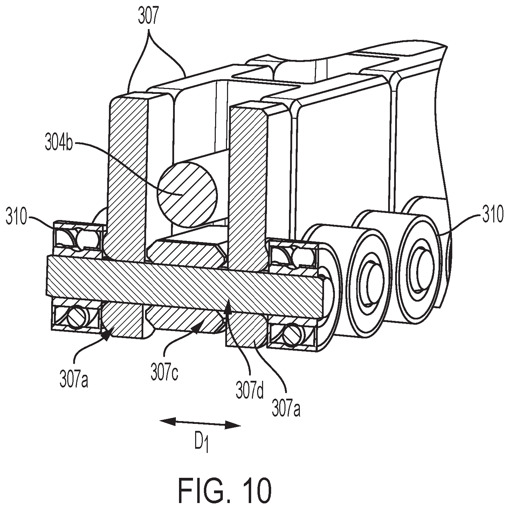

In examples with links 307, such as shown in Figs. 8 to 10, each link 307 can comprise opposing sides 307a, and opposing ends 307b. The opposing sides 307a can be opposite one another along the first direction D₁. The opposing sides 307a can extend between the opposing ends 307b. The links 307 can be disposed adjacent one another such that the opposing ends 307b are arranged end-to-end along the length of the plunger rod 306. Each adjacent pair of links 307 can be connected by a connector 307c. In some examples, each connector 307c can be pivotably coupled to an adjacent pair of links 307 as shown in Fig. 10. In other examples, each connector 307c can be fixedly attached to one end 307b of a respective link 307 and can be received between the opposing sides 307a of an adjacent link 307 as shown in Fig. 12. It will be understood that other configurations of links are contemplated within the scope of this disclosure.

The at least one roller or bearing 310 can be supported by, such as attached to, each of one or more of the links 307, up to all of the links 307. Each roller or bearing 310 can be supported outboard of a side 307a of a respective link 307 as shown. In other examples (not shown), each roller or bearing 310 can be supported between opposing sides 307a of a link 307. In some examples, each respective link 307 can support at least one pair of rollers or bearings 310. The rollers or bearings 310 of each pair can be supported on opposing sides 307a of a respective link 307. Each roller or bearing 310 is configured to roll along the track 110 to limit friction between the plunger rod 306 and the track 110. Each roller or bearing 310 can be supported by a respective axle 307d that extends from or through a respective link 307.

Turning to Fig. 11, in an alternative example, the at least one roller or bearing 310 can be positionally fixed relative to the housing 102 of the drug delivery system 100, and the plunger 306 can be configured to move relative to and along the at least one roller or bearing 310. Each link 307 of the plunger 306 can have an inner end 307e and an outer end 307f that are opposite one another. The inner end 307e and outer end 307f of each link can be opposite one another in a plane defined by the second direction D₂ and third direction D₃. The inner ends 307e can face inwards to define a curve at a bend in the plunger 306 that has a first radius. The outer ends 307f can face outwards to define a curve having at the bend that has a second radius, greater than the first radius. The at least one roller or bearing 310 can be configured to engage the outer ends 307f of the links 307 of the plunger 306. The at least one roller or bearing 310 can disposed along the curve defined by the track 110.

Referring back to Figs. 6 and 7, driving a liquid drug from the drug container 200 with the higher driving forces mentioned above can exert significant opposing forces on the drug delivery system 100, and particularly, on the housing 102. These opposing forces can be applied by the plunger rod 306 at the curve defined by the track 110 at one end, and by the drug container 200 and/or driver 304 at the other end as indicated by the arrows in Fig. 7. These forces can be so significant that, without reinforcements, the forces can cause the housing 102 to burst. Therefore, the drug delivery system 100 can comprise reinforcement structure 126 that is configured to absorb at least some, up to all, of the opposing forces. The reinforcement structure 126 is configured to limit or prevent the opposing forces from being exerted on the housing 102. In some examples, the reinforcement structure can comprise a rigid plate formed from a suitably rigid material such as metal. The reinforcement structure 126 can have a first end 126a that resists outward movement of the at least one curved track at a first end of the drug delivery system along a select direction (e.g., downwards in Figs. 6 and 7), and a second end 126b that resists outward movement of the drug container and/or driver at a second end of the drug delivery system along a direction (e.g., upwards in Figs. 6 and 7) opposite the select direction.

The reinforcement structure 126 can define the at least one track 110. For example, the at least one track 110 can be defined by an opening or recess that extends into or through the reinforcement structure 126. The opening or recess can be configured to receive the at least one roller or bearing 310 therein. In some examples, the reinforcement structure 126 can be disposed on opposing sides of the plunger rod 306. For example, the reinforcement structure 126 can comprise a pair of opposing rigid plates disposed on opposing sides of the plunger rod 306, where each rigid plate defines a corresponding track 110, each corresponding track 110 configured to receive at least one roller or bearing 310.

With reference to Figs. 5 to 7, in operation, a method of delivering a drug to a patient with a drug delivery system 100 can comprise inserting a needle or cannula 101 of the drug delivery system 100 into the patient. The method comprises causing a flexible plunger rod 306 of the drug delivery system 100 to translate along at least one curved track 110 of the drug delivery system 100 such that the flexible plunger rod 306 bends as it translates along the at least one curved track 110 into the drug container 200 of the drug delivery system 100 to drive a liquid drug from the drug container 200 into the patient. At least one roller or bearing 310 of the drug delivery system 100 can guide the flexible plunger rod 306 as the flexible plunger rod 306 translates along the at least one curved track 110. The at least one roller or bearing 310 can ride along the at least one curved track 110 with the flexible plunger rod 306 (e.g., Figs. 6, 7), or the plunger rod 306 can ride along the at least one roller or bearing 310 (e.g., Fig. 11). Additionally or alternatively, the method can comprise causing a flexible plunger rod 306 of the drug delivery system 100 to translate along at least one curved track 110 of the drug delivery system 100 such that the flexible plunger rod 306 bends as it translates along the at least one curved track 110 into the drug container 200 of the drug delivery system 100 to drive a liquid drug from the drug container 200 with a force of at least 50N, such as at least 100 N, 150N, 200N, 250N, 300N, 350N, or 400N. The method can comprise a step of causing a driver 304 to cause the threaded rod 303 to rotate to cause the plunger 306 to translate along the at least one track 110.

### Septum Piercing Assembly

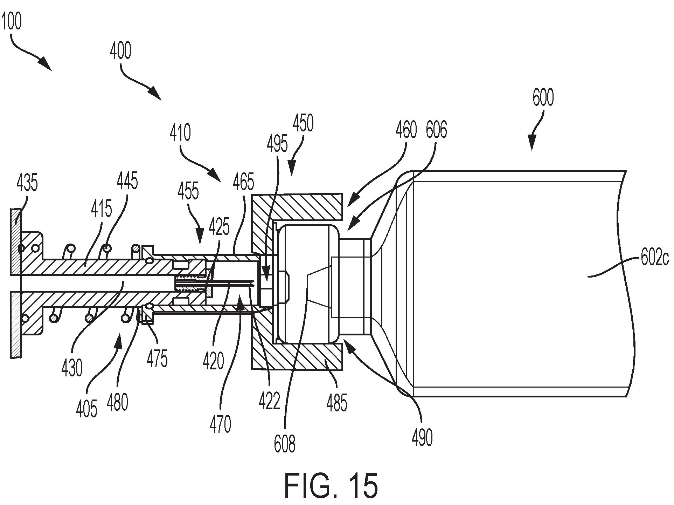

Referring to Fig. 15, internal features of the drug delivery system 100 are shown, including one example of a septum piercing assembly 400. The septum piercing assembly 400 can be configured to removably receive portions of a drug container 600 that can implement and be generally similar to the drug container 200. The septum piercing assembly 400 can implement the septum piercing needle 116 of the drug delivery system 100 The septum piercing assembly 400 can comprise a needle assembly 405 and a biasing assembly 410. The biasing assembly 410 can be movably attached to the needle assembly 405.

The needle assembly 405 can comprise a needle support 415 and a septum piercing needle 420. The septum piercing needle 420 can be received and/or attached to the needle support 415 via any suitable technique such as, for example, a mechanical fastener, a press-fit connection, adhesive, a connecting member, and/or any other suitable technique. For example, the septum piercing needle 420 can be attached to the needle support 415 via a connecting member 425 that can be received in a cavity 430 formed in the needle support 415. The septum piercing needle 420 can include a hollow cavity that can be fluidly connected to the cavity 430 of the needle support 415, which can form a portion of the fluid connection between the septum piercing needle 420 to the nozzle 101 as described above. The septum piercing needle 420 can include a tip 422 for piercing a septum of the drug container 600 as described for example below.

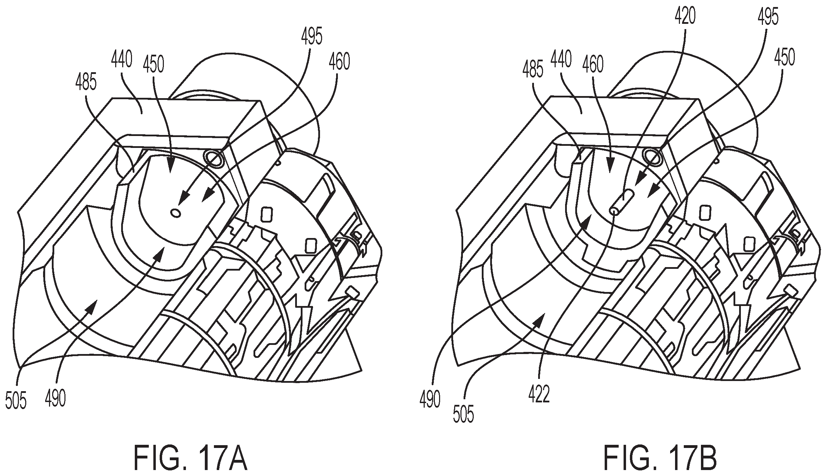

The needle support 415 can be attached to a housing portion 435 of a housing 440, which can implement the housing 102 as further described below regarding Figs. 17A and 17B. Referring again to Fig. 15, the housing portion 435 can be attached to and/or can be an integral part of the housing 440. The housing portion 435 can be fixedly attached to and/or integrated into the housing 440 so that the needle assembly 405 (e.g., including the septum piercing needle 420) remains substantially stationary relative to the housing 440. The needle support 415 and the connecting member 425 can be formed from material that is compatible with the drug 20. The needle support 415 and the connecting member 425 can be formed from any suitable material such as, for example, structural plastic material (e.g., and the material of the housing 440 may be similar to this material). The septum piercing needle 420 can be formed from any suitable material such as, for example, metal material (e.g., stainless steel).

The biasing assembly 410 can comprise a biasing member 445 and a needle shield 450. The biasing member 445 and the needle shield 450 can be movably supported by (e.g., attached to) the needle support 415 of the needle assembly 405.

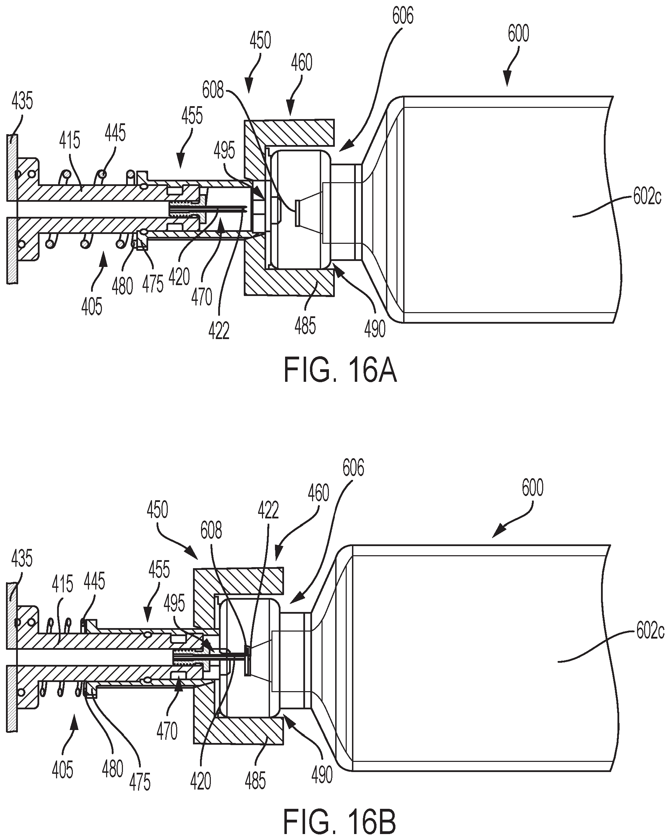

The biasing member 445 can be any suitable member for biasing the needle shield 450 relative to the needle support 415. In some examples, the biasing member 445 can be configured to receive the needle support 415. The biasing member 445 can be a spring such as, for example, a metal spring (e.g., a stainless steel spring) or a spring formed from any other suitable material for deforming to store potential energy that can be used to selectively bias the needle shield 450 as described below. The biasing member 445 can be any other suitable biasing member such as, for example, an elastic or flexible member (e.g., an elastomeric or rubber member) or any other suitable material for biasing the needle shield 450. The biasing member 445 can compress and expand relative to the needle support 415 and the septum piercing needle 420 based on a position of the needle shield 450 and the drug container 600 for example as described below and referring to Figs. 16A and 16B.

Returning to Fig. 15, the needle shield 450 can be any suitable member for receiving a portion of the drug container 600 and selectively shielding the tip 422 of the septum piercing needle 420. The needle shield 450 can be disposed within the housing 440. For example, the needle shield 450 may not extend out of (e.g., outside of) the housing 440. The needle shield 450 can move based on biasing from the biasing member 445 and a position of the drug container 600 for example as described below and referring to Figs. 17A and 17B. Returning to Fig. 15, the needle shield 450 can be formed from material similar to the needle support 415, the connecting member 425, and/or the housing 440 for example as described above.

The needle shield 450 can have any suitable dimensions and configuration for selectively covering and exposing the tip 422 of the septum piercing needle 420 and receiving a portion of the drug container 600. For example as illustrated in Fig. 15, the needle shield 450 can comprise a needle receiving portion 455 and a container receiving portion 460. The needle receiving portion 455 and the container receiving portion 460 can be integrally formed or can be separate portions that are attached using any suitable technique (e.g., bonding, welding such as ultrasonic welding, adhesives, and/or mechanical attachment such as via fasteners).

The needle receiving portion 455 can include a needle housing 465 that can form a needle cavity 470. The needle cavity 470 can selectively contain part or all of the septum piercing needle 420 for example as described further below. The needle housing 465 can be configured and dimensioned to be movably received by the needle support 415. In some examples, the needle support 415 can include one or more recesses or slots for receiving one or more portions of the needle housing 465 (or the needle housing 465 can include one or more recesses or slots for receiving one or more portions of the needle support 415) to guide a movement of the needle housing 465 along the needle support 415. The needle housing 465 can include a biasing member portion 475 (e.g., a protrusion or a lip) that can abut, engage, and/or be attached to an end portion 480 of the biasing member 445. The biasing member 445 can thereby be compressed via contact of the biasing member portion 475 against end portion 480. Biasing member 445 can bias needle shield 450 via contact of the end portion 480 against biasing member portion 475.

The container receiving portion 460 can be configured and dimensioned to receive a cap 606 of the drug container 600. The cap 606 can implement and be generally similar to the cap 206. The cap 606 can include a septum 608 that can implement and be generally similar to the septum 208. The container receiving portion 460 can include one or more receiving protrusions 485 that can form a container recess 490 that can receive (e.g., surround or enclose) some or substantially all of the cap 606. In some examples, the one or more receiving protrusions 485 (e.g., and/or the needle housing 465 of the needle receiving portion 455) can have a curved or annular shape.

Needle shield 450 can include a needle aperture 495 disposed between the container receiving portion 460 and the needle receiving portion 455. The needle aperture 495 can form a passage connecting the needle cavity 470 and the container recess 490. The tip 422 of the septum piercing needle 420 can pass through the needle aperture 495 based on a position of the drug container 600 and/or biasing of the needle shield 450 by the biasing member 445 for example as described further below.

Turning to Figs. 16A and 16B, the needle shield 450, the biasing member 445, and the drug container 600 can move between a pre-pierced position (illustrated in Fig. 16A) and a pierced position (illustrated in Fig. 16B) relative to the needle assembly 405 (e.g., including the septum piercing needle 420) attached to the housing portion 435 of the housing 440. The drug container 600 can be moved from the pre-pierced position (illustrated in Fig. 16A) to the pierced position (illustrated in Fig. 16B) based on operation of a plunger (that can implement the plunger 112) as described further below.

As illustrated in Fig. 16A, in the pre-pierced position, the biasing member 445 can be uncompressed (e.g., storing substantially no potential energy). Although the biasing member portion 475 of the needle shield 450 can be in contact with the end portion 480 of the biasing member 445, the drug container 600 has not been moved by a plunger 500 (e.g., that can implement the plunger 112, as described below) so as to compress the biasing member 445 as described further below. The septum piercing needle 420 can be disposed entirely within the needle cavity 470 of the needle housing 465. The cap 606 can be received in the container recess 490.

As described further below, the plunger 500 can operate to move the drug container 600 from the pre-pierced position illustrated in Fig. 16A to the pierced position illustrated in Fig. 16B. As illustrated in Fig. 16B, in the pierced position, the biasing member 445 can be compressed (e.g., to store potential energy). Based on an operation of the plunger 500 as described further below, the drug container 600 can be moved toward the needle assembly 405. The cap 606 can contact and move the needle shield 450 toward the needle assembly 405 from the pre-pierced position illustrated in Fig. 16A toward the pierced position illustrated in Fig. 16B. As the needle shield 450 is moved toward the needle assembly 405, the needle receiving portion 455 can move along the needle support 415 toward housing portion 435, compressing the biasing member 445, based on the biasing member portion 475 of the needle receiving portion 455 biasing the end portion 480 of the biasing member 445. The tip 422 of the septum piercing needle 420 can pass through the needle aperture 495 as the needle shield 450 moves, until the septum 608 is pierced by the tip 422 of the septum piercing needle 420 as the cap 606 of the drug container 600 disposed in the container recess 490 of the container receiving portion 460 (of the needle shield 450) moves toward the needle assembly 405 (e.g., and into the pierced position illustrated in Fig. 16B). As described above, the needle assembly 405 including the septum piercing needle 420 can remain stationary, based on being attached to the housing portion 435 and the housing 440, as the needle shield 450 and the drug container 600 are moved from the pre-pierced position illustrated in Fig. 16A to the pierced position illustrated in Fig. 16B.

In the pierced position as illustrated in Fig. 16B, portions of the septum piercing needle 420 can be disposed in the needle cavity 470, the needle aperture 495, and the container recess 490 of the needle shield 450 so that the tip 422 pierces the septum 608 of the cap 606 disposed in container recess 490. Biasing member 445 can be compressed. Based on the septum piercing needle 420 including the hollow cavity that can be fluidly connected to the cavity 430 as described above, a fluid connection can be formed from a cavity 602c (that can implement cavity 202c) of the drug container 600 to the nozzle 101 via the septum piercing assembly 400.

Turning to Figs. 17A and 17B, container receiving portion 460 of the needle shield 450 can be disposed in an opening 505 (that can implement opening 106) of the housing 440. The needle shield 450 can be moved between a shielded position as illustrated in Fig. 17A and an exposed position as illustrated in Fig. 17B. The shielded position of the needle shield 450 illustrated in Fig. 17A can correspond to the pre-pierced position of the needle shield 450 illustrated in Fig. 16A. The exposed position of the needle shield 450 illustrated in Fig. 17B can correspond to the pierced position of the needle shield 450 illustrated in Fig. 16B.

In the shielded position of the needle shield 450 illustrated in Fig. 17A, the tip 422 of the septum piercing needle 420 can be shielded (e.g., covered) by the needle shield 450. In the shielded position, the tip 422 may not be disposed in or pass through the needle aperture 495. The septum piercing needle 420 can be substantially entirely (e.g., entirely) disposed in the needle cavity 470 as illustrated in Fig. 16A when the needle shield 450 is in the shielded position illustrated in Fig. 17A. In some examples, a user (e.g., a finger of a user) of the drug delivery system 100 cannot come into contact with the tip 422 when the needle shield 450 is in the shielded position illustrated in Fig. 17A.

In the exposed position of the needle shield 450 illustrated in Fig. 17B, the tip 422 of the septum piercing needle 420 can extend through the needle shield 450. In the exposed position, the tip 422 can be disposed in and/or pass through the needle aperture 495. Portions of the septum piercing needle 420 can be disposed in the needle cavity 470, the needle aperture 495, and the container recess 490 as illustrated in Figs. 16B and 17B when the needle shield 450 is in the exposed position of Fig. 17B (e.g., for clarity for showing the needle shield 450 and the septum piercing needle 420, the drug container 600 is not shown in Fig. 17B in opening 505). In some examples, the drug container 600 having the cap 606 disposed in the container recess 490 can have its septum 608 pierced by the tip 422 extending through the needle aperture 495 as illustrated in the pierced position of Fig. 16B and the exposed position of Fig. 17B (e.g., when the drug container 600 is disposed in the opening 505 when the needle shield 450 is in the exposed position).

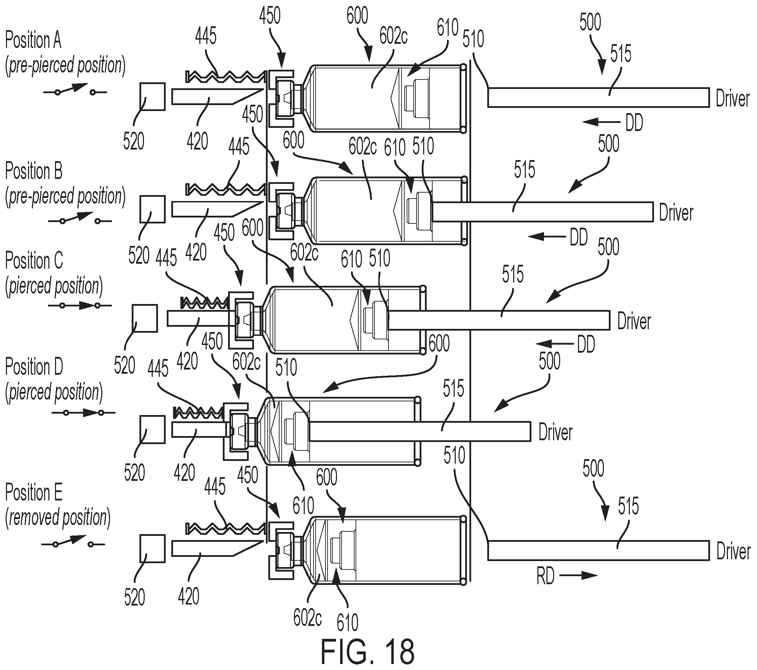

Turning to Fig. 18, an operation of the drug delivery system 100 including the septum piercing assembly 400 will now be described. As schematically illustrated in Fig. 18, the plunger 500 can move the drug container 600 relative to the septum piercing needle 420 of the needle assembly 405. The drug container 600 can include a seal 610 that can implement and be generally similar to seal 204. The plunger 500 can be any suitable plunger for moving the drug container 600 such as described above. For example, the plunger 500 can be generally similar to the plunger 302 described above. The plunger 500 can comprise a plunger end 510 and a flexible plunger rod 515. The flexible plunger rod 515 can be any suitable flexible plunger rod such as the examples described herein. For example, the flexible plunger rod 515 can be generally similar to the flexible plunger rod 306 described above. A driver that can for example be similar to the exemplary drivers described herein (e.g., the driver 114 or the driver 304) can drive the plunger 500.

At Position A as illustrated in Fig. 18, the plunger 500 can be in a pre-engaged position in which the plunger end 510 may not be in contact with the seal 610. The needle shield 450 can be in the shielded position (Fig. 17A). The drug container 600 can be placed in the opening 505 in the pre-pierced position (Fig. 16A).

The driver can operate to move the plunger 500 in a driving direction DD from the pre-engaged position at Position A to an engaged position at Position B. At Position B as illustrated in Fig. 18, the plunger 500 can be in the engaged position in which the plunger end 510 can be in contact with the seal 610. The needle shield 450 can be in the shielded position (Fig. 17A). The drug container 600 can be in the pre-pierced position (Fig. 16A).

The driver can operate to move the plunger 500 in the driving direction DD from the engaged position at Position B to an initial injection (dispensing) position at Position C. At Position C as illustrated in Fig. 18, the plunger end 510 can be in contact with the seal 610. The needle shield 450 can be in the exposed position (Fig. 17B). The drug container 600 can be in the pierced position (Fig. 16B).

In some examples, based on the drug disposed in cavity 602c being a substantially incompressible liquid, movement of the plunger 500 from position B (toward Position C) can move the drug container 600 against the needle shield 450, thereby moving the drug container 600 and the needle shield 450 toward needle assembly 405 and compressing the biasing member 445. For example, the pushing force applied by the plunger 500 against the seal 610 and the drug container 600 can be greater than a spring force of the biasing member 445 so that the biasing member 445 compresses. For example, the pushing force may be similar to the exemplary disclosed driving forces described above regarding actuator 111. For example as described above referring back to Figs. 16A and 16B, the drug container 600 can be moved from the pre-pierced position of Fig. 16A to the pierced position of Fig. 16B in which the tip 422 of septum piercing needle 420 pierces the septum 608 of the drug container 600. The needle shield 450 can also be moved from the shielded position of Fig. 17A to the exposed position of Fig. 17B as the plunger 500 moves the drug container 600 from Position B to Position C. Accordingly at Position C, the plunger 500 can be at the initial injection (dispensing) position, the drug container 600 can be at the pierced position of Fig. 16B, and the needle shield 450 can be at the exposed position of Fig. 17B.

The driver can operate to move the plunger 500 in the driving direction DD from the initial injection (dispensing) position at Position C to a final injection (dispensing) position at Position D. At Position D as illustrated in Fig. 18, the plunger end 510 may be in contact with the seal 610. The needle shield 450 can be in the exposed position (Fig. 17B). The drug container 600 can be in the pierced position (Fig. 16B).

As the plunger 500 moves from Position C to Position D, the plunger end 510 can move the seal 610 within the cavity 602c to deliver the drug contained in the cavity 602c via the septum piercing needle 420. Accordingly (referring to Positions B, C, and D), during part of the travel of the plunger 500, the plunger 500 can push the drug container 600 to pierce the septum 608 against the tip 422 of the septum piercing needle 420 (movement of the plunger 500 from Position B to Position C), and during another part, the plunger 500 can push the seal 610 of the drug container 600 (movement of the plunger 500 from Position C to Position D).

The drug delivery system 100 can include a sensor 520 that can sense when the septum 608 is pierced at Position C so that a delivery of the drug from the drug container 600 can be controlled as the plunger 500 moves from Position C to Position D. The sensor 520 can measure the instant of time (e.g., the precise instant) when the septum 608 is pierced by the tip 422 of the septum piercing needle 420 and when the drug contained in the drug container 600 starts to be delivered. The sensor 520 can be any suitable displacement sensor such as, for example, a position sensor (e.g., linear position sensor) or a laser sensor. In some examples, the sensor 520 can measure a displacement of the needle shield 450. For example, an end stop of the needle shield 450 can occur at a time (e.g., exactly at a time) of fluid flow of the drug 20, which can be in time with (e.g., synchronized with) a limit switch actuation. Also for example, the sensor 520 can comprise a needle sensor such as a microsensor tip or any other suitable sensor for measuring when the septum 608 is pierced. In some examples, the sensor 520 can be integrated into the septum piercing needle. The sensor 520 can communicate with and be controlled by a controller such as, for example, the control circuitry 128 described above.

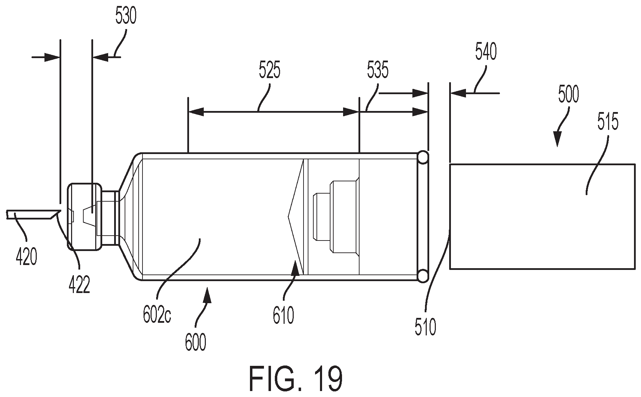

The controller (e.g., the control circuitry 128) can determine when the plunger 500 moves the drug container to Position C and the septum 608 is pierced based on data or signals provided from the sensor 520, at which instant the plunger 500 has completed moving the entire drug container 600 and begins to move the seal 610 within the cavity 602c to deliver the drug via the septum piercing needle 420. (Delivery of the drug between Positions C and D is also schematically depicted in Fig. 18 based on the schematic switch that is shown as open at Positions A, B, and D also being shown as closed at Positions C and D to represent drug delivery.) Starting from the time and/or position that the septum 608 is pierced, the controller (e.g., the control circuitry 128) can determine how much of the drug has been delivered based on the movement of the plunger 500. Accordingly, the amount of drug delivered can be controlled accurately (e.g., dose accuracy is provided) as further described below referring to Fig. 19. In some situations (e.g., weight-based dosing based on a weight of a patient), an entire container of drug may not be delivered (e.g., a portion of the drug container 600 less than the entire drug contained in the drug container can be delivered for a child). In some examples, this control can allow for the drug containers 600 to be of uniform size while being used to deliver different doses as suitable (e.g., the same-sized drug container 600 can be used to deliver a drug to an adult or to a child). This can reduce manufacturing costs by allowing the manufacturer to provide the same-sized drug container 600 to a wide variety of users (e.g., users of different age, weight, and other characteristics affecting dose amount). Also, as described above, the movement of the plunger 500 in the driving direction DD can provide a single mechanism that provides for both piercing of the septum 608 and delivery of the drug from the drug container 600.

The distance that the seal 610 is moved by the plunger 500 within the cavity 602c from Position C to Position D can determine the amount of drug that is delivered from the drug container 600. Referring to Fig. 19, a seal movement distance 525 depicts a distance that the seal 610 moves within the cavity 602c from Position C to Position D. The seal movement distance 525 can correspond to a plunger displacement that provides a corresponding amount of drug to be delivered (e.g., based on dimensions of the cavity 602c such as radius or width dimensions, which in combination with the seal movement distance 525 can correspond to a desired volume to be delivered). The seal movement distance 525 can be any suitable distance for drug delivery such as, for example, between about 10mm and about 60mm (e.g., about 45mm). In some examples, an amount of drug delivered based on the plunger 500 moving the seal 610 from Position C to Position D can be between about 5 mL and about 30 mL. Dose accuracy can thereby be provided based on providing a drug delivery amount based on the seal movement distance 525. In some examples, the position of the seal 610 at Position D can provide a gap within the cavity 602c between the seal 610 and an end of the cavity 602c (e.g., the plunger 500 may not push the seal 610 into a front end of the drug container 600).

In some examples and as illustrated in Fig. 19, any suitable travel distance 530 can be provided for movement between Position B and Position C. For example, the travel distance 530 can be between about 5mm and about 7mm (e.g., about 6mm). An initial engagement distance 535 can be any suitable distance between an end of the drug container 600 and an initial position of the seal 610 within the cavity 602c that is initially contacted by the plunger end 510 (e.g., at Position B). For example, the initial engagement distance 535 can be between about 1mm and about 40mm. An initial clearance distance 540 can be any suitable distance between the plunger end 510 and the end of the drug container 600 (e.g., at Position A) for providing initial clearance for an insertion of the drug container 600 into the opening 505. For example, the initial clearance distance 540 can be about 1 mm or about 2 mm, or can be greater than about 2 mm (e.g., based on dimensions of the housing 440).

Turning back to Fig. 18, the driver can operate to move the plunger 500 in a reverse direction RD, which can be opposite to the driving direction DD, from the final injection (dispensing) position at Position D to the post-engaged position at Position E. The plunger 500 can be moved in the reverse direction RD so that the plunger end 510 is removed from the cavity 602c as the plunger 500 moves from Position D to Position E. Accordingly, the driver can move the plunger 500 in both the driving direction DD and the reverse direction RD.

As the plunger 500 initially begins to move from Position D to Position E, the plunger end 510 can remain in contact with the seal 610. In some examples, the plunger end 510 can remain attached to the seal 610 (e.g., by any suitable technique such as mechanical interlock (e.g., snap fit), suction, or adhesion) as the plunger 500 moves in the reverse direction RD, which can urge or pull both the seal 610 and the drug container 600 in the reverse direction RD away from the needle assembly 405 (e.g., in embodiments in which a biasing member 445 may not be provided). In some examples, the plunger end 510 can remain attached to the seal 610 (e.g., which can be an elastomer seal, which can provide a relatively low release force) based on mechanical interlock such as snap fit. In other examples, as the plunger 500 moves in the reverse direction RD, biasing member 445 can bias needle shield 450 and the drug container 600 in the reverse direction RD so that the plunger end 510 remains in contact with the seal 610 as the plunger 500 moves in the reverse direction RD. For example, the biasing of biasing member 445 as it releases its stored potential energy (e.g., stored from moving from Position B to Position C) can bias the needle shield 450 to keep the drug container 600 pushed against the plunger 500 as the plunger 500 withdraws in the reverse direction RD. When the needle shield 450 reaches the shielded position (e.g., as illustrated in Fig. 17B), the biasing member 445 can stop biasing because it can be partially uncompressed with some or most stored potential energy having been released. In some examples, an end stop (e.g., of the housing 440) can control the position of the needle shield 450 in the shielded position. For example, the end stop can leave some spring force (e.g., about 5N in some embodiments) in the biasing member 445 to ensure that the septum piercing needle 420 fully retracts from the needle friction to the septum 608. The plunger 500 can continue to be moved in the reverse direction RD until the post-engaged position at Position E is reached. At Position E, the seal 610 can remain in the same position as Position D. For example, the seal 610 may be held in place due to a vacuum and, as such, remain in the same position in Position D and Position E. The plunger end 510 can disengage from the seal 610 as the plunger 500 moves from Position D to Position E.

At Position E as illustrated in Fig. 18, the plunger 500 can be in a post-engaged position in which the plunger end 510 may not be in contact with the seal 610 (e.g., similar to as in the pre-engaged position of Position A). The needle shield 450 can be in the shielded position (Fig. 17A). The drug container 600 can be in the post-pierced position, which can be similar to the pre-pierced position illustrated in Fig. 16A. The drug container 600 can then be removed from the opening 505 and replaced as desired during subsequent use of the drug delivery system 100.

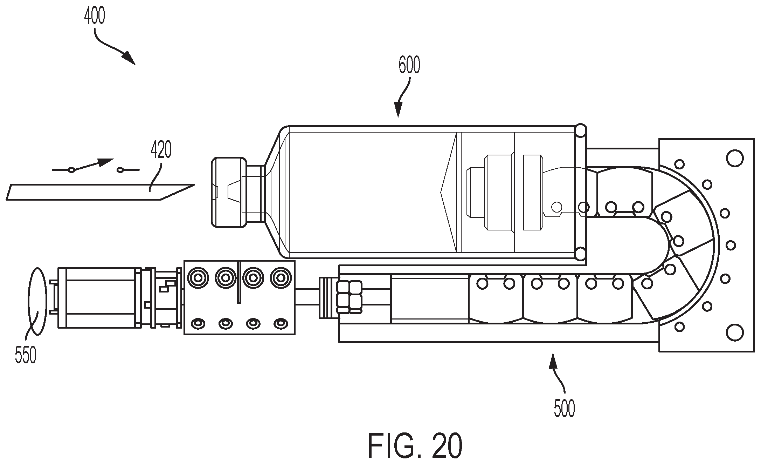

In some examples and referring to Fig. 20, a position control sensor 550 can be used for position control of the septum piercing assembly 400. The position control sensor 550 can be any suitable device for measuring a speed and/or a position of a rotational device such as, for example, rotational components of the driver and/or the plunger 500 (e.g., similar to the rotational components associated with the driver 304 and/or the plunger 302 for example as described above). The position control sensor 550 can be for example a rotary encoder such as a motor rotary shaft encoder. In some examples, the position control sensor 550 can be a magnetic rotary encoder or an optical rotary encoder. The position control sensor 550 can sense position and/or speed of rotational components of the driver and/or the plunger 500 as the plunger 500 is moved (e.g., as described above referring back to Fig. 18), which can be used to measure and control a position of the plunger 500 (e.g., regarding determining the seal movement distance 525). In some examples, an initial position of the plunger 500 can be determined using a position end switch (e.g., that can communicate with and/or be integrated into the control circuitry 128). Also in some examples, displacement of the drug container 600 and/or the plunger 500 can be controlled by any suitable pre-loading mechanism that can be integrated into the housing 440 (e.g., a spring-loaded assembly that can pre-load components of the plunger 500 such as chain components to remove slack).

In some examples, the drug delivery system 100 can comprise a curved track (e.g., similar to track 110), the plunger 500 having the flexible plunger rod 515 and the plunger end 510, the septum piercing needle 420, and the driver (e.g., similar to the driver 114 and/or the driver 304). The driver can be configured to cause the plunger 500 to translate along the curved track such that the flexible plunger rod 515 bends during translation along the curved track and the plunger 500 can be adapted to cause the drug container 600 to translate from a pre-pierced position at which the septum 608 of the drug container 600 is not pierced by the septum piercing needle 420 to a pierced position at which the septum 608 of the drug container 600 is pierced by the septum piercing needle 420. The plunger end 510 can be configured to engage the seal 610 of the drug container 600 to translate the drug container 600 from the pre-pierced position to the pierced position. When the drug container 600 is in the pierced position, movement of the flexible plunger rod 515 can cause the seal 610 of the drug container 600 to move within the drug container 600 to deliver the drug. The biasing assembly 410 can be configured to cause the drug container 600 to move from the pierced position to the removed position in which the septum piercing needle 420 is removed from the drug container 600. The sensor 520 can be configured to sense when the drug container 600 is moved to the pierced position. The controller (e.g., the control circuitry 128) can control delivery of the drug based on the sensor 520 sensing when the drug container 600 is moved to the pierced position in which the delivery of the drug begins. When the drug container 600 is in the pierced position, the controller (e.g., the control circuitry 128) can control the driver to cause the plunger 500 to translate along the curved track to move the plunger end 510 the seal movement distance 525. An amount of the drug delivered via the septum piercing needle 420 can be based on the seal movement distance 525.

In some examples, the drug delivery system 100 can comprise a curved track (e.g., similar to track 110), the plunger 500 having the flexible plunger rod 515 and the plunger end 510, the septum piercing needle 420 spaced from the plunger end 510, the septum piercing needle 420 configured to pierce the septum 608 of the drug container 600, and the driver (e.g., similar to the driver 114 and/or the driver 304). The driver can be configured to cause the plunger 500 to translate along the curved track along the driving direction DD such that the flexible plunger rod 515 bends along the curved track to drive the seal 610 of the drug container 600 to expel a liquid drug from the drug container 600, and to translate along the reverse direction RD, being opposite the driving direction DD, away from the seal 610 after expelling the liquid drug from the drug container 600 is complete. When the plunger 500 is translated along the reverse direction RD, the drug container 600 can move away from the septum piercing needle 420 to unpierce the septum 608. The biasing assembly 410 can be configured to translate the drug container 600 in the reverse direction RD so as to cause the septum piercing needle 420 to unpierce the septum 608. The biasing assembly 410 can comprise the needle shield 450 configured to house the tip 422 of the septum piercing needle 420 in the removed position when the drug container 600 is translated in the reverse direction RD. The biasing assembly 410 can include the biasing member 445 configured to translate the drug container 600 in the reverse direction RD when the plunger 500 is translated along the reverse direction RD. The plunger 500 including the plunger end 510 can be configured to pull the drug container 600 in the reverse direction RD. When the plunger 500 is translated along the reverse direction RD, the plunger 500 can be configured to move the drug container 600 away from the septum piercing needle 420 to unpierce the septum 608. The plunger 500 can be adapted to cause the drug container 600 to translate in the driving direction DD from the pre-pierced position at which the septum 608 of the drug container 600 is not pierced by the septum piercing needle 420 to the pierced position at which the septum 608 is pierced by the septum piercing needle 420. The flexible plunger rod 515 can be translatable between the disengaged position in which the plunger end 510 does not engage the drug container 600 and the engaged position in which the plunger end 510 engages the drug container 600. The driving direction DD and the reverse direction RD can be relative to the housing 440 that supports the septum piercing needle 420 in a stationary position. At least one roller or bearing disposed along the curved track can be configured to guide the flexible plunger rod 515 as the flexible plunger rod 515 translates along the curved track. A reinforcement structure can be configured to resist opposing forces applied by the plunger 500 at a curve defined by the curved track at a first end portion of the drug delivery system 100, and by the drug container 600 or the driver at a second end portion of the drug delivery system 100.

In some examples, the drug delivery system 100 can comprise a curved track (e.g., similar to track 110), the plunger 500 having the flexible plunger rod 515 and the plunger end 510, and the driver (e.g., similar to the driver 114 and/or the driver 304). The driver can be configured to cause the plunger 500 to translate along the curved track along the driving direction DD such that the flexible plunger rod 515 bends along the curved track to drive the seal 610 of the drug container 600 to expel the liquid drug from the drug container 600. The septum piercing needle 420 can be spaced from the plunger end 510, the septum piercing needle 420 configured to pierce the septum 608 of the drug container 600. The needle shield 450 can be configured to be moved between the shielding position, in which the needle shield 450 extends beyond the tip 422 of the septum piercing needle 420, and the exposed position, in which the tip 422 of the septum piercing needle 420 is exposed to allow the septum piercing needle 420 to pierce the septum 608 of the drug container 600. The needle shield 450 can be configured to move from the exposed position to the shielding position after the septum piercing needle 420 is removed from the septum 608 of the drug container 600. The biasing member 445 can bias the needle shield 450 from the exposed position to the shielding position when the plunger 500 is translated along the curved track along the reverse direction RD, opposite the driving direction DD, away from the seal 610. The needle shield 450 can move between the exposed position and the shielding position relative to the housing 440 that can support the septum piercing needle 420 in a stationary position.

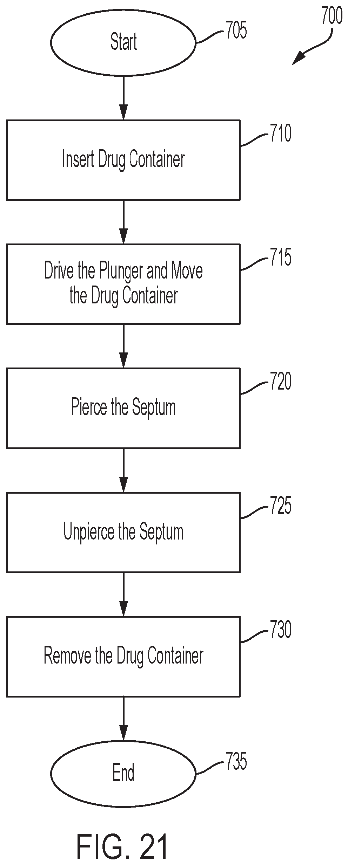

In some examples and referring to Fig. 21, an exemplary disclosed method (e.g., process 700) of using the drug delivery system 100 can comprise the following steps. Process 700 can begin at step 705. At step 710, process 700 can include inserting the drug container 600 into the opening 505 of the housing 440, the housing 440 including the septum-piercing needle 420. At step 715, process 700 can include driving the plunger 500 having the flexible plunger rod 515 and the plunger end 510 along a curved track, and moving the drug container 600 in the driving direction DD toward the septum-piercing needle 420 by pushing the drug container 600 with the plunger end 510. At step 720, process 700 can include piercing the septum 608 of the drug container 600 with the septum-piercing needle 420 based on driving the plunger 500 in the driving direction DD. At step 725, process 700 can include unpiercing the septum 608 of the drug container 600 with the septum-piercing needle 420 based on driving the plunger 500 in the reverse direction RD that is opposite to the driving direction DD. At step 730, process 700 can include removing the drug container 600 from the opening 505 of the housing 440. At step 735, process 700 can end.

In some examples regarding process 700, the driving direction DD and the reverse direction RD can be relative to the housing 440 that supports the septum piercing needle 420 in a stationary position. The needle shield 450 can be moved between the shielding position, in which the needle shield 450 extends beyond the tip 422 of the septum piercing needle 420, and the exposed position, in which the tip 422 of the septum piercing needle 420 is exposed to allow the septum piercing needle 420 to pierce the septum 608 of the drug container 600. The needle shield 450 can be configured to move from the exposed position to the shielding position after the septum piercing needle 420 is removed from the septum 608 of the drug container 600. The needle shield 450 can be biased from the exposed position to the shielding position using the biasing member 445 when the plunger 500 is translated along the curved track along the reverse direction RD.

### Drug Container Replaceability During Use

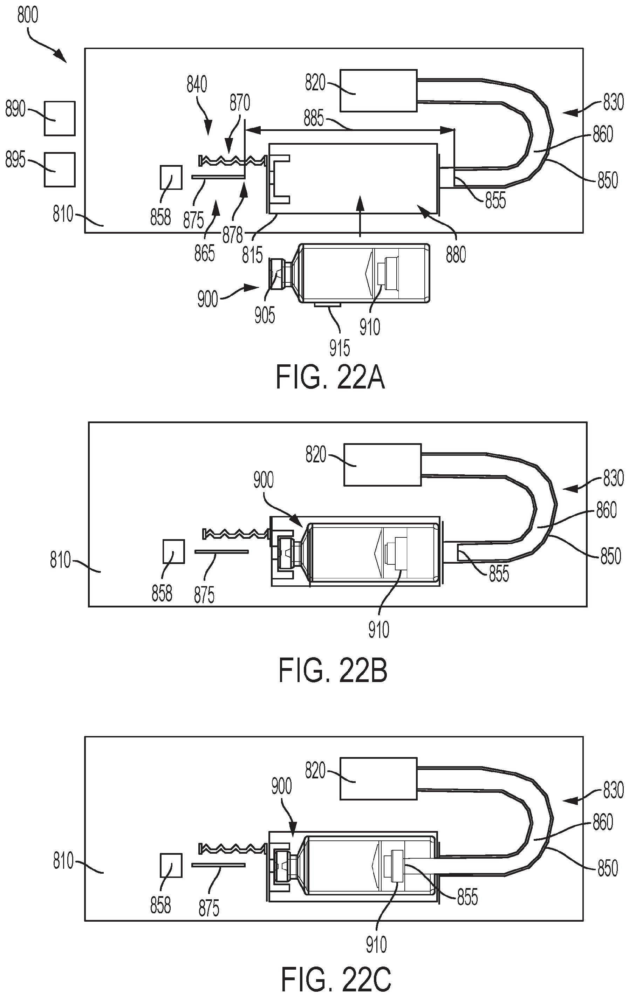

Referring to Fig. 22A, another example of the exemplary disclosed drug delivery system is shown. A drug delivery system 800 can comprise components similar to the above disclosed exemplary systems. The drug delivery system 800 can comprise a housing 810, a driver 820, a plunger 830, and a septum piercing assembly 840. The housing 810 can house the driver 820 that can drive the plunger 830 relative to the septum piercing assembly 840.

The housing 810 can implement the housing 102 and can be similar for example to the housing 102 and/or the housing 440 described above. The housing 810 can include an opening 815. The opening 815 can implement the opening 106 and can be similar to the opening 106 and/or the opening 505. The housing 810 can include a curved track 850 that can implement the curved track 110. The housing 810 can include a sensor 858 that can be similar to the sensor 520.

The driver 820 can implement the driver 114 and can be similar to the driver 114 for example as described above. The plunger 830 can implement the plunger 112 and can be similar for example to the plunger 112 and/or the plunger 500 described above. The plunger 830 can comprise a plunger end 855 and a flexible plunger rod 860. The plunger end 855 can be similar for example to the second plunger end 306b and/or the plunger end 510. The flexible plunger rod 860 can be similar for example to the flexible plunger rod 306 and/or the flexible plunger rod 515. The driver 820 can operate to cause the plunger 830 to translate along the curved track 850 such that the flexible plunger rod 860 bends during translation along the curved track 850 and the plunger end 855 drives a plunger seal of the exemplary disclosed drug container to expel a liquid drug from the drug container.

The septum piercing assembly 840 can implement the septum piercing needle 116. The septum piercing assembly 840 can be similar for example to the septum piercing needle 116 and/or the septum piercing assembly 400. The septum piercing assembly 840 can comprise a needle assembly 865 and a biasing assembly 870. The needle assembly 865 can be similar for example to the needle assembly 405. The needle assembly 865 can comprise a septum piercing needle 875. The septum piercing needle 875 can implement the septum piercing needle 116 and can be similar for example to the septum piercing needle 116 and/or the septum piercing needle 420.

A space 880 can be defined between the plunger end 855 and the septum piercing needle 875, the space 880 configured to receive the exemplary disclosed drug container when the drug container is received into the housing 810 through the opening 815. The space 880 can be associated with a distance 885 that can be defined from an edge of a tip 878 of the septum piercing needle 875 to the plunger end 855. The distance 885 can define the distance between the edge of the tip 878 of the septum piercing needle 875 to the plunger end 855 when the plunger 830 is in a pre-use position and the flexible plunger rod 860 is in a disengaged position for example as illustrated in Fig. 22A. In some examples, the distance 885 can be greater than or equal to a height of the drug container that is to be received in the opening 815. For example, the distance 885 can increase or decrease proportionally to the height of the drug container that is to be received in the opening 815 (e.g., the distance 885 can be driven by the total length of the drug container). In some examples, the distance 885 can vary based on the size of the drug container to be received in the opening 815 and/or can be defined in terms of the clearance on both ends of the drug container (e.g., the clearance between the needle and the drug container plus the length of the drug container plus the clearance on the plunger side). For example, if the drug container happens to be about 75mm long, the distance 885 can be about 77mm (e.g., the drug container length plus about 1mm needle recess to the exemplary disclosed spring plate, and about 1mm clearance on the plunger side). In other examples, the distance 885 can be greater than or equal to a distance from a second end (e.g., 202b) of the drug container to an end of the plunger seal. It is also contemplated that, in some examples, the drug container can be inserted in an angle such that the plunger end 855 can be received in a distal end of the drug container (e.g., either spaced from the plunger seal or in contact with the plunger seal).

A drug container 900 can be received through the opening 815 into the space 880 of the housing 810. The drug container 900 can implement the drug container 200 and can be similar for example to the drug container 200 and/or the drug container 600. The drug container 900 can include a septum 905 that can implement the septum 208 and can be similar to the septum 208 and/or the septum 608. The drug container 900 can also include a plunger seal such as a seal 910 that can implement the seal 204 and can be similar to the seal 204 and/or the seal 610. In some examples, the seal 910 can be a plunger seal such as a plugged seal. The drug container 900 can contain a liquid drug, and the liquid drug can be expelled from the drug container 900 similar for example to as described above regarding the drug container 200 and/or the drug container 600. The drug container 900 can also include a data storage component 915. The data storage component 915 can be a near-field communication tag or an RFID tag such as, for example, as described above.

The drug delivery system 800 can also include control circuitry 890 that can be similar to control circuitry 128 and a reader 895 that can be similar to the reader 130. The reader 895 can be configured to read the data storage component 915 of the drug container 900. The control circuitry 890 can be configured to receive data from the data storage component 915 (e.g., a near-field communication tag or an RFID tag of the drug container) via the reader 895 and/or to control the driver 820 based on the data.

Figs. 22A through 22G illustrate an exemplary operation of the drug delivery system 800. Figs. 22A and 22B illustrate receiving the drug container 900 through the opening 815 of the housing 810 into the space 880 of the housing 810. The plunger 830 is illustrated in the pre-use position, and the flexible plunger rod 860 is illustrated in the disengaged position. The plunger end 855 and the flexible plunger rod 860 may not be engaged with and/or disposed in the drug container 900 in the pre-use / disengaged position. For example, the plunger end 855 can be disposed outside of the drug container 900 when the plunger 830 is in the pre-use position in which the space 880 is defined between the septum piercing needle 875 (e.g., the edge of the tip 878 of the septum piercing needle 875) and the plunger end 855. The opening 815 may be selectively blocked using a lockable door (e.g., similar to closure 108) that can prevent tampering with the drug container during septum piercing and delivery. The lockable door can be lockable by a user or can be automatically lockable upon the occurrence of an event, such as initiation of an injection.

Figs. 22B and 22C illustrate driving the plunger 830 having the flexible plunger rod 860 and the plunger end 855 along the curved track 850. The plunger 830 and the flexible plunger rod 860 can be moved from the pre-use / disengaged position illustrated in Fig. 22B to an engaged position of the plunger 830 and the flexible plunger rod 860 as illustrated in Fig. 22C. In the engaged position illustrated in Fig. 22C, the plunger end 855 can be moved from outside of the drug container 900 (e.g., illustrated in Fig. 22B) to inside or within the drug container 900 to engage the plunger seal 910 of the drug container 900 when the plunger 830 is in the engaged position as illustrated in Fig. 22C. In some examples, the drug container 900 can be inserted at an angle so that the plunger 830 is received in the drug container 900 in the pre-use / disengaged position, but the plunger end 855 is not in engagement with the plunger seal 910. In other examples, the drug container 900 can be received so that the plunger 830 is entirely outside of the drug container 900 in the pre-use / disengaged position.

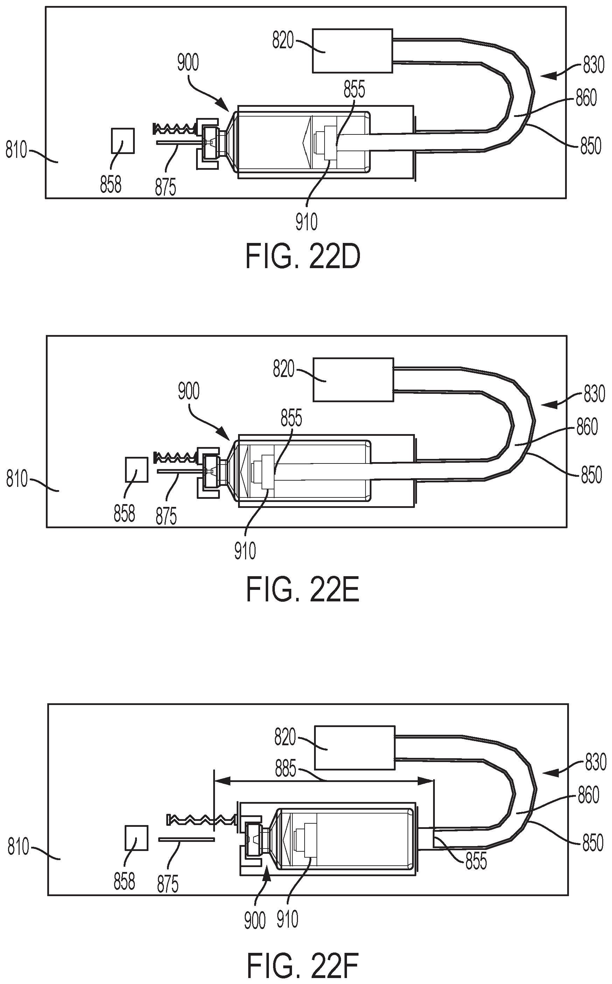

Figs. 22C and 22D illustrate moving the drug container 900 from a pre-pierced position to a pierced position similar for example to as described above regarding Figs. 16A, 16B, and 18. Sensor 858 can sense when the septum 905 is pierced similar for example to the operation of sensor 520 described above.

Figs. 22D and 22E illustrate driving the seal 910 using the driver 820 and the plunger 830 to expel the liquid drug from the drug container 900. The plunger 830 can be driven so that the flexible plunger rod 860 bends during translation along the curved track 850 and the plunger end 855 can drive the seal 910 of the drug container 900 to expel the liquid drug from the drug container 900. For example, the plunger 830 can be moved from an initial injection position to a final injection position to drive the seal 910 similar for example to as described above regarding Fig. 18.

Figs. 22E and 22F illustrate returning the plunger 830 to the pre-use / disengaged position. After expelling the liquid drug from the drug container 900, the plunger 830 can be returned to the pre-use / disengaged position based on the driver 820 driving the plunger 830. The plunger end 855 can be spaced from the septum piercing needle 875 so as to define the space 880 between the plunger end 855 and the septum piercing needle 875 as illustrated in Fig. 22F.

Referring to Figs. 22B through 22F, a driving speed of the plunger 830 can be fixed or it can be varied by the driver 820 based on control by the control circuitry 890. For example at Figs. 22D to 22E (delivery of the drug), the plunger 830 can move at a relatively slower speed in order to deliver the drug at the desired flow rate. However for example at Figs. 22B to 22D (septum piercing) and at Figs. 22E to 22F (withdrawal of the plunger 830), the plunger 830 can move at a relatively faster (e.g., a much faster speed) so that use of the system by the user is not unduly delayed. For example, the plunger 830 can move at a first speed at Figs. 22D to 22E (delivery of the drug), at a second speed at Figs. 22B to 22D (septum piercing), and at a third speed at Figs. 22E to 22F (withdrawal of the plunger 830), with the first speed (delivery of the drug) being slower than the second speed (septum piercing) and/or the third speed (withdrawal of the plunger 830). Also for example, at a first speed at Figs. 22D to 22E (delivery of the drug), the driving speed of the plunger 830 can be constant or variable (e.g., noncontinuous or intermittent delivery).

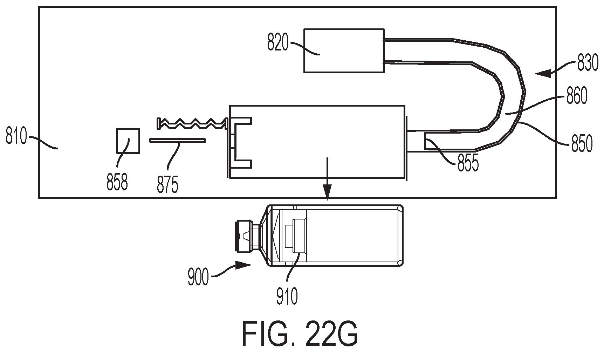

Figs. 22F and 22G illustrate removing the drug container 900 from the space 880 through the opening 815. Removal of the drug container 900 can include toolless removal and/or non-destructive removal of the drug container 900. For example, toolless removal can include removal of the drug container 900 without the use of tools (e.g., by the fingers of a user without using tools). Also for example, non-destructive removal can include removal of the drug container 900 from the housing 810 without damage to the drug container 900. Also for example, non-destructive removal can include removal of the drug container 900 from the housing 810 without damage to the housing 810.

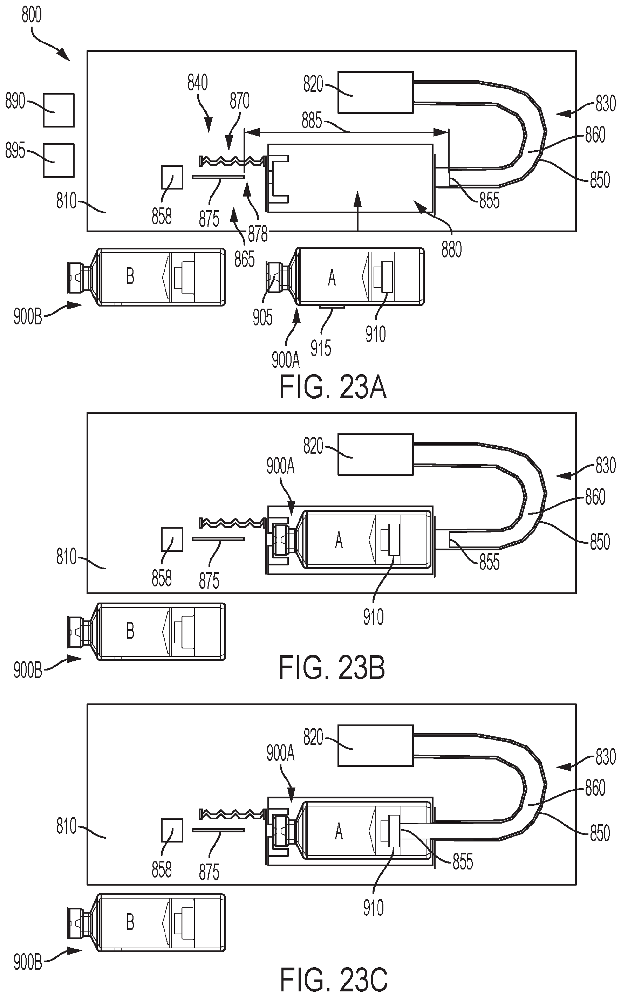

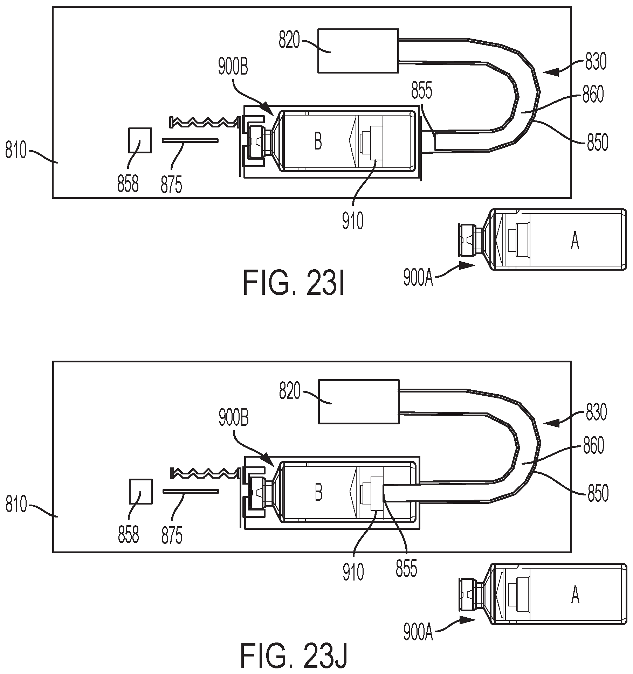

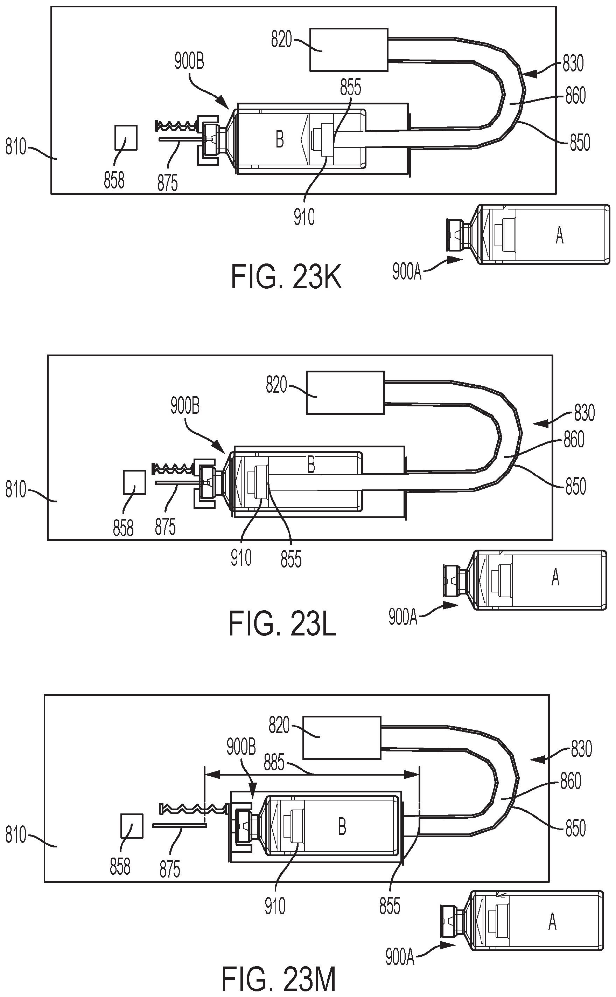

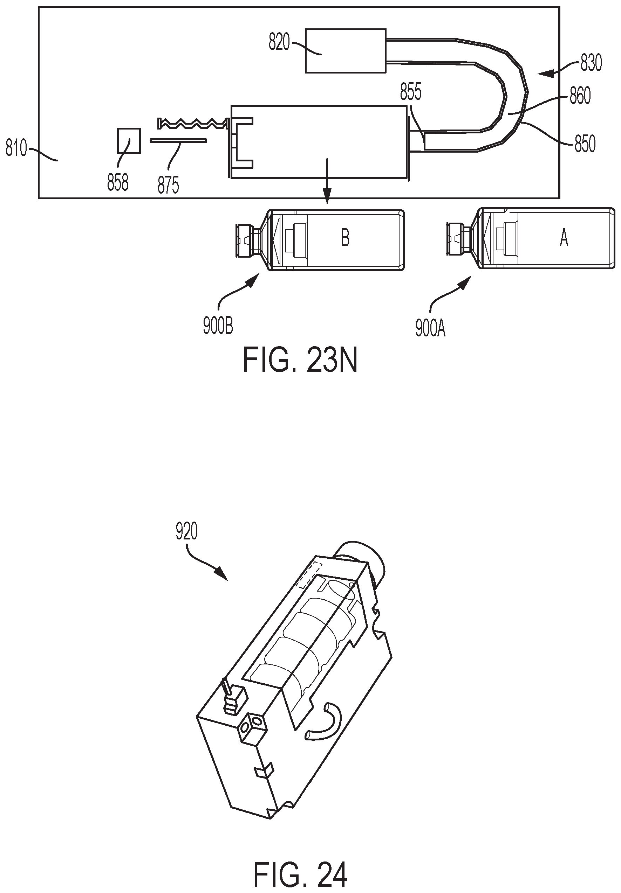

Figs. 23A through 23N illustrate another exemplary operation of the drug delivery system 800. A drug container 900A ("A") and a drug container 900B ("B") can be selectively removably inserted into and removed from the housing 810. The drug container 900A and the drug container 900B can be similar to the drug container 900. The drug container 900A and the drug container 900B can contain a same liquid drug or a different liquid drug. For example, the drug container 900A can contain a liquid drug that has a same or different viscosity than a second liquid drug contained in the drug container 900B.

As illustrated in Figs. 23A through 23G, the drug container 900A can be received in the space 880 of the housing 810, a liquid drug can be expelled from the drug container 900A, and the drug container 900A can be removed from the housing 810 similarly to as described above regarding the drug container 900 in Figs. 22A through 22G. The drug container 900B can be set aside when the drug container 900A is utilized as illustrated in Figs. 23A through 23G.

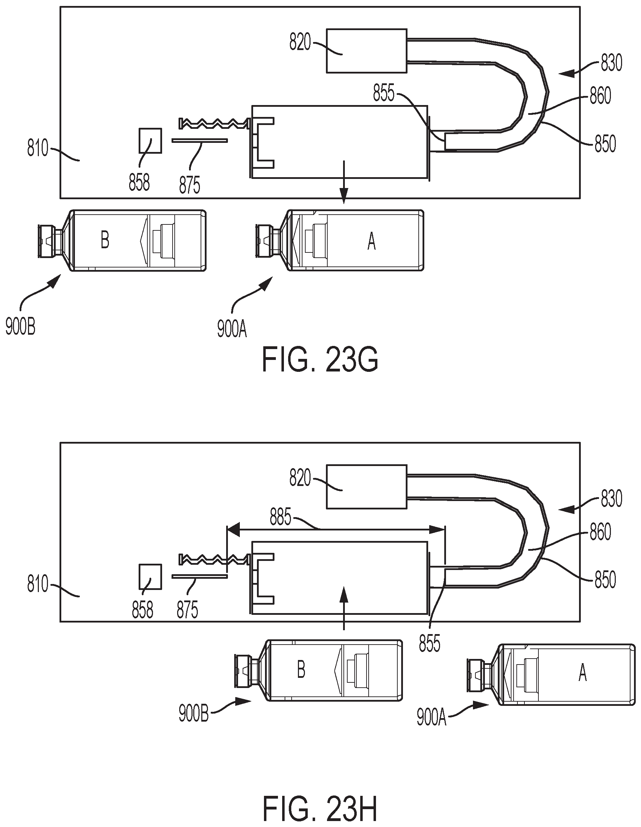

As illustrated in Figs. 23G and 23H, after the drug container 900A is removed, the drug container 900B can be received into the space 880 of the housing 810 through the opening 815. The drug container 900A can be used or expended (e.g., the liquid drug can be partially or substantially entirely expended) before it is removed. The drug container 900B can be new or unused (e.g., the liquid drug can be substantially entirely unused) or partially expended (e.g., the liquid drug can be partially or substantially expended, or the drug container 900B can contain less liquid drug than initially provided by a manufacturer).

When a drug container (e.g., the drug container 900A) is removed, back pressure from the drug residing in the patient and/or from the patient's skin can cause the drug and/or blood to flow back into the exemplary disclosed device. The exemplary disclosed drug delivery system can accordingly include a backflow prevention valve. Referring back to Fig. 1, an example of a backflow prevention valve 132 is illustrated. The backflow prevention valve 132 can be any suitable valve for preventing backflow such as, for example, a check valve.

As illustrated in Figs. 23H through 23N, the drug container 900B can be received in the space 880 of the housing 810, a liquid drug can be expelled from the drug container 900B, and the drug container 900B can be removed from the housing 810 similarly to as described above regarding the drug container 900 in Figs. 22A through 22G. The previously used or expended drug container 900A can be set aside when the drug container 900B is utilized as illustrated in Figs. 23H through 23N. Additional drug containers having configurations and containing liquid drugs that can be the same as or different from the drug containers 900A and 900B may be utilized following the drug containers 900A and 900B, similar to as described above regarding Figs. 23A through 23N.

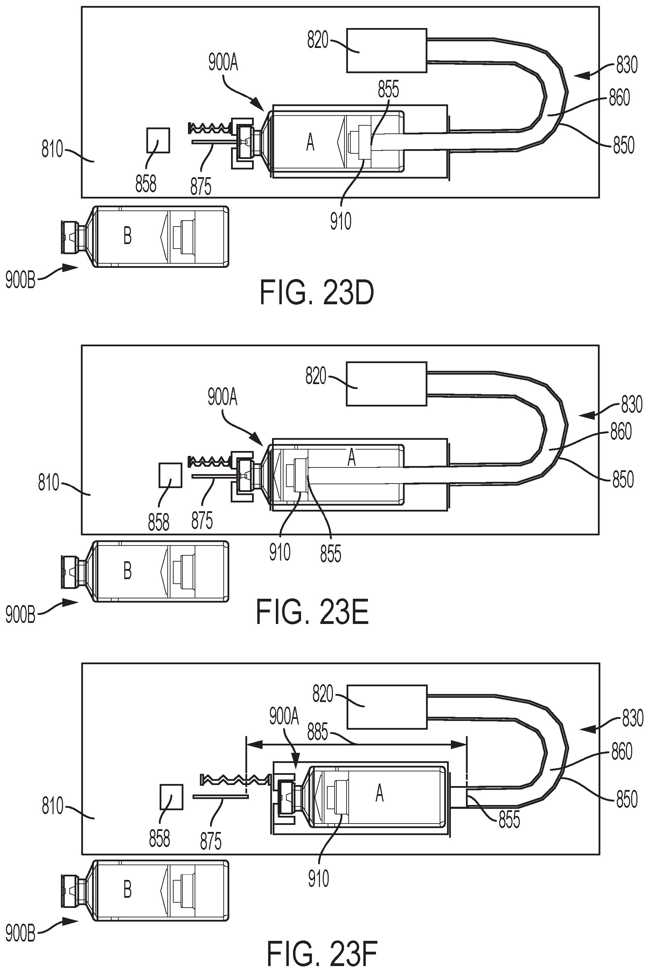

According to some examples, driving the seal 910 of the drug container 900A can include expelling a first amount of the liquid drug from the drug container 900A as illustrated in Figs. 23D and 23E. Driving the seal 910 of the second drug container 900B can include expelling a second amount of the second liquid drug from the second drug container 900B (e.g., as illustrated in Figs. 23K and 23L) that can be different from the first amount of the liquid drug from the drug container 900A. The first amount and the second amount can be any suitable amount such as, for example, between about 1mL and about 50mL, between about 1mL and about 30 mL, between about 20mL and about 50mL, or between about 15mL and about 35mL.

According to some examples, driving the seal 910 of the drug container can include expelling the liquid drug from the drug container 900A having a first volume capacity as illustrated in Figs. 23D and 23E. Driving the seal 910 of the second drug container 900B can include expelling the second liquid drug from the drug container 900B having a second volume capacity (e.g., as illustrated in Figs. 23K and 23L) that can be different from the first volume capacity of the drug container 900A. In some examples, drug containers having the first volume capacity and the second volume capacity can be filled to the same level or may be filled to different levels. The first volume capacity and the second volume capacity can be any suitable volume capacity such as, for example, between about 5mL and about 50mL, between about 10mL and about 50mL, between about 20mL and about 50mL, or between about 30mL and about 50mL.

According to some examples, driving the seal 910 of the drug container 900A can include expelling the liquid drug having a first viscosity from the drug container 900A as illustrated in Figs. 23D and 23E. Driving the seal 910 of the second drug container 900B can include expelling the second liquid drug having a second viscosity from the drug container 900B (e.g., as illustrated in Figs. 23K and 23L) that can be different from the first viscosity of the liquid drug of the drug container 900A. The first viscosity and the second viscosity can be any suitable viscosity such as, for example, between about 1cP and about 300cP, between about 1cP and about 200cP, between about 100cP and about 300cP, or between about 100cP and about 200cP.

According to some examples, the control circuitry 890 can control the operation of the drug delivery system 800 differently for different drug containers (e.g., differently for each of the drug container 900A and the drug container 900B). The control circuitry 890 can control the driver 820 to drive the plunger 830 with a first driving force when the drug container 900A is received in the space 880. The control circuitry 890 can also control the driver 820 to drive the plunger 830 with a second driving force that is different from the first driving force when the drug container 900B is received in the space 880. The first driving force and the second driving force can be any suitable driving force such as, for example, the exemplary disclosed driving forces described above.

According to another example, the control circuitry 890 can control the driver 820 to drive the plunger 830 to drive the seal 910 with a first force and/or speed to expel the liquid drug from the drug container 900A at a first flow rate when the drug container 900A is received in the space 880. The control circuitry 890 can also control the driver 820 to drive the plunger 830 to drive the seal 910 with a second force and/or speed to expel the liquid drug from the drug container 900B at a second flow rate that is different from the first flow rate when the drug container 900B is received in the space 880. The first flow rate and the second flow rate can be any suitable flow rate such as, for example, between about 0.1mL/min and about10mL/min.

Referring to Fig. 24, another example of the exemplary disclosed drug delivery system is shown. Drug delivery system 920 can be generally similar to the drug delivery system 800 and can provide for a prefilled and/or preassembled configuration. For example, the exemplary disclosed cartridge can be similar to the drug container 900 and can be integrated into the exemplary disclosed housing that can be similar to the housing 810. In some examples, the cartridge can have a capacity of up to 50mL (e.g., 10 mL, 20 mL, 30mL, 40mL, or 50mL).

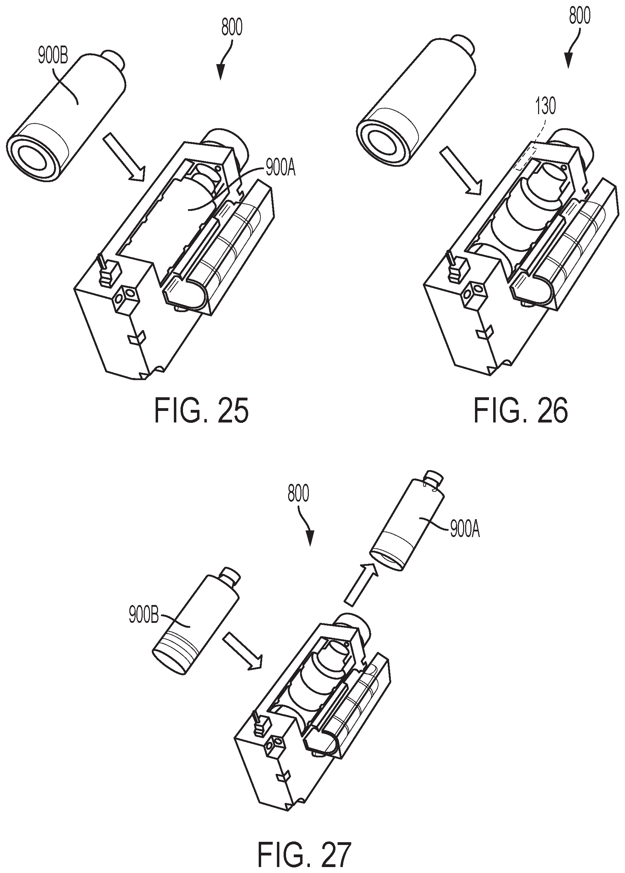

Referring to Fig. 25, a use case of the drug delivery system 800 is shown. In this use case, the drug container 900A and the drug container 900B can include the same drug. For example in this use case, the drug delivery system 800 can deliver relatively larger volumes of the drug, via separate containers 900A and 900B, rather than one larger container that would involve the size of the system 800 being larger to accommodate the larger container. This use case can reduce the weight and size of the system, because a relatively larger and/or heavier drug container may not be utilized. The drug delivery system 800 can provide for on-body cartridge replacement by a user. For example, a user may load additional cartridges during use (e.g., after removing expended cartridges for example as described above). In some examples, the cartridges can have a capacity of 20mL or more (e.g., 10 mL, 20 mL, 30mL, 40mL, or 50mL). For example, additional capacity can be provided by the user adding additional cartridges. The drug delivery system 800 can provide an increased injection volume by allowing the use of multiple cartridges in place of relatively large cartridges that may be impractical for use (e.g., impractical for on-body use). The drug delivery system 800 is shown in a state similar to the one illustrated in Fig. 23F, where a drug in a first drug container has been expelled and a second drug container is ready to be placed in the drug delivery system 800.

Referring to Fig. 26, another use case of the drug delivery system 800, weight-based dosing, is shown. In this case, the drug contained within the drug container can have a first volume, and the system can deliver a second volume of the drug that can be less than or equal to the first volume, depending for example on a patient's body weight. The system can receive an input and adjust based on this input to deliver the second volume. The input can be from the data storage component or another input from e.g., the user or a remote computing system. For example, the reader 130 and/or the reader 895 can be used to read the exemplary disclosed data storage components of cartridges to dispense a desired volume (e.g., for example as described above). In some examples, the drug delivery system 800 can be used for example to provide weight-based dosing based on data provided by the exemplary disclosed data storage components that may be similar to the data storage component 210 and/or the data storage component 915. The drug delivery system 800 is shown in a state similar to the one illustrated in Fig. 22A, Fig. 23A, or 23H, where a drug container is ready to be placed in the drug delivery system 930.

Referring to Fig. 27, another use case of the drug delivery system 800 is shown. In this use case, multiple doses of different drugs can be provided (e.g., the drug containers 900A and 900B can have different drugs). The drug delivery system 800 can thereby provide a multi-therapy use. For example, a user can sequentially change cartridges during use (e.g., a first drug of the drug container 900A may be dispensed, followed by a second drug of the drug container 900B that can be different from the first drug, and so on, similar to as described above regarding Figs. 23A through 23N). The drug delivery system 800 can provide sequential delivery of two or more drug types within a single session. The drug delivery system 800 is shown in a state similar to the one illustrated in Fig. 23H, where the first drug container 900A with a first drug has been removed from the drug delivery system 800 and the second drug container 900B with the second drug is ready to be placed in the drug delivery system 800.

Some or all features of the exemplary disclosed configurations can be used in the exemplary disclosed drug delivery system. For example, a given exemplary disclosed drug delivery system can include some or substantially all features of the drug delivery system 100, the drug delivery system 800, the drug delivery system 920, the drug delivery system 925, the drug delivery system 930, and/or the drug delivery system 935. Moreover, a given exemplary disclosed drug delivery system can implement one or more, such as any combination of two or more, of the use cases described herein. For example, a given exemplary disclosed drug delivery system can perform weight-based dosing for two different drugs in two separate containers, or can deliver multiple doses of a first drug with separate containers with one or more doses of a second drug in a separate container(s).

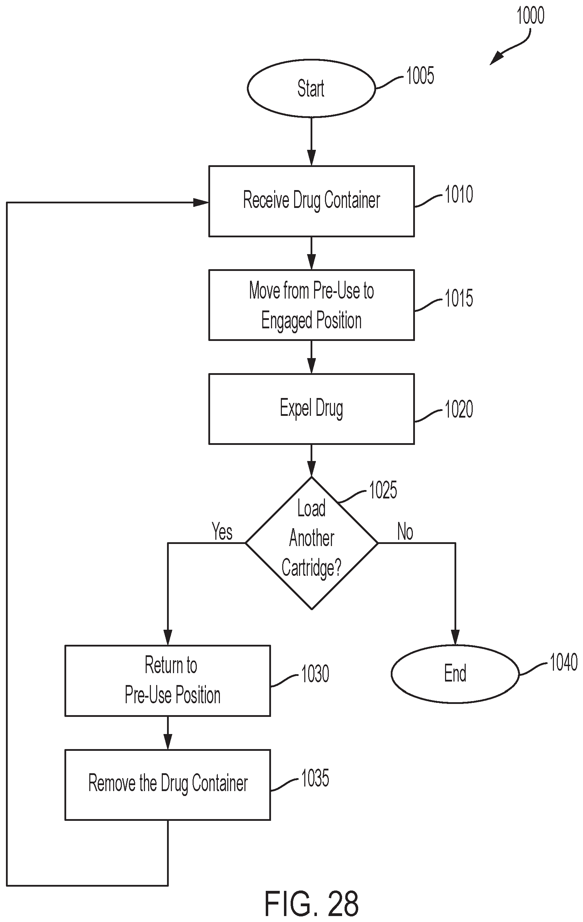

In some examples and referring to Fig. 28, an exemplary disclosed method (e.g., process 1000) of using the drug delivery system 800 can comprise the following steps. Process 1000 can begin at step 1005. At step 1010, process 1000 can include receiving the drug container 900 through the opening 815 of the housing 810 and into the space 880. At step 1015, process 1000 can include driving the plunger 830 having the flexible plunger rod 860 and the plunger end 855 along the curved track 850 from the pre-use / disengaged position to the engaged position for example as described above regarding Figs. 22B and 22C. Returning to Fig. 28 at step 1020, process 1000 can include expelling the liquid drug for example as described above regarding Figs. 22D and 22E. At step 1025 it can be determined whether another drug container is to be loaded (e.g., based on a user and/or the control circuitry 890). For example, the drug container 900A can be utilized at steps 1010 through 1020 as described above at Figs. 23A through 23E. If at step 1025 it is determined that another drug container is to be loaded, process 1000 can proceed to step 1030.

Returning to Fig. 28 at step 1030, process 1000 can include returning to the pre-use position by driving the plunger 830 having the flexible plunger rod 860 and the plunger end 855 along the curved track 850 from the engaged position to the pre-use / disengaged position for example as described above regarding Figs. 22E and 22F (e.g., or Figs. 23E and 23F). Returning to Fig. 28 at step 1035, process 1000 can include removing the drug container 900 from the space 880 through the opening 815 of the housing 810. Process 1000 can then return to step 1010.

For example, the drug container 900B can then be utilized in steps 1010 through 1020 as described in Figs. 23H through 23L. At step 1025, it can then again be determined whether or not another drug container is to be loaded. As many iterations as desired can be performed if additional cartridges are determined at step 1025 to be loaded. If at step 1025 it is determined that no additional cartridges are to be loaded, process 1000 can end at step 1040. Process 1000 can also apply to the other exemplary disclosed drug delivery systems such as, for example, drug delivery system 100, drug delivery system 925, drug delivery system 930, and drug delivery system 935.

In some examples, the drug delivery system 800 (e.g., and/or the other exemplary disclosed drug delivery systems) can comprise the housing 810 defining the opening 815 therein configured to receive the drug container 900 into the housing 810, the curved track 850, the plunger 830 having the flexible plunger rod 860 and the plunger end 855, the driver 820 configured to cause the plunger 830 to translate along the curved track 850 such that the flexible plunger rod 860 bends during translation along the curved track 850 and the plunger end 855 drives the seal 910 of the drug container 900 to expel the liquid drug from the drug container 900, and the septum piercing needle 875 configured to pierce the septum 905 of the drug container 900. The plunger end 855 can be spaced from the septum piercing needle 875 when the plunger 830 is in the pre-use position so as to define the space 880 between the plunger end 855 and the septum piercing needle 875, the space 880 configured to receive the drug container 900 when the drug container 900 is received into the housing 810 through the opening 815. The flexible plunger rod 860 can be configured so that the plunger end 855 is disposed outside of the drug container 900 when the plunger 830 is in the pre-use position. The flexible plunger rod 860 can be configured to move the plunger end 855 from outside of the drug container 900 when the plunger 830 is in the pre-use position to engage the drug container 900 when the plunger 830 is in the engaged position. The plunger end 855 can be configured to engage the seal 910 of the drug container 900 in the engaged position. The plunger end 855 can be configured to be disposed inside of the drug container 900 to engage the seal 910 of the drug container 900 in the engaged position. The plunger end can be configured to engage the seal 910, which can be a plugged seal, in the engaged position. The control circuitry 890 can be configured to receive data from a near-field communication tag or an RFID tag of the drug container 900 via a reader 895, the control circuitry 890 configured to control the driver based on the data.

In some examples, the drug delivery system 800 (e.g., and/or the other exemplary disclosed drug delivery systems) can comprise the curved track 850, the plunger 830 having the flexible plunger rod 860 and the plunger end 855, and the driver 820 configured to cause the plunger 830 to translate along the curved track 850 such that the flexible plunger rod 860 bends during translation along the curved track 850 and the plunger end 855 drives the seal 910 of the drug container 900 to expel the liquid drug from the drug container 900. The flexible plunger rod 860 can be translatable between the disengaged position in which the plunger end 855 does not engage the drug container 900 and the engaged position in which the plunger end 855 engages the drug container 900. In the disengaged position, the plunger end 855 can be disposed outside of the drug container 900. In the engaged position, the plunger end 855 can be disposed inside of the drug container 900.

In some examples, the drug delivery system 800 (e.g., and/or the other exemplary disclosed drug delivery systems) can comprise the housing 810 defining the opening 815 therein configured to receive the first drug container 900A and the second drug container 900B into the housing 810, the first drug container 900A containing the first liquid drug and the second drug container 900B containing the second liquid drug, the control circuitry 890, the curved track 850, the plunger 830 having the flexible plunger rod 860 and the plunger end 855, and the driver 820 configured to cause the plunger 830 to translate along the curved track 850 such that the flexible plunger rod 860 bends during translation along the curved track 850. The opening 815 can be configured to removably receive the first drug container 900A, and the control circuitry 890 can be configured to cause the driver 820 to drive the plunger end 855 to drive the seal 910 of the first drug container 900A to expel the first liquid drug from the first drug container 900A. The opening 815 can be configured to removably receive the second drug container 900B, and the control circuitry 890 can be configured to cause the driver 820 to drive the plunger end 855 to drive the seal 910 of the second drug container 900B to expel the second liquid drug from the second drug container 900B. The first amount of the first liquid drug expelled from the first drug container 900A can be different from a second amount of the second liquid drug expelled from the second drug container 900B. The first liquid drug can be the same as the second liquid drug. The first liquid drug can be different from the second liquid drug. The first viscosity of the first liquid drug of the first drug container 900A can be different from the second viscosity of the second liquid drug of the second drug container 900B. The first volume capacity of the first drug container 900A can be different from the second volume capacity of the second drug container 900B. The control circuitry 890 can be configured to cause the driver 820 to drive the plunger 830 with the first driving force when the first drug container 900A is received in the space 880, and the second driving force that can be different from the first driving force when the second drug container 900B is received in the space 880. The control circuitry 890 can be configured to cause the driver 820 to drive the plunger 830 to drive the seal 910 of the first drug container 900A to expel the first liquid drug from the first drug container 900A at the first flow rate, and the control circuitry 890 can be configured to cause the driver 820 to drive the plunger 830 to drive the seal 910 of the second drug container 900B to expel the second liquid drug from the second drug container 900B at the second flow rate that is different from the first flow rate. The control circuitry 890 can be configured to cause the driver 820 to drive the plunger 830 based on at least one of the first data received from the first near-field communication tag or the first RFID tag of the first drug container 900A or the second data received from the second near-field communication tag or the second RFID tag of the second drug container 900B. The first data can identify at least one of the volume of the first liquid to expel, the flow rate to expel the first liquid, the viscosity of the first liquid, or the driving force to drive the plunger 830 to expel the first liquid. The second data can identify at least one of the volume of the second liquid to expel, the flow rate to expel the second liquid, the viscosity of the second liquid, or the driving force to drive the plunger 830 to expel the second liquid. Removably receiving the first drug container 900A and the second drug container 900B in the space 880 can include non-destructive removal of the first and second drug containers. Removably receiving the first drug container 900A and the second drug container 900B in the opening can include toolless removal of the first and second drug containers.

In some examples, the exemplary disclosed method can comprise receiving the drug container 900A through the opening 815 of the housing 810 into the space 880 of the housing 810, the space 880 defined between the septum piercing needle 875 of the housing 810 and the plunger end 855 of the plunger 830 disposed in the housing 810. The exemplary disclosed method can also comprise driving the plunger 830 having the flexible plunger rod 860 and the plunger end 855 along the curved track 850, the plunger 830 and the curved track 850 being situated inside the housing 810, the plunger 830 being arranged to interact with the drug container 900A situated in the space 880 inside the housing 810, and the septum-piercing needle 875 configured to pierce the septum 905 of the drug container 900A. The exemplary disclosed method can further comprise driving the plunger 830 so that the flexible plunger rod 860 bends during translation along the curved track 850 and the plunger end 855 drives the seal 910 of the drug container 900A to expel the liquid drug from the drug container 900A. The exemplary disclosed method can additionally comprise disposing the plunger end 855 outside of the drug container 900A when the plunger 830 is in the pre-use position in which the space 880 is defined between the septum piercing needle 875 and the plunger end 855. The exemplary disclosed method can also comprise moving the plunger end 855 from outside of the drug container 900A when the plunger 830 is in the pre-use position to engage the seal 910 of the drug container 900A when the plunger 830 is in the engaged position. The exemplary disclosed method can further comprise, after expelling the liquid drug from the drug container 900A, returning the plunger 830 to the pre-use position by spacing the plunger end 855 from the septum piercing needle 875 so as to define the space 880 between the plunger end 855 and the septum piercing needle 875, removing the drug container 900A from the space 880 through the opening 815, and after the drug container 900A is removed, receiving the second drug container 900B into the space 880 of the housing 810 through the opening 815.

The exemplary disclosed method can additionally comprise driving the plunger 830 so that the flexible plunger rod 860 bends during translation along the curved track 850 and the plunger end 855 drives the seal 910 of the second drug container 900B to expel the second liquid drug from the second drug container 900B. Driving the seal 910 of the drug container 900A can comprise expelling the first amount of the liquid drug from the drug container 900A and driving the seal 910 of the second drug container 900B can comprise expelling the second amount of the second liquid drug from the second drug container 900B that can be different from the first amount. Driving the seal 910 of the drug container 900A can comprise expelling the liquid drug from the drug container 900A having the first volume capacity and driving the seal 910 of the second drug container 900B can comprise expelling the second liquid drug from the second drug container 900B having the second volume capacity that can be different from the first volume capacity. Driving the seal 910 of the drug container 900A can comprise expelling the liquid drug that can be the same as the second liquid drug expelled by driving the seal 910 of the second drug container 900B. Driving the seal 910 of the drug container 900A can comprise expelling the liquid drug that is different from the second liquid drug expelled by driving the seal 910 of the second drug container 900B. Driving the seal 910 of the drug container 900A can comprise expelling the liquid drug having the first viscosity and driving the seal 910 of the second drug container 900B can comprise expelling the second liquid drug having the second viscosity that is different from the first viscosity. The exemplary disclosed method can also comprise driving the plunger 830 with the first driving force when the drug container 900A is received in the space 880, and driving the plunger 830 with the second driving force that can be different from the first driving force when the second drug container 900B is received in the space 880. The exemplary disclosed method can further comprise driving the plunger 830 to drive the seal 910 of the drug container 900A to expel the liquid drug from the drug container 900A at the first flow rate, and driving the plunger 830 to drive the seal 910 of the second drug container 900B to expel the second liquid drug from the second drug container 900B at the second flow rate that can be different from the first flow rate. The exemplary disclosed method can additionally comprise moving the plunger end 855 of the plunger 830 from the septum piercing needle 875 in the pre-use position prior to receiving the exemplary disclosed drug container into the space 880 of the housing 810.

### Sterile Barrier

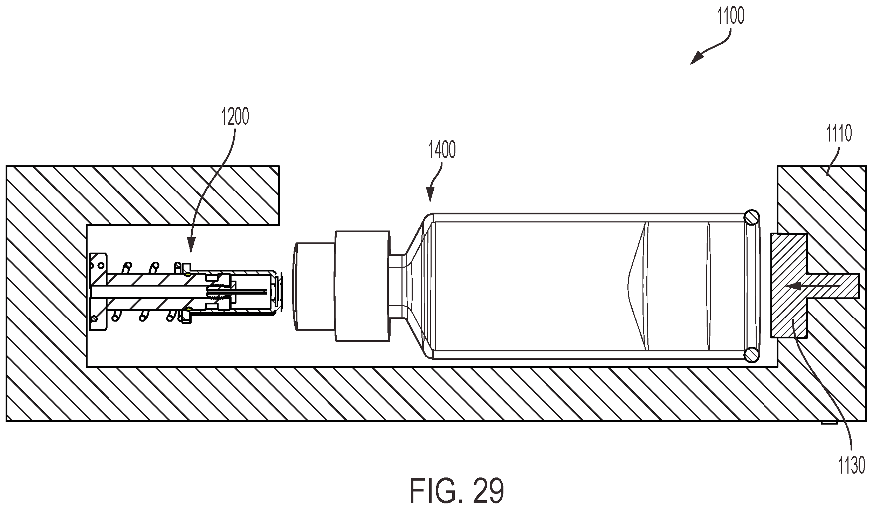

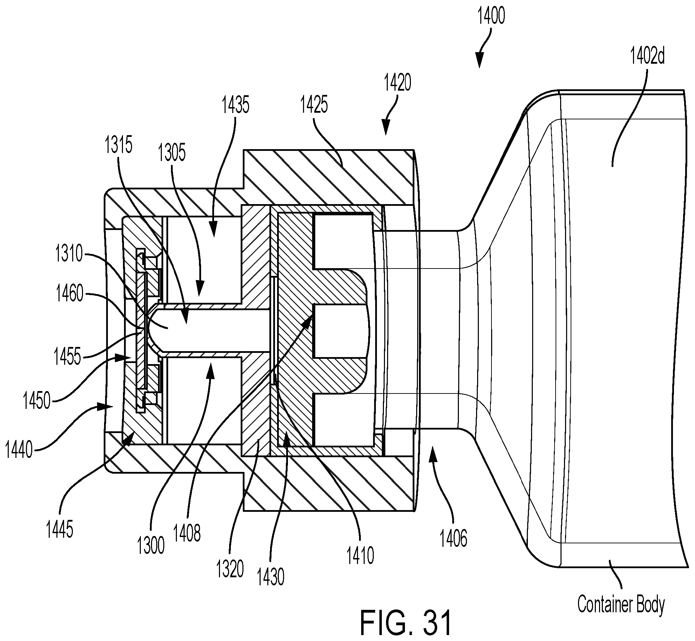

Referring to Figs. 29-31, another example of the exemplary disclosed drug delivery system is shown. A drug delivery system 1100 can comprise components similar to the above disclosed exemplary systems. The drug delivery system 1100 can comprise a septum piercing assembly 1200 and a piercing member 1300 (e.g., illustrated in Fig. 31) that may operate together for the sterile withdrawal of a liquid drug from a drug container 1400. The drug delivery system 1100 can be configured to operate with components (e.g., a driver, a plunger, and control circuitry that can be disposed in a housing) similar to as described above regarding the drug delivery systems 100 and 800 (e.g., as described regarding Figs. 1-11, 18, and 22A through 23N). In some examples, the drug delivery system 1100 can be housed in a housing 1110 that can implement the housing 102 and can be similar, for example, to the housing 102, the housing 440, and/or the housing 810 described above. Also in some examples, a plunger 1130, which can implement the plunger 500 and/or the plunger 830, can cause the drug container 1400 to move similarly to, for example, as described above regarding Figs. 18 and 22A through 23N.

The septum piercing assembly 1200 can implement the septum piercing needle 116. The septum piercing assembly 1200 can be similar, for example, to the septum piercing needle 116, the septum piercing assembly 400, and/or the septum piercing assembly 840. In some examples, the septum piercing assembly 1200 can comprise a needle assembly 1205 that can be similar to the needle assembly 405 and a biasing assembly 1210 that can be similar to the biasing assembly 410. The needle assembly 1205 can comprise a needle support 1215 that can be similar to the needle support 415 and a septum piercing needle 1220 that can be similar to the septum piercing needle 420. The septum piercing needle 1220 can include a tip 1222 that can be similar to the tip 422 for piercing a septum of the drug container 1400. The needle support 1215 can be attached to a housing portion 1235 of the housing 1110.

The biasing assembly 1210 can comprise a biasing member 1245 that can be similar to the biasing member 445 and a needle shield 1250 that can be similar to the needle shield 450. The biasing member 1245 and the needle shield 1250 can be movably supported by (e.g., movably attached to) the needle support 1215 of the needle assembly 1205. The needle shield 1250 can selectively shield the tip 1222 of the septum piercing needle 1220. The needle shield 1250 can move based on biasing from the biasing member 1245 and/or a position of the drug container 1400 similarly to as described above regarding Figs. 17A and 17B.

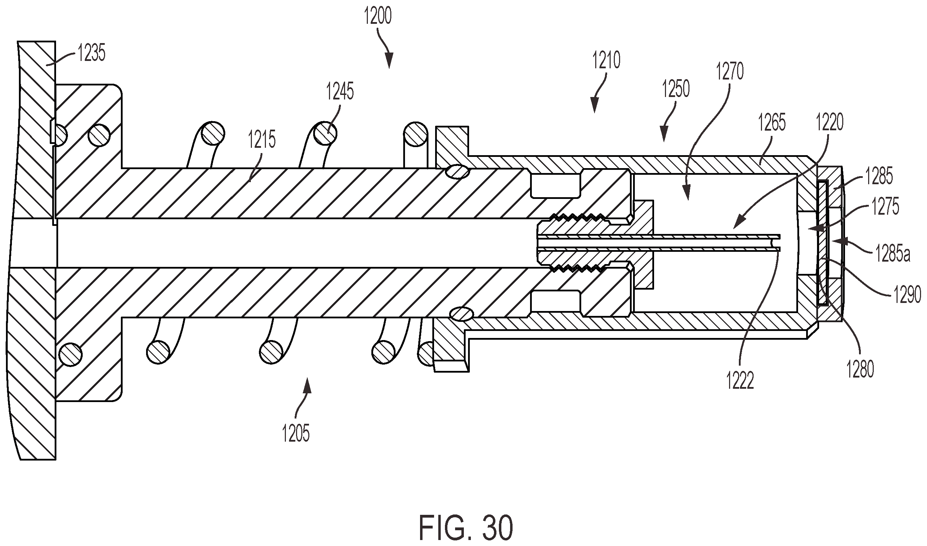

Returning to Fig. 30, the needle shield 1250 can include a needle housing 1265 that can be similar to the needle housing 465 and that can form a needle cavity 1270 that can be similar to the needle cavity 470. The needle cavity 1270 can selectively contain part or substantially all of the septum piercing needle 1220 when the drug delivery system 1100 is in the unpierced configuration. The needle housing 1265 can be configured and dimensioned to be movably received by the needle support 1215 similarly to as described above regarding the needle housing 465 and the needle support 415. The needle housing 1265 of the needle shield 1250 can include a needle aperture 1275 through which the septum piercing needle 1220 can selectively extend, for example, as described further below. In some examples, the tip 1222 can be aligned (e.g., centered) with the needle aperture 1275. The needle shield 1250 can further include a shield pierceable element 1280. The shield pierceable element 1280 can be a sterile barrier. The shield pierceable element 1280 can be attached to the needle housing 1265 via any suitable technique such as, for example, mechanical fastening, adhesive, attachment components, and/or any other suitable technique. In some examples, an attachment member 1285 that can be formed from similar material as the needle housing 1265 can be attached to or integrally formed with the needle housing 1265. For example, the attachment member 1285 can receive the shield pierceable element 1280 to thereby attach the shield pierceable element 1280 to the needle housing 1265, for example, as illustrated in Fig. 30 (e.g., with the attachment member 1285 having an aperture 1285a that can be similarly dimensioned and/or aligned with the needle aperture 1275). The shield pierceable element 1280 can thereby be attached to the needle housing 1265 via any suitable technique so that the shield pierceable element 1280 can cover substantially all (e.g., or some) of the needle aperture 1275. The shield pierceable element 1280 can seal the needle cavity 1270 to reduce or substantially block admission of germs into the needle cavity 1270. In some examples, the needle cavity 1270 can be formed between the shield pierceable element 1280, inside walls (e.g., interior wall surfaces) of the needle housing 1265, and surfaces of the needle support 1215 facing the needle cavity 1270. The needle cavity 1270 can thereby serve as a sterile chamber (e.g., can be sterilized at a time of manufacture).

The shield pierceable element 1280 can be a self-healing member. In some examples, the shield pierceable element 1280 can re-close after being pierced. The shield pierceable element 1280 can be formed from any suitable self-healing (e.g., reclosing or resealing) material such as, for example, elastomer material. In some examples, the shield pierceable element 1280 can be formed from rubber or silicone material. The shield pierceable element 1280 can be formed from self-healing polymeric material. The shield pierceable element 1280 can include a shield piercing line 1290. The shield piercing line 1290 can be a score line, a slit, a portion of reduced thickness, and/or any other suitable feature to facilitate piercing by the piercing member 1300. For example, the shield piercing line 1290 may provide for predictable opening and closing of the shield pierceable element. The shield piercing line 1290 can facilitate piercing of the shield pierceable element 1280 by the piercing member 1300 and/or self-healing (e.g., reclosing or resealing) of the shield pierceable element 1280 when it is unpierced by the piercing member 1300, for example, as described further below.

As illustrated in Fig. 31, the drug container 1400 can implement the drug container 200 and can be generally similar to the drug container 600 and/or the drug container 900. The drug container 1400 can include a cavity 1402c that can be similar to the cavity 602c, and a container end 1406 defining a container opening 1410. The container opening 1410 can be an opening to the cavity 1402c. The drug container 1400 can also include a septum 1408 that can be similar to the septum 608. The septum 1408 can be configured to seal the container opening 1410, which can seal a liquid drug in the cavity 1402c.

The drug container 1400 can also include a cap assembly 1420. The cap assembly 1420 can include a cap housing 1425. The cap housing 1425 can be formed from materials similar to the needle support 1215 and/or the needle shield 1250 (e.g., similar to the exemplary disclosed structural materials, for example, as described above). The cap housing 1425 can form a container cavity 1430 that can be configured to receive and/or attach to the container end 1406. The container cavity 1430 can be a sterile cavity. In some examples, the container cavity 1430 can be configured to receive, fit around, and/or attach to the container end 1406, for example, as illustrated in Fig. 31. The cap housing 1425 can attach to the container end 1406 and/or be received in the container cavity 1430 by any suitable technique such as, for example, crimping, snap-fit or press-fit connection, mechanical fasteners, adhesive, and/or any other suitable connection technique. In some examples, the cap housing 1425 can be attached to the container end 1406 via crimping (e.g., via an aluminum crimp or any other suitable crimping). In some examples, the cap housing 1425 can be integrally formed with the container end 1406 (e.g., the cap assembly 1420 can be an integral portion of the drug container 1400).

The cap housing 1425 can also form a cap cavity 1435. The cap cavity 1435 can be configured to receive (e.g., support and/or hold) components of the cap assembly 1420 and/or the piercing member 1300 (e.g., in some embodiments). In some examples, the cap cavity 1435 can be configured to receive a sliding assembly 1440 and the piercing member 1300. The cap cavity 1435 can be a sterile cavity (e.g., a cartridge sterile chamber serving as an aseptic environment).

The sliding assembly 1440 can be movably disposed in the cap cavity 1435. In some examples, the sliding assembly 1440 can move toward the septum 1408 within the cap assembly 1420 based on moving in the cap cavity 1435. The cap cavity 1435 and/or the sliding assembly 1440 (e.g., a sliding member 1445 of the sliding assembly 1440) can be configured to have corresponding protrusions, slots, grooves, and/or any other suitable features to allow the sliding assembly 1440 to move (e.g., to translate) within the cap assembly 1420. The sliding member 1445 can be formed from material similar to the cap housing 1425. A sliding member aperture 1450 can be formed in sliding member 1445. In some examples, the sliding member aperture 1450 can be formed at a central portion of the sliding member 1445 and can extend through a thickness (e.g., an entire thickness) of the sliding member 1445. The sliding member 1445 can also be configured to receive (e.g., hold and support) a container pierceable element 1455. The container pierceable element 1455 can cover substantially all (e.g., or some) of the sliding member aperture 1450. In some examples, the container pierceable element 1455 can be concentric with the septum 1408 and a through-hole of the piercing member 1300 as illustrated in Fig. 31 and further described below. The container pierceable element 1455 can be similar to the shield pierceable element 1280 and can be formed from materials similar to the shield pierceable element 1280, for example, as described above. For example, the container pierceable element 1455 can be a sterile barrier. Also, for example, the container pierceable element 1455 can include a container piercing line 1460 similar to the shield piercing line 1290.

The piercing member 1300 can be formed from materials similar to the needle support 1215 and/or the needle shield 1250 (e.g., similar to the exemplary disclosed structural materials described above). The piercing member 1300 can include a piercing portion 1305 having a piercing tip 1310. The piercing tip 1310 can have any suitable configuration (e.g., a point and/or a relatively sharpened edge) for piercing shield pierceable element 1280 and/or container pierceable element 1455. A through-hole 1315 can be formed in the piercing portion 1305. The piercing member 1300 can also include a member base 1320 that can be attached to other components of the drug delivery system 1100 to support the piercing member 1300 at a desired position and/or orientation (e.g., for piercing shield pierceable element 1280 and/or container pierceable element 1455). The through-hole 1315 can extend substantially entirely through the piercing member 1300 (e.g., including along substantially an entire length of the piercing portion 1305 and substantially an entire thickness of the member base 1320).

In some examples and as illustrated in Fig. 31, the piercing member 1300 can be received (e.g., supported and/or held) in the cap cavity 1435 of the cap assembly 1420. For example, the member base 1320 can be attached (e.g., fixedly attached) to the cap housing 1425 so that the through-hole 1315 is aligned with the septum 1408. Also, for example, the piercing tip 1310 can be aligned with the sliding member aperture 1450 and the container pierceable element 1455 of the sliding assembly 1440. The through-hole 1315 may thereby be disposed between and aligned with the container pierceable element 1455 and the septum 1408.

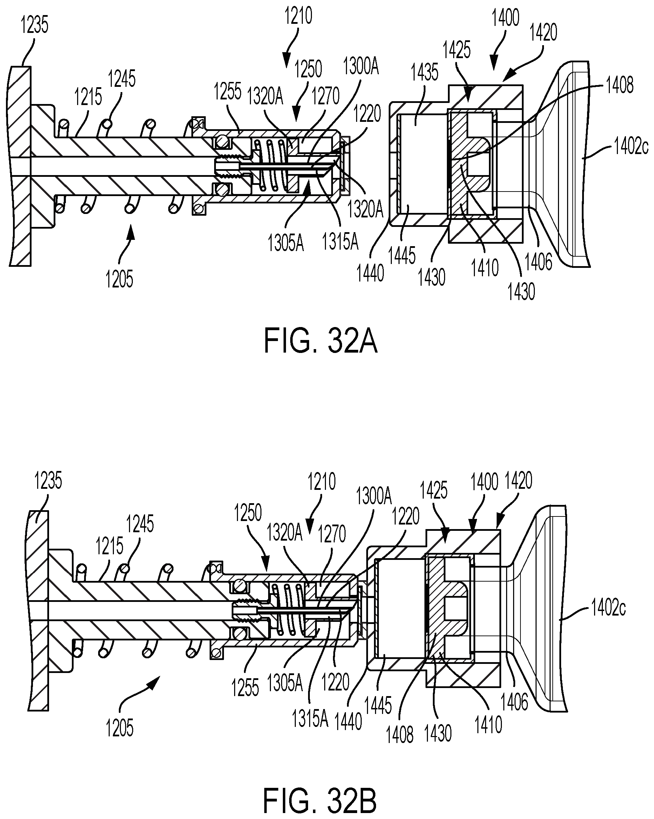

In other examples and as illustrated in Fig. 32A (unengaged configuration) and Fig. 32B (engaged configuration, prior to piercing of drug container 1400), the exemplary disclosed piercing member (e.g., a piercing member 1300A) can be received (e.g., supported and/or held) in the needle cavity 1270 of the needle shield 1250. The piercing member 1300A can be generally similar to the piercing member 1300, for example, as described above (e.g., including a piercing portion 1305A, a piercing tip 1310A, a through-hole 1315A, and a base portion 1320A). The septum piercing needle 1220 can be disposed in the through-hole 1315A and can remain within (e.g., at least partially within or substantially entirely within) the through-hole 1315A during an operation of the exemplary disclosed drug delivery system. The needle support 1215 and base portion 1320A are separated by internal biasing member or spring 1255 which is compressed as needle support 1215 is urged toward base portion 1320A, thereby driving the piercing needle 1220 to drug container 1400.

Accordingly, in some embodiments (e.g., Fig. 31), the piercing member 1300 can be supported by the drug container 1400 (e.g., the cap assembly 1420), and in other embodiments (e.g., Figs. 32A and 32B), the piercing member 1300A can be supported by the septum piercing assembly 1200. In some examples, the exemplary disclosed piercing member may thereby be disposed on either of a septum piercing assembly side or a drug container side of the drug delivery system 1100.

Figs. 29-32 illustrate exemplary embodiments of the drug delivery system 1100 in an unpierced configuration. As further described below, the drug delivery system 1100 can be moved from the unpierced configuration to a pierced configuration and a post-pierced configuration during an operation of the drug delivery system 1100.

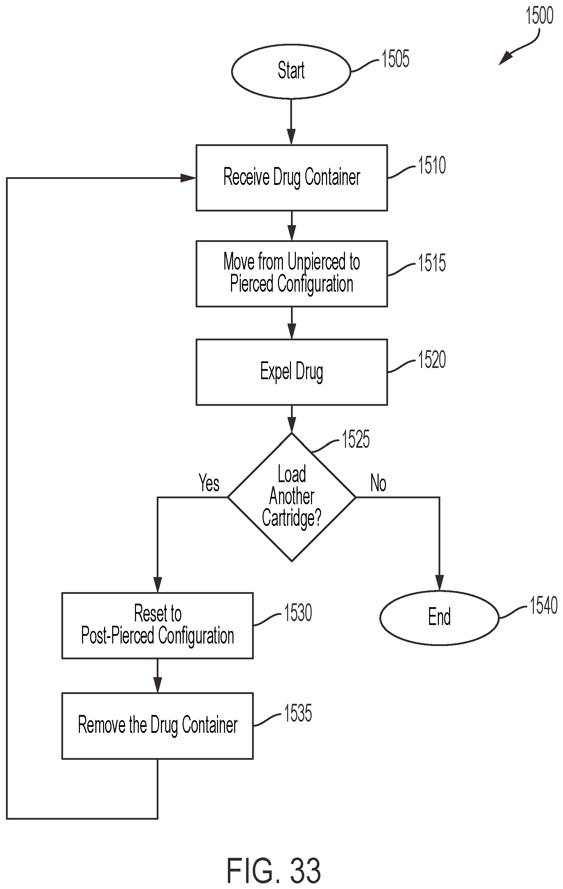

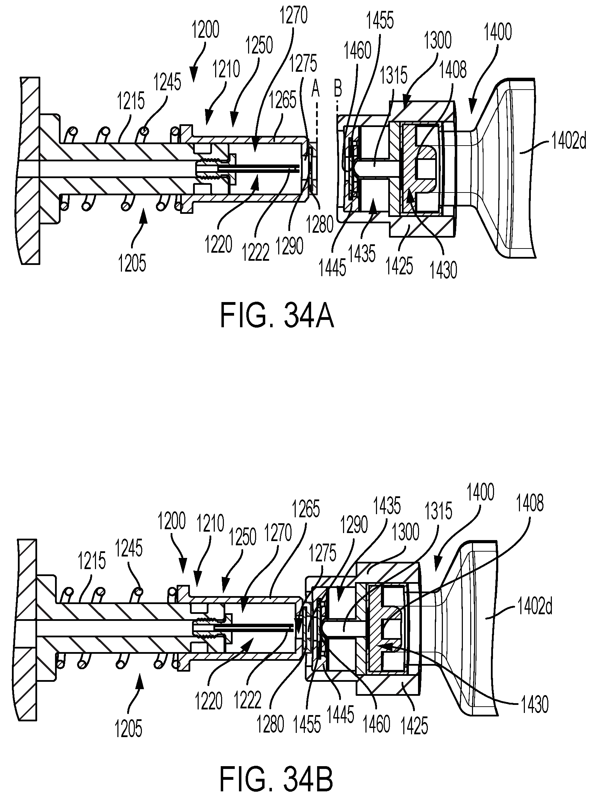

In some examples and referring to Fig. 33, an exemplary disclosed method (e.g., process 1500) of using the drug delivery system 1100 can comprise the following steps. Process 1500 can begin at step 1505. At step 1510, process 1500 can include receiving the drug container 1400. The drug container 1400 can be received in the housing 1110 similar to, for example, as described above regarding the drug delivery system 800. Fig. 34A schematically illustrates an unpierced configuration of the drug delivery system 1100 when the drug container 1400 is received at step 1510. The drug container 1400 can be received with any suitable clearance between datum A and B, for example, as illustrated in Fig. 34A and described above regarding Figs. 19, 22A, and 23A to allow for introduction of the drug container 1400 to the housing 1110 along a perpendicular direction from an axis of movement of the drug container 1400 (e.g., the axis of movement when moved by the plunger 1130).

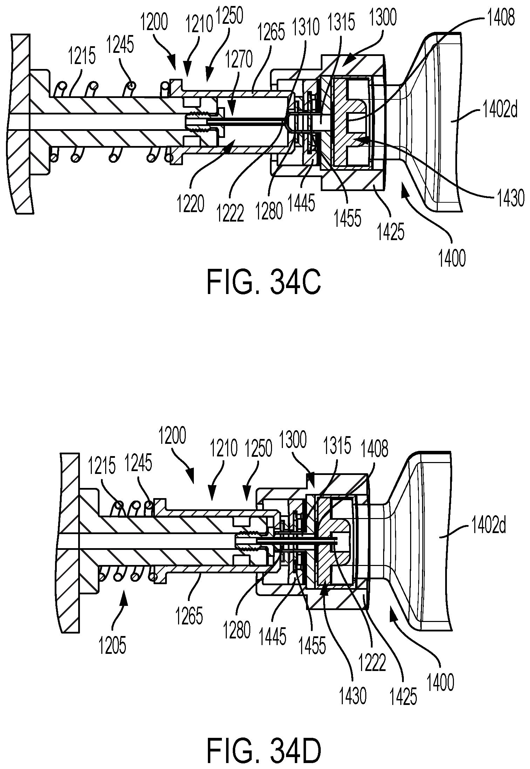

Returning to Fig. 33 at step 1515, process 1500 can include moving the drug delivery system 1100 from the unpierced configuration to the pierced configuration. As illustrated in Figs. 34A and 34B, the drug container 1400 can be moved toward the septum piercing assembly 1200 based on being contacted and moved by the plunger 1130 (e.g., which can be driven similarly to as described above regarding the plunger 500 and/or the plunger 830). After the needle shield 1250 contacts the sliding member 1445 as illustrated in Fig. 34B, the plunger 1130 can continue to move the drug container 1400 toward the septum piercing assembly 1200. At this point, as illustrated in Figs. 34B and 34C, the movement of the drug container 1400 can cause the sliding member 1445 to move within the cap housing 1425. That is, the drug container 1400 can continue to move toward the septum piercing assembly 1200 so that a portion of the needle shield 1250 is received in the cap housing 1425, which can move the sliding member 1445 from the position illustrated in Fig. 34B into an interior of the cap housing 1425 so that the sliding member 1445 contacts the piercing member 1300 (e.g., the member base 1320) as illustrated in Fig. 34C. Because a force threshold for moving the sliding member 1445 can be less than a force threshold for deforming (e.g., compressing) the biasing member 1245, the biasing member 1245 may not deform (e.g., compress) as the sliding member 1445 moves. In some examples, the sliding member 1445 can be biased by a biasing member similar to the biasing member 1245, but can deform (e.g., compress) based on less force than the biasing member 1245 (e.g., so that the force threshold to move the sliding member 1445 can be less than the force threshold to deform the biasing member 1245). Accordingly, the needle shield 1250 may not move relative to the needle support 1215 as the sliding member 1445 is moved (e.g., as illustrated in Figs. 34B and 34C) based on the plunger 1130 moving the drug container 1400.

Also as illustrated in Figs. 34B and 34C, as the sliding member 1445 and the needle shield 1250 move into an interior of the cap housing 1425 and toward the piercing member 1300, the piercing tip 1310 of the piercing member 1300 can first come into contact with the container pierceable element 1455 and pierce the container pierceable element 1455. For example, the piercing tip 1310 can contact and pierce the container piercing line 1460 of the container pierceable element 1455 and can pass through an opening formed in the container pierceable element 1455. As the plunger 1130 continues to move the drug container 1400, the piercing tip 1310 and the piercing portion 1305 (e.g., some, most of, or substantially all of the piercing portion 1305) can pass through the container pierceable element 1455. As the plunger 1130 continues to move the drug container 1400 toward the septum piercing assembly 1200, the piercing tip 1310 of the piercing member 1300 can next come into contact with the shield pierceable element 1280 and pierce the shield pierceable element 1280. For example, the piercing tip 1310 can contact and pierce the shield piercing line 1290 of the shield pierceable element 1280 and pass through an opening formed in the shield pierceable element 1280. As the plunger 1130 continues to move the drug container 1400, the piercing tip 1310 and the piercing portion 1305 (e.g., some, most of, or substantially all of the piercing portion 1305) can pass through the shield pierceable element 1280 (e.g., as illustrated in Fig. 34C). After piercing tip 1310 has pierced the container pierceable element 1455 and the shield pierceable element 1280, the through-hole 1315 can serve as an unobstructed passage connecting the needle cavity 1270 (e.g., that can be sterile) and the unpierced septum 1408 facing the container cavity 1430 (e.g., that can be sterile). That is, for example, the through-hole 1315 can provide a sterile passage between the sterile needle cavity 1270 and the sterile container cavity 1430.

As the plunger 1130 continues to move the drug container 1400 toward the septum piercing assembly 1200 and as illustrated in Figs. 34C and 34D (e.g., after the sliding member 1445 contacts the piercing member 1300), the needle shield 1250 being in contact with the sliding member 1445 pressed against the piercing member 1300 can cause the needle shield 1250 to move relative to the needle support 1215, thereby deforming (e.g., compressing) the biasing member 1245. As the drug container 1400 in contact with the needle shield 1250 moves relative to the needle support 1215 and deforms the biasing member 1245, the piercing member 1300 moves relative to the septum piercing needle 1220. Because piercing tip 1310 has already pierced the container pierceable element 1455 and the shield pierceable element 1280, the through-hole 1315 can be unobstructed. As the plunger 1130 continues to move the drug container 1400 toward the septum piercing assembly 1200, the septum piercing needle 1220 can enter the through-hole 1315 as the piercing member 1300 is moved toward the needle support 1215 along with the rest of the drug container 1400. The piercing member 1300 can move along a portion of a length of the septum piercing needle 1220 until the tip 1222 of the septum piercing needle 1220 contacts and pierces the septum 1408 and passes into the drug container 1400 to form a fluid connection, for example, as illustrated in the pierced configuration of Fig. 34D. The septum piercing needle 1220 has accordingly moved through the through-hole 1315 without contacting any surfaces other than the septum 1408, thereby maintaining sterility.

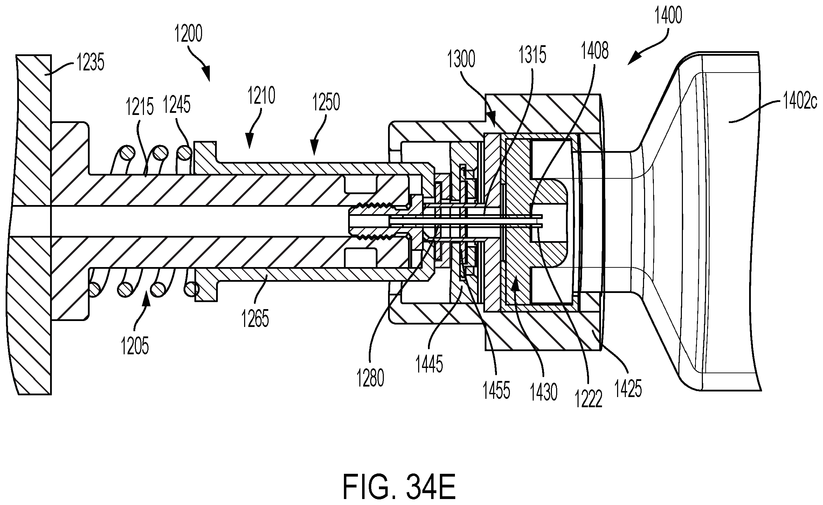

Returning to Fig. 33 at step 1520, process 1500 can include expelling a liquid drug from the drug container 1400 when the drug delivery system 1100 is in the pierced configuration. Fig. 34E illustrates a more detailed view of the pierced configuration of the drug delivery system 1100 illustrated in Fig. 34D. As illustrated in Figs. 34D and 34E when the drug delivery system 1100 is in the pierced configuration, the septum piercing needle 1220 can be disposed substantially entirely within the through-hole 1315, from an attachment of the septum piercing needle 1220 to the needle assembly 1215 to the septum 1408, which is pierced by the septum piercing needle 1220. The septum piercing needle 1220 can thereby be maintained substantially entirely within a sterile space (e.g., the through-hole 1315, the container cavity 1430, and an interior of the drug container 1400 after the septum 1408 is pierced). For example, sterility can be maintained by the shield pierceable element 1280 and the container pierceable element 1455, which may serve as penetrable sterile barriers. A liquid drug can thereby be delivered via the drug delivery system 1100, for example, similarly to as described above regarding the drug delivery system 100 (e.g., regarding Figs. 16B and 18) and/or the drug delivery system 800 (e.g., based on the plunger 1130 moving a seal similar to the seal 610 within the drug container 1400 similarly to as described above).

Returning to Fig. 33 at step 1525, it can be determined whether another drug container is to be loaded similarly to as described above regarding step 1025 of Fig. 28. If at step 1525 it is determined that another drug container is to be loaded, process 1500 can proceed to step 1530.

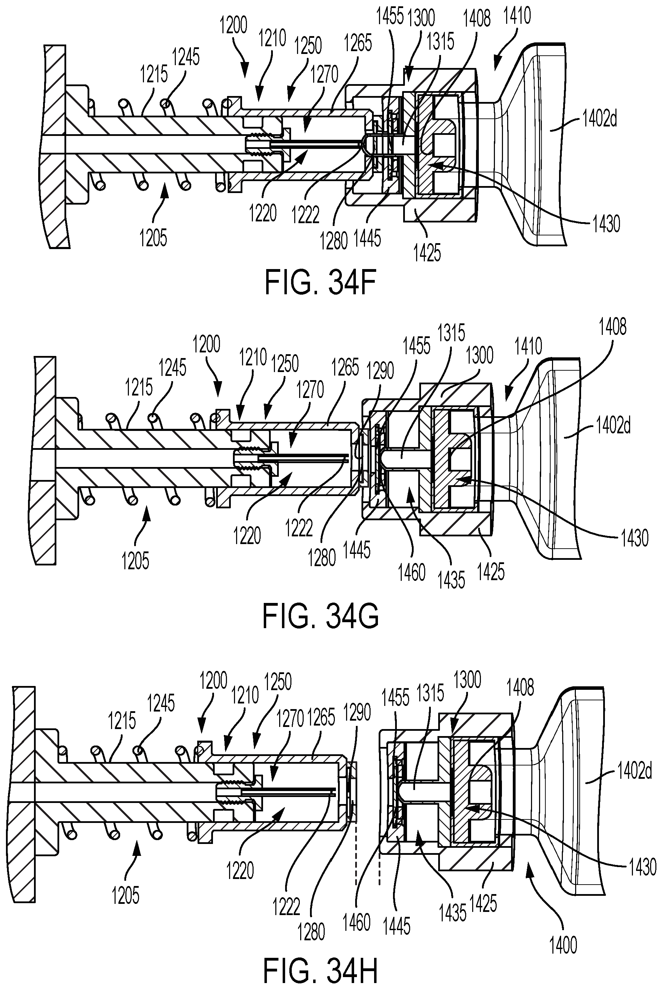

At step 1530, process 1500 can include resetting the drug delivery system 1100 from the pierced configuration to the post-pierced configuration that may be generally similar to the unpierced configuration. The plunger 1130 can reverse a direction of movement (e.g., similarly to as described above regarding Fig. 18) so that the drug container 1400 moves away from the septum piercing assembly 1200 as illustrated in Figs. 34E and 34F. For example, as the needle shield 1250 remains in contact with the sliding member 1445, the needle shield 1250 can move relative to the needle support 1215 to release potential energy from (e.g., uncompress) the biasing member 1245. The piercing member 1300 and the drug container 1400 can move relative to the septum piercing needle 1220 so that the septum piercing needle 1220 is unpierced from the septum 1408 and removed from the through-hole 1315 and is disposed in the needle cavity 1270 of the needle housing 1265 as illustrated in Fig. 34F. At this point the piercing tip 1310 can still be piercing the container pierceable element 1455 and the shield pierceable element 1280 so that the through-hole 1315 can still be unobstructed (e.g., though the septum piercing needle 1220 may no longer be disposed in the through-hole 1315, for example, as illustrated in Fig. 34F).

As illustrated in Figs. 34F and 34G, the sliding member 1445 and the needle shield 1250 can move away from an interior of the cap housing 1425 and the piercing member 1300 (e.g., based on urging of the sliding member 1445 by the biasing member similar to the biasing member 1245, for example, as described above). As the plunger 1130 continues to move the drug container 1400 away from the septum piercing assembly 1200, the piercing tip 1310 of the piercing member 1300 can be withdrawn from the shield pierceable element 1280 and unpierce the shield pierceable element 1280. After the shield pierceable element 1280 is unpierced, the shield pierceable element 1280 can self-heal (e.g., reclose or reseal). For example, the opening that was formed in the shield pierceable element 1280 can close. The plunger 1130 continues to move away from the plunger seal to allow the drug container 1400 to move away. The biasing member 1245 moves the drug container 1400 away from the piercing member 1300. The piercing tip 1310 of the piercing member 1300 can be withdrawn from the container pierceable element 1455 and unpierce the container pierceable element 1455. After the container pierceable element 1455 is unpierced, the drug container 1400 may be discarded and not reused. However, the drug container 1400 may be reused. For example, the container pierceable element 1455 can self-heal similarly to the shield pierceable element 1280. As the piercing member 1300 is unpierced from the shield pierceable element 1280 and the container pierceable element 1455, the sliding member 1445 can move from the position contacting piercing member 1300 illustrated in Fig. 34F to the position forming cap cavity 1435 illustrated in Fig. 34G. In some examples, the exemplary disclosed biasing member similar to the biasing member 1245 can urge the sliding member 1445 to the position illustrated in Fig. 34G. The plunger 1130 can continue to move the drug container 1400 further away from the septum piercing assembly 1200, from the position illustrated in Fig. 34G to the post-pierced configuration illustrated in Fig. 34H. In some examples, the self-healing of the shield pierceable element 1280 and the container pierceable element 1455 and the resetting of the drug delivery system 1100 as described at step 1530 can provide a re-usable aseptic connection (e.g., by substantially preventing incidental septic contact to the septum piercing needle 1220 during use of the drug delivery system 1100 such as, for example, during cartridge exchange at step 1510).

Returning to Fig. 33 at step 1535, the drug container 1400 can be removed from the housing 1110. Process 1500 can then return to step 1510, in which a new drug container 1400 can be received. As many iterations of steps 1510 through 1535 as desired may be performed. If at step 1525 it is determined that no additional cartridges are to be loaded, process 1500 can end at step 1540.

A process for using the exemplary embodiment illustrated in Fig. 32 and including piercing member 1300A can be generally similar to process 1500. Piercing member 1300A supported at the septum piercing assembly 1200 can pierce both the shield pierceable element 1280 attached to the needle shield 1250 and the container pierceable element 1455 attached to drug container 1400 similarly to as described above regarding Fig. 33.

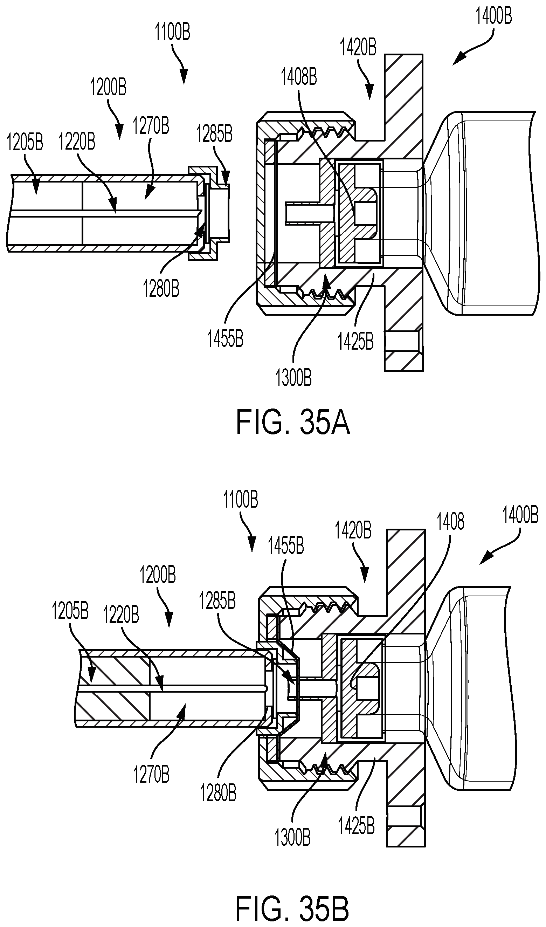

Figs. 35A and 35B illustrate another example of the drug delivery system. A drug delivery system 1100B can include a septum piercing assembly 1200B that can be generally similar to the septum piercing assembly 1200. The septum piercing assembly 1200B can include a needle assembly 1205B and a septum piercing needle 1220B disposed in a needle cavity 1270B that can be similar to the needle cavity 1270. The septum piercing assembly 1200B can also include a shield pierceable element 1280B that can be similar to the shield pierceable element 1280 and an attachment member 1285B that can be similar to attachment member 1285.

The drug delivery system 1100B can also include a drug container 1400B that can be similar to the drug container 1400. The drug container 1400B can include a cap assembly 1420B having a cap housing 1425B that can be generally similar to the cap housing 1425. The drug container 1400B can also include a container pierceable element 1455B that can be generally similar to the container pierceable element 1455. The drug container 1400B can also include a septum 1408B that can be similar to the septum 1408.

The drug delivery system 1100B can also include a piercing member 1300B. Piercing member 1300B can be similar to the piercing member 1300.

As illustrated in Figs. 35A and 35B, the drug container 1400B can be moved toward the septum piercing assembly 1200B similarly to as described above regarding the drug delivery system 1100. The container pierceable element 1455B can serve a similar purpose as the sliding member 1445 as it stretches and deforms as described below. As the drug container 1400B is moved toward the septum piercing assembly 1200B, the attachment member 1285B can deform the container pierceable element 1455B until the piercing member 1300B pierces the container pierceable element 1455B as illustrated in Fig. 35B. As the drug container 1400B is further moved toward the septum piercing assembly 1200B, the piercing member 1300B can also pierce the shield pierceable element 1280B so that the piercing member 1300B pierces both the container pierceable element 1455B and the shield pierceable element 1280B. As the drug container 1400B is further moved toward the septum piercing assembly 1200B, the piercing member 1300B can move along a portion of a length of the septum piercing needle 1220B until the septum piercing needle 1220B pierces the septum 1408B similarly to as described above regarding Figs. 34D and 34E. An operation of the drug delivery system 1100B can be generally similar to as described above regarding Fig. 33.

In some examples, the drug delivery system 1100 can comprise the drug container 1400 including a container body configured to contain a liquid drug therein, the container body having the container end 1406 defining the container opening 1410 therein, the septum 1408 configured to seal the container opening 1410, a first pierceable element (e.g., the container pierceable element 1455) spaced from the septum 1408 so as to define a first cavity (e.g., the container cavity 1430 and/or the cap cavity 1435) between the first pierceable element and the septum 1408 when the drug delivery system 1100 is in an unpierced configuration to reduce or substantially block admission of germs into the first cavity, and the piercing member 1300 defining the through-hole 1315 therethrough, the piercing member 1300 disposed in the first cavity (e.g., the container cavity 1430 and/or the cap cavity 1435) when the drug delivery system 1100 is in the unpierced configuration. The drug delivery system 1100 can also comprise the septum piercing assembly 1200, including the needle support 1215, a second pierceable element (e.g., the shield pierceable element 1280) spaced from the needle support 1215 so as to define a second cavity (e.g., the needle cavity 1270) between the second pierceable element and the needle support 1215 when the drug delivery system 1100 is in the unpierced configuration to reduce or substantially block admission of germs into the second cavity, and a needle 1220 supported by the needle support 1215 within the second cavity when the drug delivery system 1100 is in the unpierced configuration. The drug delivery system 1100 is configured such that, as the container 1400 and the septum piercing assembly 1200 are moved towards one another from the unpierced configuration to the pierced configuration, the piercing member 1300 can pierce the first and second pierceable elements and the needle 1220 is received through the through-hole 1315 of the piercing member 1300 into the septum 1408. The first and second cavities can be sterile chambers. When the drug delivery system 1100 moves from the pierced configuration to the post-pierced configuration, the first and second pierceable elements can be configured to close when the piercing member 1300 is removed. The first and second pierceable elements can be formed from elastomer material. The first and second pierceable elements can be formed from rubber or silicone material. The drug delivery system 1100 can be configured after delivery of the liquid drug in the pierced configuration to return to the post-pierced configuration that can be identical to the unpierced configuration except that the septum 1408 can be pierced and an amount of the liquid drug in the container 1400 can be reduced. At least one of the first and second pierceable elements can include a score line or a slit at a contact location of the piercing member. The drug delivery system 1100 can also comprise the sliding member 1445 that can be slidably supported in a cavity assembly (e.g., the cap housing 1425) of the container 1400 that forms the first cavity. The first pierceable element can cover an aperture (e.g., the sliding member aperture 1450) of the sliding member 1445. The first cavity can be formed between the piercing member 1300, an inside wall of the cavity assembly, the sliding member 1445, and the first pierceable element. The sliding member 1445 can be configured to slide within the cavity assembly toward the septum 1408 based on a portion of the septum piercing assembly 1200 being received in the cavity assembly and contacting the sliding member 1445 when the drug delivery system 1100 moves from the unpierced configuration to the pierced configuration. When the drug delivery system 1100 is in the pierced configuration, the piercing member 1300 is pierced through the first pierceable element covering the aperture of the sliding member 1445 and the second pierceable element. When the drug delivery system 1100 is in the pierced configuration, the needle 1220 can extend through the through-hole 1315 along a length of the piercing member 1300 through the first pierceable element, the aperture of the sliding member 1445, and the second pierceable element. As the drug delivery system 1100 moves from the unpierced configuration to the pierced configuration, the tip 1222 of the needle 1220 can move from the second cavity, through the through-hole 1315 of the piercing member 1300 that pierces the first and second pierceable elements, through the first cavity, and can pierce the septum 1408.

In some examples, the first pierceable element (e.g., the container pierceable element 1455B) can cover an opening of a cavity assembly of the container 1400B that forms the first cavity, the first pierceable element being attached at a fixed attachment portion of the first pierceable element to the cavity assembly. The first pierceable element can be configured to deflect, without displacement from the fixed attachment portion, when the septum piercing assembly 1200B contacts the first pierceable element. The drug delivery system 1100B can be configured to move from the unpierced configuration to the pierced configuration based on the container 1400B and the septum piercing assembly 1200B moving towards one another based on the deflection of the first pierceable element. When the drug delivery system 1100B is in the pierced configuration, the piercing member 1300B can be pierced through the first pierceable element that can be deflected and the second pierceable element (e.g., the shield pierceable element 1280B). The first pierceable element can be deflected and stretched inward into an interior of the cavity assembly, which can reduce a volume of the first cavity, when the container 1400B and the septum piercing assembly 1200B move towards one another.

In some examples, the drug delivery system 1100 can comprise the container 1400, including a container body configured to contain a liquid drug therein, the container body having the container end 1406 defining the container opening 1410 therein, and the septum 1408 configured to seal the container opening 1410, a resettable septum piercing mechanism (e.g., the septum piercing assembly 1200), including the needle support 1215, a pierceable element (e.g., the shield pierceable element 1280) spaced from the needle support 1215 so as to define a cavity between the pierceable element and the needle support 1215 when the drug delivery system 1100 is in the unpierced configuration to reduce or substantially block admission of germs into the cavity, and a needle 1220 supported by the needle support 1215 within the cavity when the drug delivery system 1100 is in the unpierced configuration. The drug delivery system 1100 can further comprise the biasing member 1245, and the piercing member 1300 defining the through-hole 1315 therethrough. The drug delivery system 1100 can be configured such that as the container 1400 and the resettable septum piercing mechanism are moved towards one another from the unpierced configuration to the pierced configuration, the piercing member 1300 can pierce the pierceable element and the needle 1220 can extend out of the cavity through the through-hole 1315 of the piercing member 1300 and into the septum 1408. As the container 1400 and the resettable septum piercing mechanism are moved away from one another from the pierced configuration to the unpierced configuration, the biasing member 1245 can move at least one of the pierceable element or the needle support 1215 away from the other such that the needle 1220 is returned to the cavity. The piercing member 1300 can be disposed in the cavity when the drug delivery system 1100 is in the unpierced configuration. In some examples, the needle 1220 can be disposed in the through-hole (e.g., the through-hole 1315A) of the piercing member (e.g., the piercing member 1300A) when the drug delivery system 1100 is in the unpierced configuration. The drug delivery system 1100 can further comprise the container pierceable element 1455 spaced from the septum 1408 so as to define the container cavity 1430 between the container pierceable element 1455 and the septum 1408 when the drug delivery system 1100 is in the unpierced configuration to reduce or substantially block admission of germs into the container cavity 1430. The piercing member 1300 can be disposed in the container 1400 when the drug delivery system 1100 is in the unpierced configuration. The drug delivery system 1100 can be configured such that as the container 1400 and the resettable septum piercing mechanism are moved towards one another from the unpierced configuration to the pierced configuration, the piercing member 1300 can pierce the container pierceable element 1455 and the pierceable element, and the needle 1220 can extend out of the cavity through the through-hole 1315 of the piercing member 1300 and into the septum 1408. The drug delivery system 1100 can further comprise a drive mechanism (e.g., including the plunger 1130) configured to contact the container 1400 to move the container 1400 towards the resettable septum piercing assembly 1200. The drive mechanism moves the plunger 1130 back out of the container 1400 away from the plunger seal. As the plunger 11300 moves away from the plunger seal, the biasing member 1245 is permitted to push the container 1400 off of the piercing member 1300 to the post-pierced position. The drug delivery system 1100 can further comprise a drive mechanism configured to move the at least one of the pierceable element or the needle support 1215 to return the at least one of the pierceable element or the needle support 1215 from the pierced configuration to an initial position of the unpierced configuration. The cavity can be a sterile chamber. When the container 1400 and the resettable septum piercing assembly 1200 are moved away from one another, the pierceable element can be configured to close when the piercing member 1300 is removed. The pierceable element can include a score line or a slit at a contact location of the piercing member 1300.

In some examples, the exemplary disclosed method may be a method for operating the drug delivery system 1100 comprising the drug container 1400 including the septum 1408 and a first pierceable element (e.g., the container pierceable element 1455) spaced from the septum 1408 so as to define a first cavity, and the piercing member 1300 disposed in the first cavity, the first pierceable element reducing or substantially blocking admission of germs into the first cavity, and the septum piercing assembly 1200 including the needle support 1215 and a second pierceable element (e.g., the shield pierceable element 1280) spaced from the needle support 1215 so as to define a second cavity, and the needle 1220 supported by the needle support 1215 within the second cavity, the second pierceable element reducing or substantially blocking admission of germs into the second cavity. The method can comprise moving the drug container 1400 and the septum piercing assembly 1200 towards one another from the unpierced configuration to the pierced configuration so that the piercing member 1300 pierces the first and second pierceable elements, and receiving the needle 1220 through the through-hole 1315 of the piercing member 1300 as the drug container 1400 and the septum piercing assembly 1200 move towards one another. The method can further comprise piercing the septum 1408 with the needle 1220 and delivering a liquid drug contained in the drug container 1400 via the needle 1220 when the piercing member 1300 is piercing the first and second pierceable elements and the needle 1220 is disposed in the through-hole 1315. The method can further comprise moving the drug container 1400 and the septum piercing assembly 1200 away from one another from the unpierced configuration to the post-pierced configuration that can be identical to the unpierced configuration except that the septum 1408 is pierced and an amount of the liquid drug in the drug container 1400 is reduced. The drug container 1400 can be configured to be removable when the drug container 1400 is in the post-pierced configuration and replaceable with a second drug container in the unpierced configuration. The first and second cavities can be sterile chambers. When the drug container 1400 and the septum piercing assembly 1200 are moved away from one another, the first and second pierceable elements can be configured to close when the piercing member 1300 is removed. At least one of the first and second pierceable elements can include a score line or a slit at a contact location of the piercing member 1300 for predictable opening and closing.

In some examples, the exemplary disclosed method may be a method for operating the drug delivery system 1100 comprising the container 1400, including a container body configured to contain a liquid drug therein, the container body having the container end 1406 defining the container opening 1410 therein, and the septum 1408 configured to seal the container opening 1410, a resettable septum piercing mechanism (e.g., the septum piercing assembly 1200) including the needle support 1215, a pierceable element (e.g., the shield pierceable element 1280) spaced from the needle support 1215 so as to define a cavity between the pierceable element and the needle support 1215 when the drug delivery system 1100 is in the unpierced configuration to reduce or substantially block admission of germs into the cavity, and the needle 1220 supported by the needle support 1215 within the cavity when the drug delivery system 1100 is in the unpierced configuration, the biasing member 1245, and the piercing member 1300 defining the through-hole 1315 therethrough. The method can comprise moving the container 1400 and the resettable septum piercing mechanism towards one another from the unpierced configuration to the pierced configuration so that the piercing member 1300 can pierce the pierceable element and the needle 1220 can extend out of the cavity through the through-hole 1315 of the piercing member 1300 and into the septum 1408, and moving the container 1400 and the resettable septum piercing mechanism away from one another from the pierced configuration to the unpierced configuration so that the biasing member 1245 moves at least one of the pierceable element or the needle support 1215 away from the other such that the needle 1220 is returned to the cavity. The piercing member 1300 can be disposed in the cavity when the drug delivery system 1100 is in the unpierced configuration. In some examples, the needle 1220 can be disposed in the through-hole (e.g., the through-hole 1315A) of the piercing member (e.g., the piercing member 1300A) when the drug delivery system 1100 is in the unpierced configuration. The drug delivery system 1100 can further comprise the container pierceable element 1455 spaced from the septum 1408 so as to define a container cavity 1430 between the container pierceable element 1455 and the septum 1408 when the drug delivery system 1100 is in the unpierced configuration to reduce or substantially block admission of germs into the container cavity 1430. The piercing member 1300 can be disposed in the container 1400 when the drug delivery system 1100 is in the unpierced configuration. As the container 1400 and the resettable septum piercing mechanism are moved towards one another from the unpierced configuration to the pierced configuration, the piercing member 1300 can pierce the container pierceable element 1455 and the pierceable element, and the needle 1220 can extend out of the cavity through the through-hole 1315 of the piercing member 1300 and into the septum 1408.

It should be noted that the illustrations and descriptions of the examples and embodiments shown in the figures are for exemplary purposes only, and should not be construed limiting the disclosure. One skilled in the art will appreciate that the present disclosure contemplates various embodiments. Additionally, it should be understood that the concepts described above with the above-described examples and embodiments may be employed alone or in combination with any of the other examples and embodiments described above. It should further be appreciated that the various alternative examples and embodiments described above with respect to one illustrated embodiment can apply to all examples and embodiments as described herein, unless otherwise indicated.

Unless explicitly stated otherwise, each numerical value and range should be interpreted as being approximate as if the word "about," "approximately," or "substantially" preceded the value or range. The terms "about," "approximately," and "substantially" can be understood as describing a range that is within 20 percent, 15 percent, 10 percent, or 5 percent of a specified value unless otherwise stated.

Conditional language used herein, such as, among others, "can," "could," "might," "may," "e.g.," and the like, unless specifically stated otherwise, or otherwise understood within the context as used, is generally intended to convey that certain embodiments include, while other embodiments do not include, certain features, elements, and/or steps. Thus, such conditional language is not generally intended to imply that features, elements, and/or steps are in any way required for one or more embodiments or that one or more embodiments necessarily include logic for deciding, with or without author input or prompting, whether these features, elements and/or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list.

While certain example embodiments have been described, these embodiments have been presented by way of example only and are not intended to limit the scope of the inventions disclosed herein. Thus, nothing in the foregoing description is intended to imply that any particular feature, characteristic, step, module, or block is necessary or indispensable. Indeed, the novel methods and systems described herein may be embodied in a variety of other forms; furthermore, various omissions, substitutions, and changes in the form of the methods and systems described herein may be made without departing from the spirit of the inventions disclosed herein. The accompanying claims and their equivalents are intended to cover such forms or modifications as would fall within the scope and spirit of certain of the inventions disclosed herein.

It will be understood that reference herein to "a" or "one" to describe a feature such as a component or step does not foreclose additional features or multiples of the feature. For instance, reference to a device having or defining "one" of a feature does not preclude the device from having or defining more than one of the feature, as long as the device has or defines at least one of the feature. Similarly, reference herein to "one of" a plurality of features does not foreclose the invention from including two or more, up to all, of the features. For instance, reference to a device having or defining "one of a protrusion and a recess" does not foreclose the device from having both the protrusion and the recess.

Aspects of the disclosure forming part of this description:

### Drug Delivery System Actuator

1. A drug delivery system, comprising:
   at least one curved track;
   a plunger having a flexible plunger rod;
   a driver configured to cause the plunger to translate along the at least one curved track such that the flexible plunger rod bends as it translates along the at least one curved track into a drug container to drive a liquid drug from the drug container into a patient; and
   at least one roller or bearing configured to guide the flexible plunger rod as the flexible plunger rod translates along the at least one curved track.
2. The drug delivery system of claim 1, wherein the flexible plunger rod supports the least one roller or bearing such that the least one roller or bearing rides along the at least one curved track with the flexible plunger rod.
3. The drug delivery system of claim 1, wherein the track supports the least one roller or bearing such that plunger rod rides along the at least one roller or bearing.
4. The drug delivery system of any one of claims 1 to 3, wherein the flexible plunger rod has a first outboard side and a second outboard side that are opposite one another, and the least one roller or bearing can comprise one or more rollers or bearings disposed on the first outboard side of the flexible plunger rod.
5. The drug delivery system of claim 4, wherein:
   the at least one roller or bearing can comprise one or more rollers or bearings disposed on the second outboard side of the flexible plunger rod;
   the at least one track can comprise a pair of tracks that are opposite one another; and
   the one or more rollers or bearings of the first outboard side ride along a first one of the tracks, and the one or more rollers or bearings of the second outboard side ride along a second one of the tracks.
6. The drug delivery system of claim 4, wherein the flexible plunger rod has a first outboard side and a second outboard side that are opposite one another, and the least one roller or bearing is disposed between the first outboard side and the second outboard side.
7. The drug delivery system of any one of claims 1 to 6, wherein the flexible plunger rod comprises a plurality of links that are pivotably connected to one another.
8. The drug delivery system of claim 6, wherein each of the at least one roller or bearing is supported by one link of the plurality of links.
9. The drug delivery system of any one of claims 1 to 6, wherein the flexible plunger rod comprises a flexible material that is capable of bending as the plunger rod translates along the at least one curved track.
10. The drug delivery system of any one of claims 1 to 9, comprising:
   a threaded rod that is configured to engage internal threads of the plunger; and
   a motor that is configured to cause the threaded rod to rotate to cause the plunger to translate along the at least one track.
11. The drug delivery system of any one of claim 1 to 10, comprising reinforcement structure that is configured to resist opposing forces applied by the plunger at a curve defined by the at least one curved track at a first end of the drug delivery system, and by the drug container and/or driver at a second end of the drug delivery system, opposite the first end.
12. The drug delivery system of claim 11, wherein the reinforcement structure comprises a rigid plate.
13. The drug delivery system of any one of claims 11 and 12, wherein the reinforcement structure defines the at least one track.
14. The drug delivery system of any one of claims 11 to 13, wherein the reinforcement structure has a first end that resists outward movement of the at least one curved track at a first end of the drug delivery system along a select direction, and a second end that resists outward movement of the drug container and/or driver at a second end of the drug delivery system along a direction opposite the select direction.
15. The drug delivery system of claim 13, wherein the at least one track is defined by an opening or recess that extends into or through the reinforcement structure, the opening or recess configured to receive the at least one roller or bearing therein.
16. The drug delivery system of any one of claims 1 to 15, comprising the drug container, wherein the drug container is a cartridge comprising a container body and a seal that forms a seal with an interior surface of the container body, and the plunger is configured to engage the seal to drive the liquid drug from the container.
17. The drug delivery system of any one of claims 1 to 16, wherein the drug delivery system is configured such that a force needed to translate a seal within the drug container with the flexible plunger rod is no greater than 30% more than a force needed to translate the seal within the container with a straight plunger rod.
18. The drug delivery system of any one of claims 1 to 17, wherein the drug delivery system is capable of driving the plunger seal within the container with a force of at least 50N.
19. The drug delivery system of any one of claims 1 to 18, wherein the drug delivery system comprises:
   a needle or cannula; and
   an insertion mechanism configured to insert a needle or cannula of the drug delivery system into a patient.
20. A method of delivering a drug to a patient with a drug delivery system, the method comprising:
   inserting a needle or cannula of the drug delivery system into the patient; and
   causing a flexible plunger rod of the drug delivery system to translate along at least one curved track of the drug delivery system such that the flexible plunger rod bends as it translates along the at least one curved track into a drug container of the drug delivery system to drive a liquid drug from the drug container into the patient, wherein at least one roller or bearing of the of the drug delivery system guides the flexible plunger rod as the flexible plunger rod translates along the at least one curved track.
21. The method of claim 20, wherein the flexible plunger rod supports the least one roller or bearing, and the causing step comprises causing the least one roller or bearing to ride along the at least one curved track with the flexible plunger rod.
22. The method of claim 20, wherein the track supports the least one roller or bearing, and the causing step comprises causing the plunger rod to ride along the at least one roller or bearing.
23. The method of any one of claims 20 to 22, wherein the drug delivery system comprises a threaded rod that is configured to engage internal threads of the plunger, and the method comprises causing a driver to cause the threaded rod to rotate to cause the plunger to translate along the at least one track.
24. A drug delivery system, comprising:
   a curved track;
   a plunger having a flexible plunger rod;
   a driver configured to cause the plunger to translate along the curved track such that the flexible plunger rod bends along the curved track to drive a plunger seal of a drug container to expel a liquid drug from the drug container,
   wherein the drug delivery system is configured such that a force needed for the driver to translate the plunger seal within the container with the flexible plunger rod is no greater than 30% more than a force needed to translate the plunger seal within the container with a straight plunger rod.
25. The drug delivery system of claim 24, wherein the drug delivery system is configured such that the force needed for the driver to translate the plunger seal within the container with the flexible plunger rod is no greater than 25% more than the force needed to translate the plunger seal within the container with a straight plunger rod.
26. The drug delivery system of claim 24, wherein the drug delivery system is configured such that the force needed for the driver to translate the plunger seal within the container with the flexible plunger rod is no greater than 20% more than the force needed to translate the plunger seal within the container with a straight plunger rod.
27. The drug delivery system of claim 24, wherein the drug delivery system is configured such that the force needed for the driver to translate the plunger seal within the container with the flexible plunger rod is no greater than 15% more than the force needed to translate the plunger seal within the container with a straight plunger rod.
28. The drug delivery system of any one of claims 24 to 27, comprising:
   a threaded rod that is configured to engage internal threads of the plunger; and
   a motor that is configured to cause the threaded rod to rotate to cause the plunger to translate along the at least one track.
29. The drug delivery system of any one of claim 24 to 28, comprising reinforcement structure that is configured to resist opposing forces applied by the plunger at a curve defined by the at least one curved track at a first end of the drug delivery system, and by the drug container and/or driver at a second end of the drug delivery system, opposite the first end.
30. The drug delivery system of claim 29, wherein the reinforcement structure comprises a rigid plate.
31. The drug delivery system of any one of claims 29 to 30, wherein the reinforcement structure has a first end that resists outward movement of the at least one curved track at a first end of the drug delivery system along a select direction, and a second end that resists outward movement of the drug container and/or driver at a second end of the drug delivery system along a direction opposite the select direction.
32. The drug delivery system of any one of claims 24 to 31, comprising the drug container, wherein the drug container is a cartridge comprising a container body and a seal that forms a seal with an interior surface of the container body, and the plunger is configured to engage the seal to drive the liquid drug from the container.
33. The drug delivery system of any one of claims 24 to 32, wherein the drug delivery system is capable of driving the plunger seal within the container with a force of at least 50N.
34. The drug delivery system of any one of claims 24 to 33, wherein the drug delivery system comprises:
   a needle or cannula; and
   an insertion mechanism configured to insert a needle or cannula of the drug delivery system into a patient.
35. A drug delivery system, comprising:
   a curved track;
   a plunger having a flexible plunger rod;
   a driver configured to cause the plunger to translate along the curved track such that the flexible plunger rod bends along the curved track to drive a plunger seal of a drug container to expel a liquid drug from the drug container,
   wherein the drug delivery system is capable of driving the plunger seal within the container with a force of at least 50N.
36. The drug delivery system of claim 35, wherein the drug delivery system is capable of driving the plunger seal within the container with a force of at least 150N.
37. The drug delivery system of claim 35, wherein the drug delivery system is capable of driving the plunger seal within the container with a force of at least 200N.
38. The drug delivery system of claim 35, wherein the drug delivery system is capable of driving the plunger seal within the container with a force of at least 250N.
39. The drug delivery system of claim 35, wherein the drug delivery system is capable of driving the plunger seal within the container with a force of at least 300N.
40. The drug delivery system of any one of claims 35 to 39, comprising:
   a threaded rod that is configured to engage internal threads of the plunger; and
   a motor that is configured to cause the threaded rod to rotate to cause the plunger to translate along the at least one track.
41. The drug delivery system of any one of claim 35 to 40, comprising reinforcement structure that is configured to resist opposing forces applied by the plunger at a curve defined by the at least one curved track at a first end of the drug delivery system, and by the drug container and/or driver at a second end of the drug delivery system, opposite the first end.
42. The drug delivery system of claim 41, wherein the reinforcement structure comprises a rigid plate.
43. The drug delivery system of any one of claims 41 to 42, wherein the reinforcement structure has a first end that resists outward movement of the at least one curved track at a first end of the drug delivery system along a select direction, and a second end that resists outward movement of the drug container and/or driver at a second end of the drug delivery system along a direction opposite the select direction.
44. The drug delivery system of any one of claims 35 to 43, comprising the drug container, wherein the drug container is a cartridge comprising a container body and a seal that forms a seal with an interior surface of the container body, and the plunger is configured to engage the seal to drive the liquid drug from the container.
45. The drug delivery system of any one of claims 40 to 44, wherein the drug delivery system comprises:
   a needle or cannula; and
   an insertion mechanism configured to insert a needle or cannula of the drug delivery system into a patient.
46. A method of delivering a drug to a patient with a drug delivery system, the method comprising:
   inserting a needle or cannula of the drug delivery system into the patient; and
   causing a flexible plunger rod of the drug delivery system to translate along at least one curved track of the drug delivery system such that the flexible plunger rod bends as it translates along the at least one curved track into a drug container of the drug delivery system to drive a liquid drug from the drug container with a force of at least 50N.
47. The method of claim 46, wherein the drug delivery system is capable of driving the plunger seal within the container with a force of at least 150N.
48. The method of claim 46, wherein the drug delivery system is capable of driving the plunger seal within the container with a force of at least 200N.
49. The method of claim 46, wherein the drug delivery system is capable of driving the plunger seal within the container with a force of at least 250N.
50. The method of claim 46, wherein the drug delivery system is capable of driving the plunger seal within the container with a force of at least 300N.
51. The method of any one of claims 46 to 50, wherein the drug delivery system comprises a threaded rod that is configured to engage internal threads of the plunger, and the method comprises causing a driver to cause the threaded rod to rotate to cause the plunger to translate along the at least one track.

### Septum Piercing Assembly

1. A drug delivery system, comprising:
   a curved track;
   a plunger having a flexible plunger rod and a plunger end;
   a septum piercing needle; and
   a driver configured to cause the plunger to translate along the curved track such that the flexible plunger rod bends during translation along the curved track and the plunger is adapted to cause a drug container to translate from a pre-pierced position at which a septum of the drug container is not pierced by the septum piercing needle to a pierced position at which the septum of the drug container is pierced by the septum piercing needle.
2. The drug delivery system of claim 1, wherein the plunger end is configured to engage a seal of the drug container to translate the drug container from the pre-pierced position to the pierced position.
3. The drug delivery system of any one of claims 1 to 2, wherein when the drug container is in the pierced position, movement of the flexible plunger rod causes a seal of the drug container to move within the drug container to deliver the drug.
4. The drug delivery system of any one of claims 1 to 3, further comprising a biasing assembly configured to cause the drug container to move from the pierced position to a removed position in which the septum piercing needle is removed from the drug container.
5. The drug delivery system of any one of claims 1 to 4, further comprising a sensor configured to sense when the drug container is moved to the pierced position.
6. The drug delivery system of claim 5, further comprising a controller that controls delivery of the drug based on the sensor sensing when the drug container is moved to the pierced position in which the delivery of the drug begins.
7. The drug delivery system of any one of claims 1 to 5, further comprising a controller;
   wherein when the drug container is in the pierced position, the controller controls the driver to cause the plunger to translate along the curved track to move the plunger end a predetermined distance.
8. The drug delivery system of claim 7, wherein an amount of the drug delivered via the septum piercing needle is based on the predetermined distance.
9. A drug delivery system, comprising:
   a curved track;
   a plunger having a flexible plunger rod and a plunger end;
   a septum piercing needle spaced from the plunger end, the septum piercing needle configured to pierce a septum of the drug container; and
   a driver configured to cause the plunger to translate along the curved track along a driving direction such that the flexible plunger rod bends along the curved track to drive a seal of the drug container to expel a liquid drug from the drug container, and to translate along a reverse direction, being opposite the driving direction, away from the seal after expelling the liquid drug from the drug container is complete,
   wherein when the plunger is translated along the reverse direction, the drug container moves away from the septum piercing needle to unpierce the septum.
10. The drug delivery system of claim 9, further comprising a biasing assembly configured to translate the drug container in the reverse direction so as to cause the septum piercing needle to unpierce the septum.
11. The drug delivery system of claim 10, wherein the biasing assembly comprises a needle shield configured to house a tip of the septum piercing needle in a removed position when the drug container is translated in the reverse direction.
12. The drug delivery system of any one of claims 10 to 11, wherein the biasing assembly includes a biasing member configured to translate the drug container in the reverse direction when the plunger is translated along the reverse direction.
13. The drug delivery system of any one of claims 9 to 12, wherein the plunger including the plunger end is configured to pull the drug container in the reverse direction,
   wherein when the plunger is translated along the reverse direction, the plunger is configured to move the drug container away from the septum piercing needle to unpierce the septum.
14. The drug delivery system of any one of claims 9 to 13, wherein the plunger is adapted to cause the drug container to translate in the driving direction from a pre-pierced position at which the septum of the drug container is not pierced by the septum piercing needle to a pierced position at which the septum is pierced by the septum piercing needle.
15. The drug delivery system of any one of claims 9 to 14, wherein the flexible plunger rod is translatable between a disengaged position in which the plunger end does not engage the drug container and an engaged position in which the plunger end engages the drug container.
16. The drug delivery system of any one of claims 9 to 15, wherein the driving direction and the reverse direction are relative to a housing that supports the septum piercing needle in a stationary position.
17. The drug delivery system of any one of claims 9 to 16, further comprising at least one roller or bearing disposed along the curved track and configured to guide the flexible plunger rod as the flexible plunger rod translates along the curved track.
18. The drug delivery system of any one of claims 9 to 17, further comprising a reinforcement structure that is configured to resist opposing forces applied by the plunger at a curve defined by the curved track at a first end portion of the drug delivery system, and by the drug container or the driver at a second end portion of the drug delivery system.
19. A drug delivery system, comprising:
   a curved track;
   a plunger having a flexible plunger rod and a plunger end;
   a driver configured to cause the plunger to translate along the curved track along a driving direction such that the flexible plunger rod bends along the curved track to drive a seal of the drug container to expel the liquid drug from the drug container;
   a septum piercing needle spaced from the plunger end, the septum piercing needle configured to pierce a septum of the drug container; and
   a needle shield configured to be moved between a shielding position, in which the needle shield extends beyond a tip of the septum piercing needle, and an exposed position, in which the tip of the septum piercing needle is exposed to allow the septum piercing needle to pierce the septum of the drug container.
20. The drug delivery system of claim 19, wherein the needle shield is configured to move from the exposed position to the shielding position after the septum piercing needle is removed from the septum of the drug container.
21. The drug delivery system of claim 20, further comprising a biasing member that biases the needle shield from the exposed position to the shielding position when the plunger is translated along the curved track along a reverse direction, opposite the driving direction, away from the seal.
22. The drug delivery system of claim 19, wherein the needle shield moves between the exposed position and the shielding position relative to a housing that supports the septum piercing needle in a stationary position.
23. A method, comprising:
   driving a plunger having a flexible plunger rod and a plunger end along a curved track,
      the plunger and the curved track being situated inside a housing,
      the plunger being arranged to interact with a drug container situated inside the housing,
      the housing having a septum-piercing needle;
   moving the drug container in a driving direction toward the septum-piercing needle by pushing the drug container with the plunger end;
   piercing a septum of the drug container with the septum-piercing needle based on driving the plunger in the driving direction; and
   unpiercing the septum of the drug container with the septum-piercing needle based on driving the plunger in a reverse direction that is opposite to the driving direction.
24. The method of claim 23, wherein the driving direction and the reverse direction are relative to the housing that supports the septum piercing needle in a stationary position.
25. The method of any one of claims 23 and 24, further comprising:
   moving a needle shield between a shielding position, in which the needle shield extends beyond a tip of the septum piercing needle, and an exposed position, in which the tip of the septum piercing needle is exposed to allow the septum piercing needle to pierce the septum of the drug container.
26. The method of any one of claims 23 to 25, wherein the needle shield is configured to move from the exposed position to the shielding position after the septum piercing needle is removed from the septum of the drug container.
27. The method of claim 26, further comprising:
   biasing the needle shield from the exposed position to the shielding position when the plunger is translated along the curved track along the reverse direction.
28. The method of any one of claims 23 to 27, further comprising:
   providing an opening in the housing to receive the drug container in the housing and to remove the drug container from the housing.

### Drug Container Replaceability During Use

1. A drug delivery system, comprising:
   a housing defining an opening therein configured to receive a drug container into the housing;
   a curved track;
   a plunger having a flexible plunger rod and a plunger end;
   a driver configured to cause the plunger to translate along the curved track such that the flexible plunger rod bends during translation along the curved track and the plunger end drives a plunger seal of the drug container to expel a liquid drug from the drug container; and
   a septum piercing needle configured to pierce a septum of the drug container, the plunger end spaced from the septum piercing needle when the plunger is in a pre-use position so as to define a space between the plunger end and the septum piercing needle, the space configured to receive the drug container when the drug container is received into the housing through the opening.
2. The drug delivery system of claim 1, wherein the flexible plunger rod is configured so that the plunger end is disposed outside of the drug container when the plunger is in the pre-use position.
3. The drug delivery system of any of claims 1 to 2, wherein the flexible plunger rod is configured to move the plunger end from outside of the drug container when the plunger is in the pre-use position to engage the drug container when the plunger is in an engaged position.
4. The drug delivery system of any of claims 1 to 3, wherein the plunger end is configured to engage a seal of the drug container in the engaged position.
5. The drug delivery system of any of claims 1 to 4, wherein the plunger end is configured to be disposed inside of the drug container to engage the seal of the drug container in the engaged position.
6. The drug delivery system of any of claims 1 to 5, wherein the plunger end is configured to engage the seal, which is a plugged seal, in the engaged position.
7. The drug delivery system of claim 1, further comprising control circuitry configured to receive data from a near-field communication tag or an RFID tag of the drug container via a reader, the control circuitry configured to control the driver based on the data.
8. A drug delivery system, comprising:
   a curved track;
   a plunger having a flexible plunger rod and a plunger end; and
   a driver configured to cause the plunger to translate along the curved track such that the flexible plunger rod bends during translation along the curved track and the plunger end drives a plunger seal of a drug container to expel a liquid drug from the drug container,
   wherein the flexible plunger rod is translatable between a disengaged position in which the plunger end does not engage the drug container and an engaged position in which the plunger end engages the drug container.
9. The drug delivery system of claim 8, wherein in the disengaged position, the plunger end is disposed outside of the drug container.
10. The drug delivery system of any of claims 8 to 9, wherein in the engaged position, the plunger end is disposed inside of the drug container.
11. A drug delivery system, comprising:
   a housing defining an opening therein configured to receive a first drug container and a second drug container into the housing, the first drug container containing a first liquid drug and the second drug container containing a second liquid drug;
   control circuitry;
   a curved track;
   a plunger having a flexible plunger rod and a plunger end; and
   a driver configured to cause the plunger to translate along the curved track such that the flexible plunger rod bends during translation along the curved track,
   wherein the opening is configured to removably receive the first drug container, and the control circuitry is configured to cause the driver to drive the plunger end to drive a plunger seal of the first drug container to expel the first liquid drug from the first drug container, and
   wherein the opening is configured to removably receive the second drug container, and the control circuitry is configured to cause the driver to drive the plunger end to drive a plunger seal of the second drug container to expel the second liquid drug from the second drug container.
12. The drug delivery system of claim 11, wherein a first amount of the first liquid drug expelled from the first drug container is different from a second amount of the second liquid drug expelled from the second drug container.
13. The drug delivery system of any of claims 11 and 12, wherein the first liquid drug is the same as the second liquid drug.
14. The drug delivery system of any of claims 11 and 12, wherein the first liquid drug is different from the second liquid drug.
15. The drug delivery system of any of claims 11, 12, and 14, wherein a first viscosity of the first liquid drug of the first drug container is different from a second viscosity of the second liquid drug of the second drug container.
16. The drug delivery system of any of claims 11 and 12, wherein a first volume capacity of the first drug container is different from a second volume capacity of the second drug container.
17. The drug delivery system of any of claims 11 and 12, wherein the control circuitry is configured to cause the driver to drive the plunger with a first driving force when the first drug container is received in the space, and a second driving force that is different from the first driving force when the second drug container is received in the space.
18. The drug delivery system of any of claims 11, 12, and 17, wherein:
   the control circuitry is configured to cause the driver to drive the plunger to drive the plunger seal of the first drug container to expel the first liquid drug from the first drug container at a first flow rate; and
   the control circuitry is configured to cause the driver to drive the plunger to drive the plunger seal of the second drug container to expel the second liquid drug from the second drug container at a second flow rate that is different from the first flow rate.
19. The drug delivery system of any of claims 11, 12, 17, and 18, wherein the control circuitry is configured to cause the driver to drive the plunger based on at least one of a first data received from a first near-field communication tag or a first RFID tag of the first drug container or a second data received from a second near-field communication tag or a second RFID tag of the second drug container.
20. The drug delivery system of any of claims 11, 12, and 17 to 19, wherein the first data identifies at least one of a volume of the first liquid to expel, a flow rate to expel the first liquid, a viscosity of the first liquid, or a driving force to drive the plunger to expel the first liquid, or
   wherein the second data identifies at least one of a volume of the second liquid to expel, a flow rate to expel the second liquid, a viscosity of the second liquid, or a driving force to drive the plunger to expel the second liquid.
21. The drug delivery system of claim 11, wherein removably receiving the first drug container and the second drug container in the space includes non-destructive removal of the first and second drug containers.
22. The drug delivery system of any of claims 11 and 21, wherein removably receiving the first drug container and the second drug container in the space includes toolless removal of the first and second drug containers.
23. A method, comprising:
   receiving a drug container through an opening of a housing of a drug delivery system into a space of the housing, the space defined between a septum piercing needle of the drug delivery system and a plunger end of a plunger of the drug delivery system, wherein the septum-piercing needle is configured to pierce a septum of the drug container;
   driving the plunger having a flexible plunger rod and the plunger end along a curved track so that the plunger engages the drug container situated in the space inside the housing; and
   driving the plunger so that the flexible plunger rod bends during translation along the curved track and the plunger end drives a plunger seal of the drug container to expel a liquid drug from the drug container.
24. The method of claim 23, wherein the receiving step comprises receiving the drug container into the space such that the plunger is in a pre-use position in which the plunger end is not engaged with the plunger seal.
25. The method of any of claims 23 to 24, further comprising moving the plunger from the pre-use position, wherein the plunger end is not engaged with the plunger seal, to an engaged position, wherein the plunger engages the plunger seal of the drug container.
26. The method of claim 23, further comprising:
   after expelling the liquid drug from the drug container, returning the plunger to the pre-use position by spacing the plunger end from the plunger seal; and
   after the drug container is removed, receiving a second drug container into the space of the housing through the opening.
27. The method of claim 26, further comprising driving the plunger so that the flexible plunger rod bends during translation along the curved track and the plunger end drives a plunger seal of the second drug container to expel a second liquid drug from the second drug container.
28. The method of any of claims 26 to 27, wherein driving the plunger seal of the drug container comprises expelling a first amount of the liquid drug from the drug container and driving the plunger seal of the second drug container comprises expelling a second amount of the second liquid drug from the second drug container that is different from the first amount.
29. The method of any of claims 26 to 28, wherein driving the plunger seal of the drug container comprises expelling the liquid drug from the drug container having a first volume capacity and driving the plunger seal of the second drug container comprises expelling the second liquid drug from the second drug container having a second volume capacity that is different from the first volume capacity.
30. The method of any of claims 26 to 29, wherein driving the plunger seal of the drug container comprises expelling the liquid drug that is the same as the second liquid drug expelled by driving the plunger seal of the second drug container.
31. The method of any of claims 26 to 29, wherein driving the plunger seal of the drug container comprises expelling the liquid drug that is different from the second liquid drug expelled by driving the plunger seal of the second drug container.
32. The method of any of claims 26 to 29 and 31, wherein driving the plunger seal of the drug container comprises expelling the liquid drug having a first viscosity and driving the plunger seal of the second drug container comprises expelling the second liquid drug having a second viscosity that is different from the first viscosity.
33. The method of any of claims 26 to 32, further comprising:
   driving the plunger with a first driving force when the drug container is received in the space; and
   driving the plunger with a second driving force that is different from the first driving force when the second drug container is received in the space.
34. The method of any of claims 26 to 33, further comprising:
   driving the plunger to drive the plunger seal of the drug container to expel the liquid drug from the drug container at a first flow rate; and
   driving the plunger to drive the plunger seal of the second drug container to expel the second liquid drug from the second drug container at a second flow rate that is different from the first flow rate.
35. The method of any of claims 24 to 34, further comprising:
   moving the plunger end of the plunger from the septum piercing needle in the pre-use position prior to receiving the drug container into the space of the housing.

### Sterile Barrier

1. A drug delivery system, comprising:
   a container, including:
      a container body configured to contain a liquid drug therein, the container body having a container end defining a container opening therein;
      a septum configured to seal the container opening;
      a first pierceable element spaced from the septum so as to define a first cavity between the first pierceable element and the septum when the drug delivery system is in an unpierced configuration to reduce or substantially block admission of germs into the first cavity; and
      a piercing member defining a through-hole therethrough, the piercing member disposed in the first cavity when the drug delivery system is in the unpierced configuration; and
   a septum piercing assembly, including:
      a needle support;
      a second pierceable element spaced from the needle support so as to define a second cavity between the second pierceable element and the needle support when the drug delivery system is in the unpierced configuration to reduce or substantially block admission of germs into the second cavity; and
      a needle supported by the needle support within the second cavity when the drug delivery system is in the unpierced configuration;
   wherein the drug delivery system is configured such that, as the container and the septum piercing assembly are moved towards one another from the unpierced configuration to a pierced configuration, the piercing member pierces the first and second pierceable elements and the needle is received through the through-hole of the piercing member into the septum.
2. The drug delivery system of claim 1, wherein the first and second cavities are sterile chambers.
3. The drug delivery system of any of claims 1 and 2, wherein when the drug delivery system moves from the pierced configuration to a post-pierced configuration, the first and second pierceable elements are configured to close when the piercing member is removed.
4. The drug delivery system of any of claims 1 to 3, wherein the first and second pierceable elements are formed from elastomer material.
5. The drug delivery system of any of claims 1 to 4, wherein the first and second pierceable elements are formed from rubber or silicone material.
6. The drug delivery system of any of claims 1 to 5, wherein the drug delivery system is configured after delivery of the liquid drug in the pierced configuration to move to the post-pierced configuration that is identical to the unpierced configuration except that an amount of the liquid drug in the container is reduced.
7. The drug delivery system of any of claims 1 to 6, wherein at least one of the first and second pierceable elements includes a score line or a slit at a contact location of the piercing member.
8. The drug delivery system of any of claims 1 to 7, further comprising a sliding member that is slidably supported in a cavity assembly of the container that forms the first cavity.
9. The drug delivery system of any of claims 1 to 8, wherein the first pierceable element covers an aperture of the sliding member.
10. The drug delivery system of any of claims 1 to 9, wherein the first cavity is formed between the piercing member, an inside wall of the cavity assembly, an inside wall of the sliding member, and an inside wall of the first pierceable element.
11. The drug delivery system of any of claims 1 to 10, wherein the sliding member is configured to slide within the cavity assembly toward the septum based on a portion of the septum piercing assembly being received in the cavity assembly and contacting the sliding member when the drug delivery system moves from the unpierced configuration to the pierced configuration.
12. The drug delivery system of any of claims 1 to 11, wherein when the drug delivery system is in the pierced configuration, the piercing member is pierced through the first pierceable element covering the aperture of the sliding member and the second pierceable element.
13. The drug delivery system of any of claims 1 to 12, wherein when the drug delivery system is in the pierced configuration, the needle extends through the through-hole along a length of the piercing member through the first pierceable element, the aperture of the sliding member, and the second pierceable element.
14. The drug delivery system of any of claims 1 to 13, wherein as the drug delivery system moves from the unpierced configuration to the pierced configuration, a tip of the needle moves from the second cavity, through the through-hole of the piercing member that pierces the first and second pierceable elements, through the first cavity, and pierces the septum.
15. The drug delivery system of any of claims 1 to 7, wherein the first pierceable element covers an opening of a cavity assembly of the container that forms the first cavity, the first pierceable element being attached at a fixed attachment portion of the first pierceable element to the cavity assembly.
16. The drug delivery system of any of claims 1 to 7 and 15, wherein the first pierceable element is configured to deflect, without displacement from the fixed attachment portion, when the septum piercing assembly contacts the first pierceable element.
17. The drug delivery system of any of claims 1 to 7, 15, and 16, wherein the drug delivery system is configured to move from the unpierced configuration to the pierced configuration based on the container and the septum piercing assembly moving towards one another based on the deflection of the first pierceable element.
18. The drug delivery system of any of claims 1 to 7 and 15 to 17, wherein when the drug delivery system is in the pierced configuration, the piercing member is pierced through the first pierceable element that is deflected and the second pierceable element.
19. The drug delivery system of any of claims 1 to 7 and 15 to 18, wherein the first pierceable element is deflected and stretched inward into an interior of the cavity assembly, which reduces a volume of the first cavity, when the container and the septum piercing assembly move towards one another.
20. A drug delivery system, comprising:
   a container, including:
      a container body configured to contain a liquid drug therein, the container body having a container end defining a container opening therein; and
      a septum configured to seal the container opening;
   a resettable septum piercing mechanism, including:
      a needle support;
      a pierceable element spaced from the needle support so as to define a cavity between the pierceable element and the needle support when the drug delivery system is in an unpierced configuration to reduce or substantially block admission of germs into the cavity;
      a needle supported by the needle support within the cavity when the drug delivery system is in the unpierced configuration;
      a biasing member; and
      a piercing member defining a through-hole therethrough,
   wherein the drug delivery system is configured such that:
      as the container and the needle are moved towards one another from the unpierced configuration to a pierced configuration, the piercing member pierces the pierceable element and the needle extends out of the cavity through the through-hole of the piercing member and into the septum; and
      as the container and the resettable septum piercing mechanism are moved away from one another from the pierced configuration to the unpierced configuration, the biasing member moves at least one of the pierceable element or the needle support away from the other such that the needle is returned to the cavity.
21. The drug delivery system of claim 20, wherein the piercing member is disposed in the cavity when the drug delivery system is in the unpierced configuration.
22. The drug delivery system of any of claims 20 and 21, wherein the needle is disposed in the through-hole of the piercing member when the drug delivery system is in the unpierced configuration.
23. The drug delivery system of claim 20, further comprising a container pierceable element spaced from the septum so as to define a container cavity between the container pierceable element and the septum when the drug delivery system is in the unpierced configuration to reduce or substantially block admission of germs into the container cavity.
24. The drug delivery system of any of claims 20 and 23, wherein the piercing member is disposed in the container when the drug delivery system is in the unpierced configuration.
25. The drug delivery system of any of claims 20, 23, and 24, wherein the drug delivery system is configured such that as the container and the resettable septum piercing mechanism are moved towards one another from the unpierced configuration to the pierced configuration, the piercing member pierces the container pierceable element and the pierceable element, and the needle extends out of the cavity through the through-hole of the piercing member and into the septum.
26. The drug delivery system of claim 20, further comprising a drive mechanism configured to contact the container to move the container towards the resettable septum piercing assembly.
27. The drug delivery system of claim 20, wherein the drive mechanism withdraws the plunger rod from the container and the container is moved to a post-pierced configuration.
28. The drug delivery system of claim 20, wherein the biasing member is configured to move the at least one of the pierceable element or the needle support to return the at least one of the pierceable element or the needle support from the pierced configuration to a post-pierced configuration.
29. The drug delivery system of any of claims 20 to 28, wherein the cavity is a sterile chamber.
30. The drug delivery system of any of claims 20 to 29, wherein when the container and the resettable septum piercing assembly are moved away from one another, the pierceable element is configured to close when the piercing member is removed.
31. The drug delivery system of any of claims 20 to 30, wherein the pierceable element includes a score line or a slit at a contact location of the piercing member.
32. A method of operating a drug delivery system comprising a drug container and a septum piercing assembly, wherein the drug container comprises a septum, a first pierceable element spaced from the septum so as to define a first cavity, and a piercing member disposed in the first cavity, wherein the first pierceable element reduces or substantially blocks admission of germs into the first cavity, wherein the septum piercing assembly comprises a needle support, a second pierceable element spaced from the needle support so as to define a second cavity, and a needle supported by the needle support within the second cavity, wherein the second pierceable element reduces or substantially blocks admission of germs into the second cavity; wherein the method comprises:
   moving the drug container and the septum piercing assembly towards one another from an unpierced configuration to a pierced configuration, the moving step comprising:
   causing the piercing member to pierce the first and second pierceable elements; and
   causing the needle to extend through a through-hole of the piercing member.
33. The method of claim 32, wherein the moving step comprises: causing the needle that is extended through the through-hole to pierce the septum; and
   delivering a liquid drug contained in the drug container via the needle while the piercing member is piercing the first and second pierceable elements and the needle is disposed in the through-hole.
34. The method of any of claims 32 and 33, further comprising moving the drug container and the septum piercing assembly away from one another from the unpierced configuration to a post-pierced configuration that is identical to the unpierced configuration except that an amount of the liquid drug in the drug container is reduced.
35. The method of any of claims 32 to 34, wherein the drug container is removed and discarded when the drug container is in the post-pierced configuration and replaceable with a second drug container in the unpierced configuration.
36. The method of any of claims 32 to 35, wherein the first and second cavities are sterile chambers.
37. The method of any of claims 32 to 36, wherein when the drug container and the septum piercing assembly are moved away from one another and the piercing member is removed, the first and second pierceable elements close.
38. The method of any of claims 32 to 37, wherein at least one of the first and second pierceable elements includes a score line or a slit at a contact location of the piercing member.
39. A method of operating a drug delivery system comprising a container, a resettable septum piercing mechanism, a biasing member, and a piercing member defining a through-hole therethrough;
   wherein the container comprises a container body configured to contain a liquid drug therein, the container body having a container end defining a container opening therein, wherein the container further comprises a septum configured to seal the container opening,
   wherein the resettable septum piercing mechanism comprises a needle support, a pierceable element spaced from the needle support so as to define a cavity between the pierceable element and the needle support when the drug delivery system is in an unpierced configuration to reduce or substantially block admission of germs into the cavity, and a needle supported by the needle support within the cavity when the drug delivery system is in the unpierced configuration wherein the method comprises:
      moving the container and the resettable septum piercing mechanism towards one another from the unpierced configuration to a pierced configuration so that the piercing member pierces the pierceable element and the needle extends out of the cavity through the through-hole of the piercing member and into the septum; and
      moving the container and the resettable septum piercing mechanism away from one another from the pierced configuration to the unpierced configuration so that the biasing member moves at least one of the pierceable element or the needle support away from the other such that the needle is returned to the cavity.
40. The method of claim 39, wherein the piercing member is disposed in the cavity when the drug delivery system is in the unpierced configuration.
41. The method of any of claims 39 and 40, wherein the needle is disposed in the through-hole of the piercing member when the drug delivery system is in the unpierced configuration.
42. The method of claim 39, wherein the drug delivery system further comprises a container pierceable element spaced from the septum so as to define a container cavity between the container pierceable element and the septum when the drug delivery system is in the unpierced configuration to reduce or substantially block admission of germs into the container cavity.
43. The drug delivery system of any of claims 39 and 42, wherein the piercing member is disposed in the container when the drug delivery system is in the unpierced configuration.
44. The drug delivery system of any of claims 39, 42, and 43, wherein as the container and the resettable septum piercing mechanism are moved towards one another from the unpierced configuration to the pierced configuration, the piercing member pierces the container pierceable element and the pierceable element, and the needle extends out of the cavity through the through-hole of the piercing member and into the septum.

### List of embodiments:

1. A drug delivery system, comprising:
   a container, including
      a container body configured to contain a liquid drug therein, the container body having a container end defining a container opening therein;
      a septum configured to seal the container opening;
      a first pierceable element spaced from the septum so as to define a first cavity between the first pierceable element and the septum when the drug delivery system is in an unpierced configuration to reduce or substantially block admission of germs into the first cavity; and
      a piercing member defining a through-hole therethrough, the piercing member disposed in the first cavity when the drug delivery system is in the unpierced configuration; and
   a septum piercing assembly, including
      a needle support;
      a second pierceable element spaced from the needle support so as to define a second cavity between the second pierceable element and the needle support when the drug delivery system is in the unpierced configuration to reduce or substantially block admission of germs into the second cavity; and
      a needle supported by the needle support within the second cavity when the drug delivery system is in the unpierced configuration;
   wherein the drug delivery system is configured such that, as the container and the septum piercing assembly are moved towards one another from the unpierced configuration to a pierced configuration, the piercing member pierces the first and second pierceable elements and the needle is received through the through-hole of the piercing member into the septum.
2. The drug delivery system of embodiment 1, wherein the first and second cavities are sterile chambers.
3. The drug delivery system of any of embodiments 1 and 2, wherein when the drug delivery system moves from the pierced configuration to a post-pierced configuration, the first and second pierceable elements are configured to close when the piercing member is removed.
4. The drug delivery system of any of embodiments 1 to 3, wherein the first and second pierceable elements are formed from elastomer material.
5. The drug delivery system of any of embodiments 1 to 4, wherein the first and second pierceable elements are formed from rubber or silicone material.
6. The drug delivery system of any of embodiments 1 to 5, wherein the drug delivery system is configured after delivery of the liquid drug in the pierced configuration to move to the post-pierced configuration that is identical to the unpierced configuration except that an amount of the liquid drug in the container is reduced.
7. The drug delivery system of any of embodiments 1 to 6, wherein at least one of the first and second pierceable elements includes a score line or a slit at a contact location of the piercing member.
8. The drug delivery system of any of embodiments 1 to 7, further comprising a sliding member that is slidably supported in a cavity assembly of the container that forms the first cavity.
9. The drug delivery system of any of embodiments 1 to 8, wherein the first pierceable element covers an aperture of the sliding member.
10. The drug delivery system of any of embodiments 1 to 9, wherein the first cavity is formed between the piercing member, an inside wall of the cavity assembly, an inside wall of the sliding member, and an inside wall of the first pierceable element.
11. The drug delivery system of any of embodiments 1 to 10, wherein the sliding member is configured to slide within the cavity assembly toward the septum based on a portion of the septum piercing assembly being received in the cavity assembly and contacting the sliding member when the drug delivery system moves from the unpierced configuration to the pierced configuration.
12. The drug delivery system of any of embodiments 1 to 11, wherein when the drug delivery system is in the pierced configuration, the piercing member is pierced through the first pierceable element covering the aperture of the sliding member and the second pierceable element.
13. The drug delivery system of any of embodiments 1 to 12, wherein when the drug delivery system is in the pierced configuration, the needle extends through the through-hole along a length of the piercing member through the first pierceable element, the aperture of the sliding member, and the second pierceable element.
14. The drug delivery system of any of embodiments 1 to 13, wherein as the drug delivery system moves from the unpierced configuration to the pierced configuration, a tip of the needle moves from the second cavity, through the through-hole of the piercing member that pierces the first and second pierceable elements, through the first cavity, and pierces the septum.
15. The drug delivery system of any of embodiments 1 to 7, wherein the first pierceable element covers an opening of a cavity assembly of the container that forms the first cavity, the first pierceable element being attached at a fixed attachment portion of the first pierceable element to the cavity assembly.
16. The drug delivery system of any of embodiments 1 to 7 and 15, wherein the first pierceable element is configured to deflect, without displacement from the fixed attachment portion, when the septum piercing assembly contacts the first pierceable element.
17. The drug delivery system of any of embodiments 1 to 7, 15, and 16, wherein the drug delivery system is configured to move from the unpierced configuration to the pierced configuration based on the container and the septum piercing assembly moving towards one another based on the deflection of the first pierceable element.
18. The drug delivery system of any of embodiments 1 to 7 and 15 to 17, wherein when the drug delivery system is in the pierced configuration, the piercing member is pierced through the first pierceable element that is deflected and the second pierceable element.
19. The drug delivery system of any of embodiments 1 to 7 and 15 to 18, wherein the first pierceable element is deflected and stretched inward into an interior of the cavity assembly, which reduces a volume of the first cavity, when the container and the septum piercing assembly move towards one another.
20. A drug delivery system, comprising:
   a container, including:
      a container body configured to contain a liquid drug therein, the container body having a container end defining a container opening therein; and
      a septum configured to seal the container opening;
   a resettable septum piercing mechanism, including:
      a needle support;
      a pierceable element spaced from the needle support so as to define a cavity between the pierceable element and the needle support when the drug delivery system is in an unpierced configuration to reduce or substantially block admission of germs into the cavity;
      a needle supported by the needle support within the cavity when the drug delivery system is in the unpierced configuration;
      a biasing member; and
      a piercing member defining a through-hole therethrough,
   wherein the drug delivery system is configured such that:
      as the container and the needle are moved towards one another from the unpierced configuration to a pierced configuration, the piercing member pierces the pierceable element and the needle extends out of the cavity through the through-hole of the piercing member and into the septum; and
      as the container and the resettable septum piercing mechanism are moved away from one another from the pierced configuration to the unpierced configuration, the biasing member moves at least one of the pierceable element or the needle support away from the other such that the needle is returned to the cavity.
21. The drug delivery system of embodiment 20, wherein the piercing member is disposed in the cavity when the drug delivery system is in the unpierced configuration.
22. The drug delivery system of any of embodiments 20 and 21, wherein the needle is disposed in the through-hole of the piercing member when the drug delivery system is in the unpierced configuration.
23. The drug delivery system of embodiment 20, further comprising a container pierceable element spaced from the septum so as to define a container cavity between the container pierceable element and the septum when the drug delivery system is in the unpierced configuration to reduce or substantially block admission of germs into the container cavity.
24. The drug delivery system of any of embodiments 20 and 23, wherein the piercing member is disposed in the container when the drug delivery system is in the unpierced configuration.
25. The drug delivery system of any of embodiments 20, 23, and 24, wherein the drug delivery system is configured such that as the container and the resettable septum piercing mechanism are moved towards one another from the unpierced configuration to the pierced configuration, the piercing member pierces the container pierceable element and the pierceable element, and the needle extends out of the cavity through the through-hole of the piercing member and into the septum.
26. The drug delivery system of embodiment 20, further comprising a drive mechanism configured to contact the container to move the container towards the resettable septum piercing assembly.
27. The drug delivery system of embodiment 20, wherein the drive mechanism withdraws the plunger rod from the container and the container is moved to a post-pierced configuration.
28. The drug delivery system of embodiment 20, wherein the biasing member is configured to move the at least one of the pierceable element or the needle support to return the at least one of the pierceable element or the needle support from the pierced configuration to a post-pierced configuration.
29. The drug delivery system of any of embodiments 20 to 28, wherein the cavity is a sterile chamber.
30. The drug delivery system of any of embodiments 20 to 29, wherein when the container and the resettable septum piercing assembly are moved away from one another, the pierceable element is configured to close when the piercing member is removed.
31. The drug delivery system of any of embodiments 20 to 30, wherein the pierceable element includes a score line or a slit at a contact location of the piercing member.
32. A method of operating a drug delivery system comprising a drug container and a septum piercing assembly, wherein the drug container comprises a septum, a first pierceable element spaced from the septum so as to define a first cavity, and a piercing member disposed in the first cavity, wherein the first pierceable element reduces or substantially blocks admission of germs into the first cavity, wherein the septum piercing assembly comprises a needle support, a second pierceable element spaced from the needle support so as to define a second cavity, and a needle supported by the needle support within the second cavity, wherein the second pierceable element reduces or substantially blocks admission of germs into the second cavity; wherein the method comprises:
   moving the drug container and the septum piercing assembly towards one another from an unpierced configuration to a pierced configuration, the moving step comprising:
   causing the piercing member to pierce the first and second pierceable elements; and
   causing the needle to extend through a through-hole of the piercing member.
33. The method of embodiment 32, wherein the moving step comprises: causing the needle that is extended through the through-hole to pierce the septum; and
   delivering a liquid drug contained in the drug container via the needle while the piercing member is piercing the first and second pierceable elements and the needle is disposed in the through-hole.
34. The method of any of embodiments 32 and 33, further comprising moving the drug container and the septum piercing assembly away from one another from the unpierced configuration to a post-pierced configuration that is identical to the unpierced configuration except that an amount of the liquid drug in the drug container is reduced.
35. The method of any of embodiments 32 to 34, wherein the drug container is removed and discarded when the drug container is in the post-pierced configuration and replaceable with a second drug container in the unpierced configuration.
36. The method of any of embodiments 32 to 35, wherein the first and second cavities are sterile chambers.
37. The method of any of embodiments 32 to 36, wherein when the drug container and the septum piercing assembly are moved away from one another and the piercing member is removed, the first and second pierceable elements close.
38. The method of any of embodiments 32 to 37, wherein at least one of the first and second pierceable elements includes a score line or a slit at a contact location of the piercing member.
39. A method of operating a drug delivery system comprising a container, a resettable septum piercing mechanism, a biasing member, and a piercing member defining a through-hole therethrough;
   wherein the container comprises a container body configured to contain a liquid drug therein, the container body having a container end defining a container opening therein, wherein the container further comprises a septum configured to seal the container opening,
   wherein the resettable septum piercing mechanism comprises a needle support, a pierceable element spaced from the needle support so as to define a cavity between the pierceable element and the needle support when the drug delivery system is in an unpierced configuration to reduce or substantially block admission of germs into the cavity, and a needle supported by the needle support within the cavity when the drug delivery system is in the unpierced configuration wherein the method comprises:
      moving the container and the resettable septum piercing mechanism towards one another from the unpierced configuration to a pierced configuration so that the piercing member pierces the pierceable element and the needle extends out of the cavity through the through-hole of the piercing member and into the septum; and
      moving the container and the resettable septum piercing mechanism away from one another from the pierced configuration to the unpierced configuration so that the biasing member moves at least one of the pierceable element or the needle support away from the other such that the needle is returned to the cavity.
40. The method of embodiment 39, wherein the piercing member is disposed in the cavity when the drug delivery system is in the unpierced configuration.
41. The method of any of embodiments 39 and 40, wherein the needle is disposed in the through-hole of the piercing member when the drug delivery system is in the unpierced configuration.
42. The method of embodiment 39, wherein the drug delivery system further comprises a container pierceable element spaced from the septum so as to define a container cavity between the container pierceable element and the septum when the drug delivery system is in the unpierced configuration to reduce or substantially block admission of germs into the container cavity.
43. The drug delivery system of any of embodiments 39 and 42, wherein the piercing member is disposed in the container when the drug delivery system is in the unpierced configuration.
44. The drug delivery system of any of embodiments 39, 42, and 43, wherein as the container and the resettable septum piercing mechanism are moved towards one another from the unpierced configuration to the pierced configuration, the piercing member pierces the container pierceable element and the pierceable element, and the needle extends out of the cavity through the through-hole of the piercing member and into the septum.

## Claims

1. A drug delivery system, comprising:
a container, including:
a container body configured to contain a liquid drug therein, the container body having a container end defining a container opening therein; and
a septum configured to seal the container opening;
a resettable septum piercing mechanism, including:
a needle support;
a pierceable element spaced from the needle support so as to define a cavity between the pierceable element and the needle support when the drug delivery system is in an unpierced configuration to reduce or substantially block admission of germs into the cavity;
a needle supported by the needle support within the cavity when the drug delivery system is in the unpierced configuration;
a biasing member; and
a piercing member defining a through-hole therethrough,
wherein the drug delivery system is configured such that:
as the container and the needle are moved towards one another from the unpierced configuration to a pierced configuration, the piercing member pierces the pierceable element and the needle extends out of the cavity through the through-hole of the piercing member and into the septum; and
as the container and the resettable septum piercing mechanism are moved away from one another from the pierced configuration to the unpierced configuration, the biasing member moves at least one of the pierceable element or the needle support away from the other such that the needle is returned to the cavity.

2. The drug delivery system of claim 1, wherein the piercing member is disposed in the cavity when the drug delivery system is in the unpierced configuration.

3. The drug delivery system of any of claims 1 and 2, wherein the needle is disposed in the through-hole of the piercing member when the drug delivery system is in the unpierced configuration.

4. The drug delivery system of claim 1, further comprising a container pierceable element spaced from the septum so as to define a container cavity between the container pierceable element and the septum when the drug delivery system is in the unpierced configuration to reduce or substantially block admission of germs into the container cavity.

5. The drug delivery system of any of claims 1 and 4, wherein the piercing member is disposed in the container when the drug delivery system is in the unpierced configuration.

6. The drug delivery system of any of claims 1, 4, and 5, wherein the drug delivery system is configured such that as the container and the resettable septum piercing mechanism are moved towards one another from the unpierced configuration to the pierced configuration, the piercing member pierces the container pierceable element and the pierceable element, and the needle extends out of the cavity through the through-hole of the piercing member and into the septum.

7. The drug delivery system of claim 1, wherein the biasing member is configured to move the at least one of the pierceable element or the needle support to return the at least one of the pierceable element or the needle support from the pierced configuration to a post-pierced configuration.

8. The drug delivery system of any of claims 1 to 7, wherein when the container and the resettable septum piercing assembly are moved away from one another, the pierceable element is configured to close when the piercing member is removed.
